(19) Europäisches Patentamt
European Patent Office
Office européen des brevets

(11) Publication number: **0 603 419 A1**

# (12) EUROPEAN PATENT APPLICATION published in accordance with Art. 158(3) EPC

(21) Application number: **93914995.1**

(22) Date of filing: **13.07.93**

(86) International application number: **PCT/JP93/00967**

(87) International publication number: **WO 94/01433 (20.01.94 94/03)**

(51) Int. Cl.5: **C07D 417/12**, C07D 263/32, A61K 31/425

(30) Priority: **13.07.92 JP 207012/92**

(43) Date of publication of application: **29.06.94 Bulletin 94/26**

(84) Designated Contracting States: **AT BE CH DE DK ES FR GB GR IE IT LI LU MC NL PT SE**

(71) Applicant: **Japan Tobacco Inc.**
**4-12-62 Higashishinagawa**
**Shinagawa-ku, Tokyo 140(JP)**

(72) Inventor: **SHIBATA, Saizo, Phar. Res. Lab.Japan Tobacco Inc.**
**i6-2, Umegaoka, Midori-ku**
**Yokohama-shi, Kanagawa 227(JP)**
Inventor: **ONOGI, Shoji, Phar. Res. Lab. Japan Tobacco Inc.**
**6-2, Umegaoka, Midori-ku,**
**Yokohama-shi, Kanagawa 227(JP)**
Inventor: **SHIRAKAWA, Eiji, Phar.Res.Lab. Japan Tobacco Inc.**
**6-2, Umegaoka, Midori-ku,**
**Yokoham-shi, Kanagawa 227(JP)**
Inventor: **SHINKAI, Hisashi, Phar.Res.Lab.Japan Tobacco Inc.**
**6-2, Umegaoka, Midori-ku,**
**Yokohama-shi, Kanagawa 227(JP)**
Inventor: **FURUKAWA, Noboru, Tox.Res.Lab. Japan Tobacco Inc.**
**23, Nakogi,**
**Hadano-shi,**
**Kanagawa 257(JP)**
Inventor: **UCHIDA, Itsuo, Phar.Res.Lab. Japan Tobacco Inc.**
**6-2, Umegaoka, Midori-ku,**
**Yokohama-shi, Kanagawa 227(JP)**

(74) Representative: **von Kreisler, Alek, Dipl.-Chem. et al**
**Patentanwälte**
**von Kreisler-Selting-Werner**
**Postfach 10 22 41**
**D-50462 Köln (DE)**

## (54) NOVEL THIAZOLIDINEDIONE COMPOUND AND USE THEREOF.

(57) A novel thiazolidinedione compound represented by general formula (I), a pharmaceutically acceptable salt thereof, and a pharmaceutical composition thereof; wherein R represents an aromatic, cycloaliphatic or heteroaromatic hydrocarbon group which may be substituted; $R_1$ and $R_2$ represent each independently hydrogen, lower alkyl, or an aromatic or heteroaromatic hydrocarbon group which may be substituted by lower alkyl, acyl, lower alkoxy, cyano, nitro, lower alkoxycarbonyl or sulfonyl; $R_3$ represents hydrogen, hydroxy, lower alkyl, acyl, lower alkoxy, cyano, nitro, lower alkoxycarbonyl or sulfonyl; $R_4$ represents lower alkyl; X represents oxygen, sulfur or sec. amino; A represents $(CH_2)_m$ (wherein m is an integer of 0, 1, or 2); and B represents $(CH_2)_n$ (wherein n is an integer of 0, 1 or 2). This compound is excellent in hypoglycemic and hypolipemic effects, so that it is useful for treating diabetes, preventing and treating the complications of diabetes, and treating hyperlipemia.

EP 0 603 419 A1

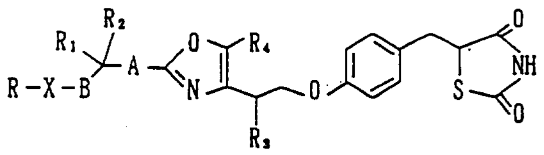

(I)

## Technical Field

The present invention relates to a novel thiazolidinedione compound and more particularly, the invention relates to a novel thiazolidinedione compound having hypoglycemic action and hypolipemic action, so that useful as a therapeutic agent for diabetes mellitus, an agent for the prevention and treatment of the complications of diabetes mellitus and a therapeutic agent for hyperlipemia, and to a pharmaceutical composition thereof.

## Background Art

The treatment of non-insulin dependent diabetes mellitus (NIDDM) generally involves a combination of alimentary therapy, ergotherapy and administration of insulin or an orally active hypoglycemic agent. As the orally active hypoglycemic agent, currently known are sulfonylurea preparations such as tolbutamide, chloropropamide, acetohexamide, glibenclamide and tolazamide, and biguanide preparations such as phenformine, buformine and metformine.

While the sulfonylurea drugs exhibit potent hypoglycemic action, they often induce grave and prolonged hypoglycemia, and when used chronically, they sometimes fail to prove effectiveness. In addition, the biguanide preparations tend to cause severe lactic acid-acidosis and considerable attention should be paid when these drugs are used.

Accordingly, development of a new therapeutic agent for diabetes and an agent for the prevention and treatment of the complications thereof, which is free of these defects, is awaited.

Incidentally, a hypoglycemic action of thiazolidine compounds such as [5-[4-[2-(5-methyl-2-phenyl-4-oxazolyl)-ethoxy]benzyl]-2,4-thiazolidinedione] is taught in Japanese Patent Unexamined Publication No. 85372/1986, that by thiazolidine compounds such as [(± )-5-[4-[6-hydroxy-2,5,7,8-tetramethylchroman-2-yl-methoxy)benzyl]-2,4-thiazolidinedione] is taught in Japanese Patent Unexamined Publication No. 51189/1985 and that by [5-methyl-2-styryl-4-oxazolacetic acid] is taught in Japanese Patent Unexamined Publication No. 219169/1983.

However, these literatures do not suggest the compounds of the present invention.

## Disclosure of the Invention

With the aim of providing a novel compound useful as a therapeutic agent for diabetes, an agent for the prevention and treatment of the complications of diabetes and a therapeutic agent for hyperlipemia as mentioned above, the present inventors have conducted intensive studies and now found a novel thiazolidinedione compound having a very potent and extremely low toxic hypoglycemic action and hypolipemic action as compared with known compounds, which resulted in the completion of the present invention.

Accordingly, the present invention relates to novel thiazolidinedione compounds, intermediates thereof and pharmaceutical compositions containing them of the following (1) through (24).

(1) A novel thiazolidinedione compound of the formula (I)

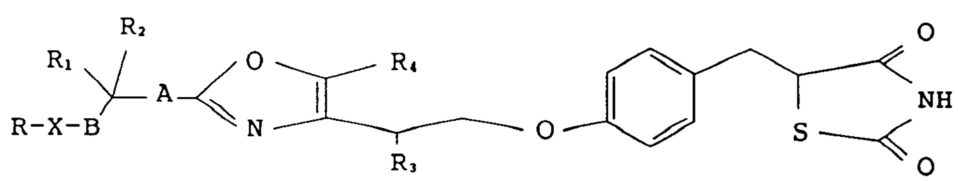

(I)

wherein;

| | |
|---|---|
| R | is optionally substituted aromatic hydrocarbon, optionally substituted alicyclic hydrocarbon or optionally substituted aromatic heterocyclic group; |
| $R_1$ and $R_2$ | are each independently hydrogen atom, lower alkyl, or aromatic hydrocarbon or aromatic heterocyclic group which may be substituted by lower alkyl, acyl, lower alkoxy, cyano, nitro, lower alkoxycarbonyl or sulfonyl; |
| $R_3$ | is hydrogen atom, hydroxy, lower alkyl, acyl, lower alkoxy, cyano, nitro, lower alkoxycarbonyl or sulfonyl; |
| $R_4$ | is lower alkyl; |

| | |
|---|---|
| X | is oxygen atom, sulfur atom or secondary amino; |
| A | is $-(CH_2)m-$ where m is an integer of 0, 1 or 2; and |
| B | is $-(CH_2)n-$ where n is an integer of 0, 1 or 2, |

or a pharmaceutically acceptable salt thereof.

(2) A novel thiazolidinedione compound of the above (1) wherein $R_1$ and $R_2$ are each independently hydrogen atom, lower alkyl or aromatic hydrocarbon, $R_3$ is hydrogen atom or hydroxy, $R_4$ is methyl, m is 0 and n is 0, or a pharmaceutically acceptable salt thereof.

(3) A novel thiazolidinedione compound of the above (2) wherein R is optionally substituted phenyl, optionally substituted 5- or 6-membered alicyclic hydrocarbon or optionally substituted 5- or 6-membered aromatic heterocyclic group which has a hetero atom selected from nitrogen atom, oxygen atom and sulfur atom, or a pharmaceutically acceptable salt thereof.

(4) A novel thiazolidinedione compound of the above (3) wherein R is optionally substituted phenyl, or a pharmaceutically acceptable salt thereof.

(5) A novel thiazolidinedione compound of the above (4) wherein the optionally substituted phenyl is 3,5-di-tert-butyl-4-hydroxyphenyl, or a pharmaceutically acceptable salt thereof.

(6) A novel thiazolidinedione compound of the above (3) wherein R is 5- or 6-membered alicyclic hydrocarbon which may have 1 or 2 double bonds, or a pharmaceutically acceptable salt thereof.

(7) A novel thiazolidinedione compound of the above (6) wherein R is cyclopentyl or cyclohexyl, or a pharmaceutically acceptable salt thereof.

(8) A novel thiazolidinedione compound of the above (3) wherein the aromatic heterocyclic group at R is a 5- or 6-membered aromatic heterocyclic group which has 1 to 3 hetero atoms selected from nitrogen atom, oxygen atom and sulfur atom, or a pharmaceutically acceptable salt thereof.

(9) A novel thiazolidinedione compound of the above (8) wherein the aromatic heterocyclic group at R is a 5- or 6-membered aromatic heterocyclic group which has 1 to 3 nitrogen atoms, or a pharmaceutically acceptable salt thereof.

(10) A novel thiazolidinedione compound of the above (1) wherein R is selected from methoxyphenyl, hydroxyphenyl, benzyloxyphenyl, pyridyl, pyrimidinyl, nitrophenyl, chlorophenyl, di-tert-butylphenyl, 1-methyl-2-imidazolyl, cyclohexyl, phenyl, dimethoxyphenyl and di-tert-butylhydroxyphenyl, or a pharmaceutically acceptable salt thereof.

(11) A compound of the formula

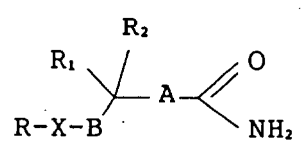

wherein;

| | |
|---|---|
| R | is optionally substituted aromatic hydrocarbon, optionally substituted alicyclic hydrocarbon or optionally substituted aromatic heterocyclic group; |
| $R_1$ and $R_2$ | are each independently hydrogen atom, lower alkyl, or aromatic hydrocarbon or aromatic heterocyclic group which may be substituted by lower alkyl, acyl, lower alkoxy, cyano, nitro, lower alkoxycarbonyl or sulfonyl; |
| X | is oxygen atom, sulfur atom or secondary amino; |
| A | is $-(CH_2)m-$ where m is an integer of 0, 1 or 2; and |
| B | is $-(CH_2)n-$ where n is an integer of 0, 1 or 2. |

(12) A compound of the above (11) wherein $R_1$ and $R_2$ are each independently hydrogen atom, lower alkyl or aromatic hydrocarbon, m is 0 and n is 0.

(13) A compound of the formula

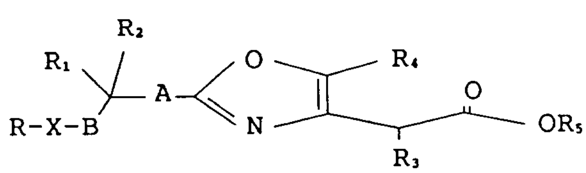

wherein;

| | |
|---|---|
| R | is optionally substituted aromatic hydrocarbon, optionally substituted alicyclic hydrocarbon or optionally substituted aromatic heterocyclic group; |
| $R_1$ and $R_2$ | are each independently hydrogen atom, lower alkyl, or aromatic hydrocarbon or aromatic heterocyclic group which may be substituted by lower alkyl, acyl, lower alkoxy, cyano, nitro, lower alkoxycarbonyl or sulfonyl; |
| $R_3$ | is hydrogen atom, hydroxy, lower alkyl, acyl, lower alkoxy, cyano, nitro, lower alkoxycarbonyl or sulfonyl; |
| $R_4$ | is lower alkyl; |
| $R_5$ | is hydrogen atom or lower alkyl; |
| X | is oxygen atom, sulfur atom or secondary amino; |
| A | is $-(CH_2)m-$ where m is an integer of 0, 1 or 2; and |
| B | is $-(CH_2)n-$ where n is an integer of 0, 1 or 2. |

(14) A compound of the above (13) wherein $R_1$ and $R_2$ are each independently hydrogen atom, lower alkyl or aromatic hydrocarbon, $R_3$ is hydrogen atom or hydroxy, $R_4$ is methyl, m is 0 and n is 0.

(15) A compound of the formula

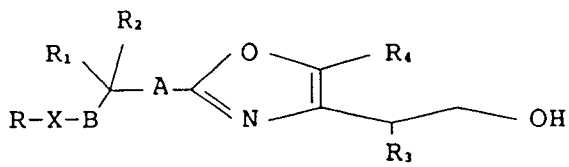

wherein;

| | |
|---|---|
| R | is optionally substituted aromatic hydrocarbon, optionally substituted alicyclic hydrocarbon or optionally substituted aromatic heterocyclic group; |
| $R_1$ and $R_2$ | are each independently hydrogen atom, lower alkyl, or aromatic hydrocarbon or aromatic heterocyclic group which may be substituted by lower alkyl, acyl, lower alkoxy, cyano, nitro, lower alkoxycarbonyl or sulfonyl; |
| $R_3$ | is hydrogen atom, hydroxy, lower alkyl, acyl, lower alkoxy, cyano, nitro, lower alkoxycarbonyl or sulfonyl; |
| $R_4$ | is lower alkyl; |
| X | is oxygen atom, sulfur atom or secondary amino; |
| A | is $-(CH_2)m-$ where m is an integer of 0, 1 or 2; and |
| B | is $-(CH_2)n-$ where n is an integer of 0, 1 or 2. |

(16) A compound of the above (15) wherein $R_1$ and $R_2$ are each independently hydrogen atom, lower alkyl or aromatic hydrocarbon, $R_3$ is hydrogen atom or hydroxy, $R_4$ is methyl, m is 0 and n is 0.

(17) A compound of the formula

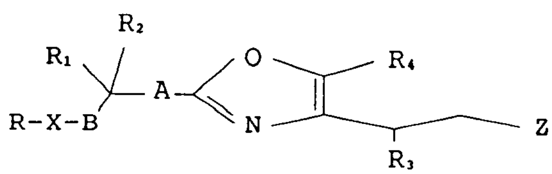

wherein;

| | |
|---|---|
| R | is optionally substituted aromatic hydrocarbon, optionally substituted alicyclic hydrocarbon or optionally substituted aromatic heterocyclic group; |
| $R_1$ and $R_2$ | are each independently hydrogen atom, lower alkyl, or aromatic hydrocarbon or aromatic heterocyclic group which may be substituted by lower alkyl, acyl, lower alkoxy, cyano, nitro, lower alkoxycarbonyl or sulfonyl; |
| $R_3$ | is hydrogen atom, hydroxy, lower alkyl, acyl, lower alkoxy, cyano, nitro, lower alkoxycarbonyl or sulfonyl; |
| $R_4$ | is lower alkyl; |
| X | is oxygen atom, sulfur atom or secondary amino; |
| A | is $-(CH_2)m-$ where m is an integer of 0, 1 or 2; |

B is $-(CH_2)n-$ where n is an integer of 0, 1 or 2; and

Z is a leaving group.

(18) A compound of the above (17) wherein $R_1$ and $R_2$ are each independently hydrogen atom, lower alkyl or aromatic hydrocarbon, $R_3$ is hydrogen atom or hydroxy, $R_4$ is methyl, m is 0 and n is 0.

(19) A novel thiazolidinedione compound of the above (1), which is selected from
5-[4-[2-[2-[1-(3,5-di-tert-butyl-4-hydroxyphenylthio)ethyl]-5-methyl-4-oxazolyl]ethoxy]benzyl]-2,4-thiazolidinedione;
5-[4-[2-[2-[1-(3,5-di-tert-butyl-4-hydroxyphenylthio)methyl]-5-methyl-4-oxazolyl]ethoxy]benzyl]-2,4-thiazolidinedione;
5-[4-[2-[2-[1-(3,5-di-tert-butyl-4-hydroxyphenylthio)propyl]-5-methyl-4-oxazolyl]ethoxy]benzyl]-2,4-thiazolidinedione;
5-[4-[2-[2-[1-(3,5-di-tert-butyl-4-hydroxyphenylthio)isopropyl]-5-methyl-4-oxazolyl]ethoxy]benzyl]-2,4-thiazolidinedione;
5-[4-[2-[2-[1-(3,5-di-tert-butyl-4-hydroxyphenylthio)-1-phenylmethyl]-5-methyl-4-oxazolyl]ethoxy]benzyl]-2,4-thiazolidinedione;
5-[4-[2-[5-methyl-2-(4-methoxyphenylthio)methyl-4-oxazolyl]ethoxy]benzyl]-2,4-thiazolidinedione;
5-[4-[2-[5-methyl-2-(4-hydroxyphenylthio)methyl-4-oxazolyl]ethoxy]benzyl]-2,4-thiazolidinedione;
5-[4-[2-[5-methyl-2-(4-benzyloxyphenoxy)methyl-4-oxazolyl]ethoxy]benzyl]-2,4-thiazolidinedione;
5-[4-[2-[5-methyl-2-[1-(2-pyridyloxy)ethyl]-4-oxazolyl]ethoxy]benzyl]-2,4-thiazolidinedione;
5-[4-[2-[5-methyl-2-[1-(2-pyridylthio)ethyl]-4-oxazolyl]ethoxy]benzyl]-2,4-thiazolidinedione;
5-[4-[2-[5-methyl-2-(2-pyrimidinylthio)methyl-4-oxazolyl]ethoxy]benzyl]-2,4-thiazolidinedione;
5-[4-[2-[5-methyl-2-(2-pyridylthio)methyl-4-oxazolyl]ethoxy]benzyl]-2,4-thiazolidinedione;
5-[4-[2-[5-methyl-2-(4-nitrophenylthio)methyl-4-oxazolyl]ethoxy]benzyl]-2,4-thiazolidinedione;
5-[4-[2-[5-methyl-2-(4-chlorophenylthio)methyl-4-oxazolyl]ethoxy]benzyl]-2,4-thiazolidinedione;
5-[4-[2-[5-methyl-2-(3,5-di-tert-butylphenoxy)methyl-4-oxazolyl]ethoxy]benzyl]-2,4-thiazolidinedione;
5-[4-[2-[5-methyl-2-(1-methyl-2-imidazolylthio)methyl-4-oxazolyl]ethoxy]benzyl]-2,4-thiazolidinedione;
5-[4-[2-(5-methyl-2-cyclohexylthiomethyl-4-oxazolyl)ethoxy]benzyl]-2,4-thiazolidinedione;
5-[4-[2-(5-methyl-2-phenylaminomethyl-4-oxazolyl)ethoxy]benzyl]2,4-thiazolidinedione;
5-[4-[2-(5-methyl-2-phenylthiomethyl-4-oxazolyl)ethoxy]benzyl]-2,4-thiazolidinedione;
5-[4-[2-(5-methyl-2-phenoxymethyl-4-oxazolyl)ethoxy]benzyl]-2,4-thiazolidinedione;
5-[4-[2-[5-methyl-2-(3,5-dimethoxyphenoxy)methyl-4-oxazolyl]ethoxy]benzyl]-2,4-thiazolidinedione; and
5-[4-[2-[5-methyl-2-(4-hydroxyphenoxy)methyl-4-oxazolyl]ethoxy]benzyl]-2,4-thiazolidinedione, or a pharmaceutically acceptable salt thereof.

(20) A compound of the above (11) which is selected from 2-(3,5-di-tert-butyl-4-hydroxyphenylthio)-propionamide;
(3,5-di-tert-butyl-4-hydroxyphenylthio)acetamide;
2-(3,5-di-tert-butyl-4-hydroxyphenylthio)butyramide;
2-(3,5-di-tert-butyl-4-hydroxyphenylthio)isobutyramide; and
2-(3,5-di-tert-butyl-4-hydroxyphenylthio)-2-phenylacetamide.

(21) A compound of the above (13) which is selected from
methyl 2-[1-(3,5-di-tert-butyl-4-hydroxyphenylthio)ethyl]-5-methyl-4-oxazolylacetate;
methyl [2-(3,5-di-tert-butyl-4-hydroxyphenylthio)methyl]-5-methyl-4-oxazolylacetate;
methyl 2-[1-(3,5-di-tert-butyl-4-hydroxyphenylthio)propyl]-5-methyl-4-oxazolylacetate;
methyl 2-[1-(3,5-di-tert-butyl-4-hydroxyphenylthio)isopropyl]-5-methyl-4-oxazolylacetate; and
methyl 2-[1-(3,5-di-tert-butyl-4-hydroxyphenylthio)-1-phenylmethyl]-5-methyl-4-oxazolylacetate.

(22) A compound of the above (15) which is selected from
2-[2-[1-(3,5-di-tert-butyl-4-hydroxyphenylthio)ethyl]-5-methyl-4-oxazolyl]ethanol;
2-[2-(3,5-di-tert-butyl-4-hydroxyphenylthio)methyl-5-methyl-4-oxazolyl]ethanol;
2-[2-[1-(3,5-di-tert-butyl-4-hydroxyphenylthio)propyl]-5-methyl-4-oxazolyl]ethanol;
2-[2-[1-(3,5-di-tert-butyl-4-hydroxyphenylthio)isopropyl]-5-methyl-4-oxazolyl]ethanol;
2-[2-[1-(3,5-di-tert-butyl-4-hydroxyphenylthio)-1-phenylmethyl]-5-methyl-4-oxazolyl]ethanol;
2-[5-methyl-2-(4-methoxyphenylthio)methyl-4-oxazolyl]ethanol;
2-[5-methyl-2-(4-hydroxyphenylthio)methyl-4-oxazolyl]ethanol;
2-[5-methyl-2-(4-benzyloxyphenoxy)methyl-4-oxazolyl]ethanol;
2-[5-methyl-2-(4-hydroxyphenoxy)methyl-4-oxazolyl]ethanol;
2-[5-methyl-2-(3,5-dimethoxyphenoxy)methyl-4-oxazolyl]ethanol;
2-[5-methyl-2-[1-(2-pyridyloxy)ethyl]-4-oxazolyl]ethanol;
2-[5-methyl-2-[1-(2-pyridylthio)ethyl]-4-oxazolyl]ethanol;

2-[5-methyl-2-(2-pyrimidinylthio)methyl-4-oxazolyl]ethanol;
2-[5-methyl-2-(2-pyridylthio)methyl-4-oxazolyl]ethanol;
2-[5-methyl-2-(4-nitrophenylthio)methyl-4-oxazolyl]ethanol;
2-[5-methyl-2-(4-chlorophenylthio)methyl-4-oxazolyl]ethanol;
2-[5-methyl-2-(3,5-di-tert-butylphenoxy)methyl-4-oxazolyl]ethanol;
2-[5-methyl-2-(1-methyl-2-imidazolylthio)methyl-4-oxazolyl]ethanol;
2-(5-methyl-2-cyclohexylthiomethyl-4-oxazolyl)ethanol;
2-(5-methyl-2-phenylaminomethyl-4-oxazolyl)ethanol;
2-(5-methyl-2-phenylthiomethyl-4-oxazolyl)ethanol; and
2-(5-methyl-2-phenoxymethyl-4-oxazolyl)ethanol.

(23) A compound of the above (17) which is selected from
2-[2-[1-(3,5-di-tert-butyl-4-hydroxyphenylthio)ethyl]-5-methyl-4-oxazolyl]ethyl p-toluenesulfonate;
[2-[2-(3,5-di-tert-butyl-4-hydroxyphenylthio)methyl]-5-methyl-4-oxazolyl]ethyl p-toluenesulfonate;
2-[2-[1-(3,5-di-tert-butyl-4-hydroxyphenylthio)propyl]-5-methyl-4-oxazolyl]ethyl p-toluenesulfonate;
2-[2-[1-(3,5-di-tert-butyl-4-hydroxyphenylthio)isopropyl]-5-methyl-4-oxazolyl]ethyl p-toluenesulfonate; and
2-[2-[1-(3,5-di-tert-butyl-4-hydroxyphenylthio)-1-phenylmethyl]-5-methyl-4-oxazolyl]ethyl p-toluenesulfonate.

(24) A pharmaceutical composition containing a novel thiazolidinedione compound of the above (1) or a pharmaceutically acceptable salt thereof and pharmaceutically acceptable additives.

As used herein, aromatic hydrocarbon means phenyl, biphenyl, naphthyl and so on.

Alicyclic hydrocarbon means alicyclic hydrocarbon having 3 to 7 carbon atoms, such as cyclopropyl, cyclobutyl, cyclopentyl, cyclohexyl, cycloheptyl, cyclopropenyl, cyclobutenyl, cyclobutadienyl, cyclopentenyl, cyclopentadienyl, cyclohexenyl, cyclohexadienyl, cycloheptenyl, cycloheptadienyl and so on. Preferred are alicyclic hydrocarbon having 5 to 7 carbon atoms, such as cyclopentyl, cyclohexyl, cycloheptyl, cyclopentenyl, cyclopentadienyl, cyclohexenyl, cyclohexadienyl, cycloheptenyl and cycloheptadienyl, with particular preference given to cyclopentyl and cyclohexyl.

Aromatic heterocyclic group means 4- to 6-membered, preferably 5- or 6-membered aromatic heterocyclic group having, besides carbon atom, 1 to 3 hetero atoms selected from nitrogen atom, oxygen atom and sulfur atom as an atom constituting the ring, and it may be a condensed ring. Specific examples thereof are thienyl, furyl, pyrrolyl, imidazolyl, pyrazolyl, thiazolyl, isothiazolyl, oxazolyl, isoxazolyl, oxadiazolyl, thiadiazolyl, triazolyl, pyridyl, pyrazinyl, pyrimidinyl, pyridazinyl, triazinyl, dithiazolyl, dioxolanyl, dithiolyl, pyrrolidinyl, dithiaziadinyl, thiaziadinyl, morpholinyl, oxazinyl, thiazinyl, piperazinyl, piperidinyl, pyranyl, thiopyranyl, furoisoxazolyl, imidazothiazolyl, thienoisothiazolyl, thienothiazolyl, imidazopyrazolyl, cyclopentapyrazolyl, pyrrolopyrrolyl, cyclopentathienyl, thienothienyl, oxadiazolopyrazinyl, benzofurazanyl, thiadiazolopyrimidinyl, triazolothiazinyl, triazolopyrimidinyl, triazolopyridinyl, benzotothiazolyl, oxazolopyrimidinyl, oxazolopyridinyl, benzoxazolyl, thiazolopyridazinyl, thiazolopyrimidinyl, benzoisothiazolyl, benzothiazolyl, pyrazolotriazinyl, pyrazolothiazinyl, imidazopyrazinyl, purinyl, pyrazolopyridazinyl, pyrazolopyrimidinyl, imidazopyridinyl, pyranopyrazolyl, benzimidazolyl, indazolyl, benzoxathiolyl, benzodioxalyl, dithiolopyrimidinyl, benzodithiolyl, indolizinyl, indolyl, isoindolyl, furopyrimidinyl, furopyridinyl, benzofuranyl, isobenzofuranyl, thienopyrazinyl, thienopyrimidinyl, thienodioxynyl, thienopyridinyl, benzothienyl, cyclopentaoxazinyl, cyclopentafuranyl, benzothiadiazinyl, benzotriazinyl, pyridoxazinyl, benzoxazinyl, pyrimidothiazinyl, benzothiazinyl, pyrimidopyridazinyl, pyrimidopyrimidinyl, pyridopyridazinyl, pyridopyrimidinyl, cinnolinyl, quinazolinyl, quinoxalinyl, benzoxathiinyl, benzodioxynyl, benzodithiinyl, naphthylidinyl, isoquinolinyl, quinolinyl, benzopyranyl, benzothiopyranyl and so on, with particular preference given to pyridyl, pyrimidinyl and imidazolyl.

Lower alkyl means straight- or branched chain alkyl having 1 to 6 carbon atoms, such as methyl, ethyl, propyl, isopropyl, butyl, isobutyl, sec-butyl, tert-butyl, pentyl, isopentyl, neopentyl, tert-pentyl, hexyl, isohexyl, neohexyl and so on. Preferred are straight- or branched chain alkyl having 1 to 4 carbon atoms, such as methyl, ethyl, propyl, isopropyl, butyl, isobutyl, sec-butyl and tert-butyl.

Acyl means straight chain, branched chain or cyclic acyl, preferably having 1 to 7 carbon atoms, such as formyl, acetyl, propionyl, butyryl, isobutyryl, valeryl, isovaleryl, pivaloyl, caproyl, isocaproyl, acryloyl, propioloyl, methacryloyl, crotonoyl, isocrotonoyl, benzoyl and so on, with preference given to formyl and acetyl.

Lower alkoxy means straight- or branched chain alkoxy having 1 to 6 carbon atoms, such as methoxy, ethoxy, propoxy, isopropoxy, butoxy, isobutoxy, sec-butoxy, tert-butoxy, pentyloxy, isopentyloxy, neopentyloxy, tert-pentyloxy, hexyloxy, isohexyloxy, neohexyloxy and so on, with preference given to straight- or branched chain alkoxy having 1 to 4 carbon atoms, such as methoxy, ethoxy, propoxy, isopropoxy, butoxy, isobutoxy, sec-butoxy and tert-butoxy.

Lower alkoxycarbonyl means straight- or branched chain alkoxycarbonyl having 2 to 5 carbon atoms, such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl and so on, with preference given to methoxycarbonyl and ethoxycarbonyl.

Leaving group means a group, the elimination of which leaves a hydroxyl group, and there is no particular limitation imposed thereon insofar as it is a group conventionally employed. Specific examples include p-toluenesulfonyloxy, benzenesulfonyloxy, methanesulfonyloxy and halogen atom, with preference given to p-toluenesulfonyloxy.

"Optionally substituted" means optionally substituted by 1 to 3 substituents and the substituents may be the same or different. Specific examples thereof include lower alkyl such as methyl, ethyl, propyl, butyl, tert-butyl and the like; lower alkoxy such as methoxy, ethoxy, propoxy, butoxy, tert-butoxy and the like; halogen atom such as fluorine, chlorine, bromine and the like; nitro; cyano; hydroxy; acyl such as formyl, acetyl, propionyl, butyryl, isobutyryl, benzoyl, naphthoyl and the like; acyloxy such as formyloxy, acetyloxy, propionyloxy, butyryloxy, isobutyryloxy, benzoyloxy and the like; aralkyloxy such as benzyloxy, phenetyloxy, phenylpropyloxy and the like; mercapto; alkylthio such as methylthio, ethylthio, propylthio, butoxythio, isobutoxythio, tert-butoxythio and the like; amino; alkylamino such as methylamino, ethylamino, propylamino, isopropylamino, butylamino and the like; dialkylamino such as dimethylamino, diethylamino, dipropylamino, diisopropylamino, dibutylamino and the like; alkoxycarbonyl such as methoxycarbonyl, ethoxycarbonyl, propoxycarbonyl, isopropoxycarbonyl, butoxycarbonyl, tert-butoxycarbonyl and the like; amide; trifluoromethyl; phosphoryl; sulfonyl; sulfonyloxy; sulfamoyl; alkylphosphonamide such as methylphosphonamide, ethylphosphonamide, propylphosphonamide, isopropylphosphonamide and the like; methylenedioxy; alkoxyphosphoryl such as methoxyphosphoryl, ethoxyphosphoryl, propyloxyphosphoryl, isopropyloxyphosphoryl and the like; alkylsulfonyl such as methylsulfonyl, ethylsulfonyl, propylsulfonyl, butylsulfonyl, tert-butylsulfonyl and the like; alkylsulfonylamino such as methylsulfonylamino, ethylsulfonylamino, propylsulfonylamino, butylsulfonylamino, tert-butylsulfonylamino and the like; carbamoyl; carboxyl; and the like, with preference given to hydroxy, lower alkyl, lower alkoxy, aralkyloxy, mercapto, lower alkylthio, nitro, halogen atom, trifluoromethyl, amino, dialkylamino, alkylamino, acyl, cyano, carbamoyl, acyloxy, sulfonyl, carboxyl and alkoxycarbonyl and with particular preference given to hydroxy, lower alkyl, lower alkoxy, aralkyloxy, nitro and halogen atom and when substituted by 3 substituents, lower alkyl and hydroxy are preferable.

Pharmaceutically acceptable salt may be any insofar as it forms a nontoxic salt with 2,4-thiazolidinedione compound of the formula (I) described above and is exemplified by alkaline metal salts such as sodium salt, potassium salt and the like; alkaline earth metal salts such as magnesium salt, calcium salt and the like; ammonium salt; organic base salts such as trimethylamine salt, triethylamine salt, pyridine salt, picoline salt, dicyclohexylamine salt, N,N'-dibenzylethylenediamine salt and the like; and amino acid salts such as lysine salt, arginine salt and the like.

The compounds of the present invention exhibit superior hypoglycemic action and hypolipemic action in mammals inclusive of mouse, rat, rabbit, dog, cat, human etc. and are useful as a therapeutic agent for diabetes and hyperlipemia and is expected to be useful as an agent for the prevention and treatment of arteriosclerosis which is a complication of diabetes.

When the compounds of the formula (I) and pharmaceutically acceptable salts thereof are used as pharmaceutical preparations, they are generally admixed with pharmacologically acceptable carrier, excipient, diluent, extender, disintegrator, stabilizer, preservative, buffer, emulsifier, aromatic substance, coloring, sweetner, viscosity-imparting agent, corrigent, solubilizer and other additives such as water, vegetable oil, alcohol (e.g. ethanol, benzyl alcohol), carbohydrates (e.g. polyethylene glycol, grycerol triacetate, gelatin, lactose, starch), magnesium stearate, talc, lanolin, petrolatum and the like and prepared into tablet, pill, powder, granule, suppository, injection, eye drop, liquid, capsule, troche, aerosol, elixir, suspension, emulsion, syrup and the like which are orally or parenterally administered.

While the dose varies depending on the kind and severity of disease, compound to be administered, administration route, age, sex, body weight of patients and the like, a daily dose for an adult is generally 0.01-1000 mg, particularly 0.05-100 mg of Compound (I).

The compounds of the formula (I) have one or more asymmetric carbons and when it has one, a pure optically active compound, a mixture thereof at an optional proportion or a racemate is present; and when it has two or more, optically pure diastereomers, racemates thereof, combination of these or a mixture at an optional proportion are(is) present, all of which fall within the scope of the present invention.

The Compounds (I) of the present invention are produced, for example, by the following methods, to which the production method of the present invention is not limited.

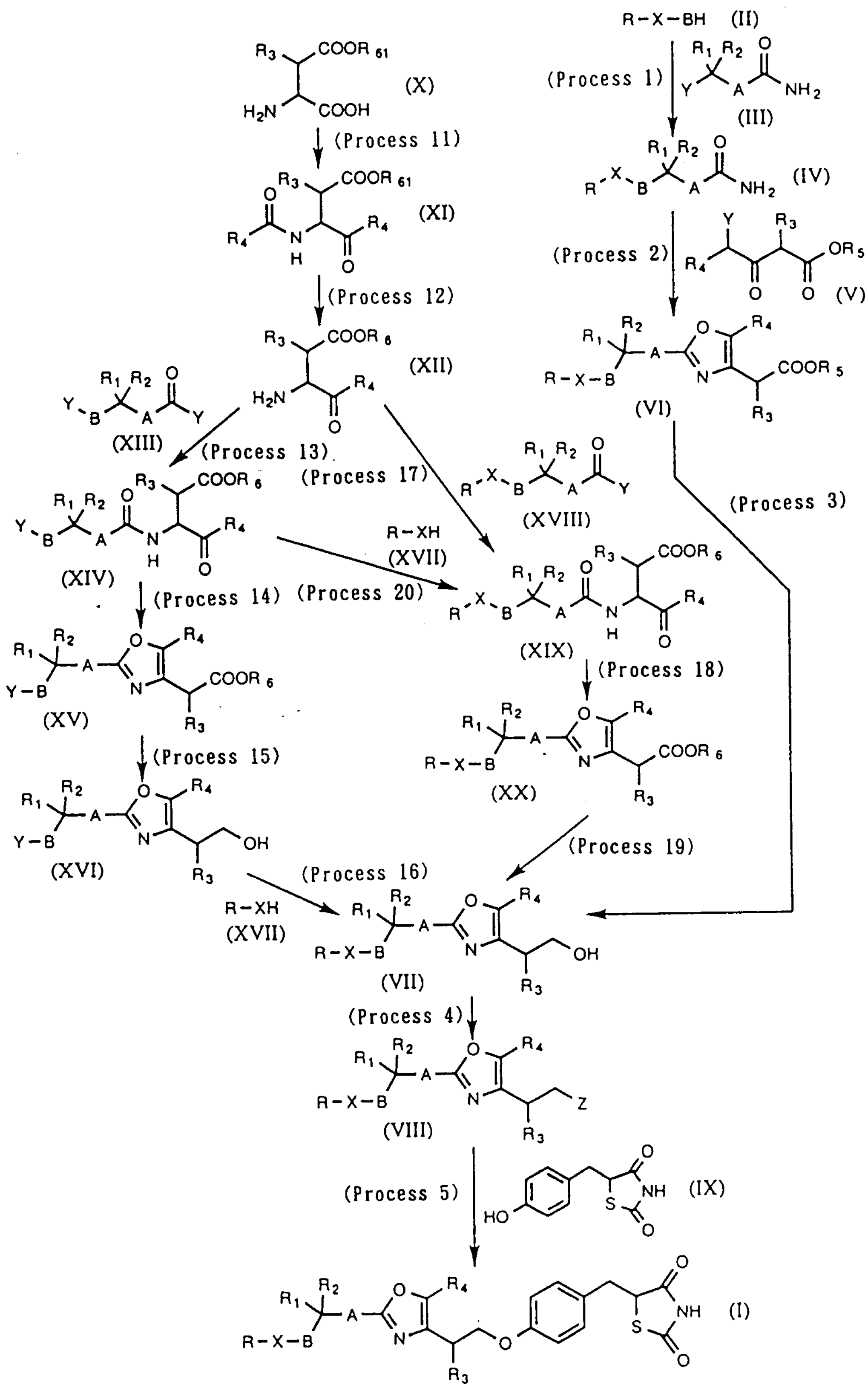


(Process 1)

Compound (IV) (wherein R, $R_1$, $R_2$, A, B and X are as defined above) can be synthesized by reacting a compound (II) (wherein R, B and X are as defined above) and a compound (III) (wherein Y is halogen atom,

and $R_1$, $R_2$ and A are as defined above) in an organic solvent such as benzene, toluene, dichloromethane, chloroform, ether, dioxane, tetrahydrofuran, acetonitrile, dimethylformamide, dimethyl sulfoxide, sulfolane, dimethoxyethane, pyridine, acetone, ethyl acetate, methanol, ethanol or the like, water or a mixed solvent thereof in the presence or absence of a base such as sodium hydride, potassium hydride, sodium amide, sodium alkoxide, potassium alkoxide, triethylamine, sodium hydroxide, sodium bicarbonate, sodium carbonate or the like under cooling to under heating.

(Process 2)

Compound (VI) (wherein R, $R_1$, $R_2$, $R_3$, $R_4$, $R_5$, A, B and X are as defined above) can be synthesized by reacting a compound (IV) (wherein R, $R_1$, $R_2$, A, B and X are as defined above) and a compound (V) (wherein $R_3$, $R_4$, $R_5$ and Y are as defined above) in an organic solvent such as benzene, toluene, dichloromethane, chloroform, ether, dioxane, tetrahydrofuran, acetonitrile, dimethylformamide, dimethyl sulfoxide, sulfolane, dimethoxyethane, pyridine, acetone, acetic acid, acetic anhydride or the like or a mixed solvent thereof or without solvent under cooling to under heating, preferably under heating.

(Process 3)

Compound (VII) (wherein R, $R_1$, $R_2$, $R_3$, $R_4$, A, B and X are as defined above) can be synthesized by reacting a compound (VI) (wherein R, $R_1$, $R_2$, $R_3$, $R_4$, $R_5$, A, B and X are as defined above) with a reducing agent such as lithium borohydride or lithium aluminium hydride in an organic solvent such as benzene, toluene, ether, dioxane, tetrahydrofuran, dimethylformamide, dimethyl sulfoxide, sulfolane, dimethoxyethane, pyridine, acetate acid or the like or a mixed solvent thereof by a conventional method. In the present reaction, addition of a suitable amount of an alcohol such as methanol, ethanol or the like sometimes promotes the reaction.

(Process 4)

Compound (VIII) (wherein R, $R_1$, $R_2$, $R_3$, $R_4$, A, B, X and Z are as defined above) can be synthesized by reacting a compound (VII) (wherein R, $R_1$, $R_2$, $R_3$, $R_4$, A, B and X are as defined above) in an organic solvent such as benzene, toluene, dichloromethane, chloroform, ether, dioxane, tetrahydrofuran, acetonitrile, dimethylformamide, dimethyl sulfoxide, sulfolane, dimethoxyethane, pyridine, acetone, ethyl acetate or the like or a mixed solvent thereof or without solvent in the presence of sulfonyl chloride such as p-toluenesulfonyl chloride, benzenesulfonyl chloride, methanesulfonyl chloride or the like and a base such as triethylamine, dimethylaminopyridine or the like under cooling to under heating.

(Process 5)

Compound (I) (wherein R, $R_1$, $R_2$, $R_3$, $R_4$, A, B and X are as defined above) can be synthesized by reacting a compound (VIII) (wherein R, $R_1$, $R_2$, $R_3$, $R_4$, A, B, X and Z are as defined above) and 5-(4-hydroxy)benzyl-2,4-thiazolidinedione (IX) in an organic solvent such as benzene, toluene, dichloromethane, chloroform, ether, dioxane, tetrahydrofuran, acetonitrile, dimethylformamide, dimethyl sulfoxide, sulfolane, dimethoxyethane, pyridine, acetone, ethyl acetate or the like, water or a mixed solvent thereof in the presence or absence of a base such as sodium hydride, potassium hydride, sodium amide, sodium alkoxide, potassium alkoxide, triethylamine, sodium hydroxide, sodium bicarbonate, sodium carbonate or the like under cooling to under heating.

The Compound (I) of the present invention can be also synthesized by the following methods.

(Process 11)

Compound (XI) (wherein $R_{61}$ is a carboxyl-protecting group such as benzyl, and $R_3$ and $R_4$ are as defined above) can be synthesized by reacting a compound (X) (wherein $R_3$ and $R_{61}$ are as defined above), which is an aspartic acid ester compound, in an acid anhydride such as acetic anhydride, propionic anhydride or the like in the presence of a base such as pyridine, triethylamine or the like and 4-dimethylaminopyridine at room temperature to under heating, and treating with water.

(Process 12)

Compound (XII) (wherein $R_6$ is alkyl having 1 to 6 carbon atoms and $R_3$ and $R_4$ are as defined above) can be synthesized by heating a compound (XI) (wherein $R_3$, $R_4$ and $R_{61}$ are as defined above) in an acidic solvent such as hydrochloric acid to allow N-acetyl to leave, and reacting the compound in an alcohol solvent such as methanol, ethanol, propanol or the like in the presence of an acid such as hydrogen chloride for esterification.

(Process 13)

Compound (XIV) (wherein Y, $R_1$, $R_2$, $R_3$, $R_4$, $R_6$, A and B are as defined above) can be synthesized by reacting a compound (XII) (wherein $R_3$, $R_4$ and $R_6$ are as defined above) and a compound (XIII) (wherein $R_1$, $R_2$, A, B and Y are as defined above) in an organic solvent such as benzene, toluene, dichloromethane, chloroform, ether, dioxane, tetrahydrofuran, acetonitrile, dimethoxyethane, pyridine, acetone or the like or a mixed solvent thereof in the presence of a base such as triethylamine, pyridine, N-methylmorpholine or the like under cooling to room temperature.

(Process 14)

Compound (XV) (wherein $R_1$, $R_2$, $R_3$, $R_4$, $R_6$, A, B and Y are as defined above) can be synthesized by reacting a compound (XIV) (wherein $R_1$, $R_2$, $R_3$, $R_4$, $R_6$, A, B and Y are as defined above) in an organic solvent such as benzene, toluene, acetonitrile, chloroform, tetrahydrofuran or the like or without a solvent in the presence of an acidic catalyst such as sulfuric acid, p-toluenesulfonic acid or the like and a dehydrating agent such as acetic anhydride or the like at room temperature to under heating, preferably under heating.

(Process 15)

Compound (XVI) (wherein $R_1$, $R_2$, $R_3$, $R_4$, A, B and Y are as defined above) can be synthesized by reacting a compound (XV) (wherein $R_1$, $R_2$, $R_3$, $R_4$, $R_6$, A, B and Y are as defined above) in an organic solvent such as benzene, toluene, ether, dioxane, tetrahydrofuran or the like using a reducing agent such as diisobutylaluminium hydride by a conventional method.

(Process 16)

Compound (VII) (wherein R, $R_1$, $R_2$, $R_3$, $R_4$, A, B and X are as defined above) can be synthesized by reacting a compound (XVI) (wherein $R_1$, $R_2$, $R_3$, $R_4$, A, B and Y are as defined above) and a compound (XVII) (wherein R and X are as defined above) in an organic solvent such as benzene, toluene, dichloromethane, chloroform, ether, dioxane, tetrahydrofuran, acetonitrile, dimethylformamide, dimethyl sulfoxide or the like, water or a mixed solvent thereof in the presence of a base such as sodium hydride, potassium hydride, sodium amide, sodium alkoxide, potassium alkoxide, triethylamine, sodium hydroxide or the like under cooling to under heating.

The Compounds (I) of the present invention can be synthesized via Processes 4 and 5. A compound wherein R is substituted by hydroxy (e.g. R is 4-hydroxyphenyl) can be synthesized by subjecting a compound which is substituted by benzyloxy, alkyloxy or the like (e.g. 4-benzyloxyphenyl, 4-methoxyphenyl) to hydrolysis under acidic conditions.

Further, a compound (VII) can be synthesizeds by the following processes.

(Process 17)

Compound (XIX) (wherein R, $R_1$, $R_2$, $R_3$, $R_4$, $R_6$, A, B and X are as defined above) can be synthesized by reacting a compound (XII) (wherein $R_3$, $R_4$ and $R_6$ are as defined above) and a compound (XVIII) (wherein R, $R_1$, $R_2$, A, B, X and Y are as defined above) in an organic solvent such as benzene, toluene, dichloromethane, chloroform, ether, dioxane, tetrahydrofuran, acetonitrile, dimethoxyethane, pyridine, acetone or the like or a mixed solvent thereof in the presence of a base such as triethylamine, pyridine, N-methylmorpholine or the like under cooling to room temperature.

(Process 18)

Compound (XX) (wherein R, $R_1$, $R_2$, $R_3$, $R_4$, $R_6$, A, B and X are as defined above) can be synthesized by reacting a compound (XIX) (wherein R, $R_1$, $R_2$, $R_3$, $R_4$, $R_6$, A, B and X are as defined above) in an organic solvent such as benzene, toluene, acetonitrile, chloroform, tetrahydrofuran or the like or without a solvent in the presence of an acidic catalyst such as sulfuric acid, p-toluenesulfonic acid or the like and a dehydrating agent such as acetic anhydride or the like at room temperature to under heating, preferably under heating.

(Process 19)

Compound (VII) (wherein R, $R_1$, $R_2$, $R_3$, $R_4$, A, B and X are as defined above) can be synthesized by reacting a compound (XX) (wherein R, $R_1$, $R_2$, $R_3$, $R_4$, $R_6$, A, B and X are as defined above) in an organic solvent such as benzene, toluene, ether, dioxane, tetrahydrofuran or the like using a reducing agent such as diisobutylaluminium hydride by a conventional method.

Compound (XIX) can be also synthesized by the following processes.

(Process 20)

Compound (XIX) (wherein R, $R_1$, $R_2$, $R_3$, $R_4$, $R_6$, A, B and X are as defined above) can be synthesized by reacting a compound (XIV) (wherein $R_1$, $R_2$, $R_3$, $R_4$, $R_6$, A, B and Y are as defined above) and a compound (XVII) (wherein R and X are as defined above) in an organic solvent such as benzene, toluene, dichloromethane, chloroform, ether, dioxane, tetrahydrofuran, acetonitrile, dimethylformamide, dimethyl sulfoxide or the like, water or a mixed solvent thereof in the presence of a base such as sodium hydride, potassium hydride, sodium amide, sodium alkoxide, potassium alkoxide, triethylamine, sodium hydroxide or the like under cooling to under heating.

The methods described above are particularly useful when $R_3$ is hydrogen atom or lower alkyl and $R_4$ is lower alkyl.

The Compound (I) thus obtained can be isolated and purified by known separation and purification methods such as concentration, concentration under reduced pressure, extraction with solvent, crystallization, recrystallization, chromatography and so on.

Of the Compounds (I), a compound (I') wherein $R_3$ is hydroxy and $R_4$ is methyl can be synthesized by the following steps.

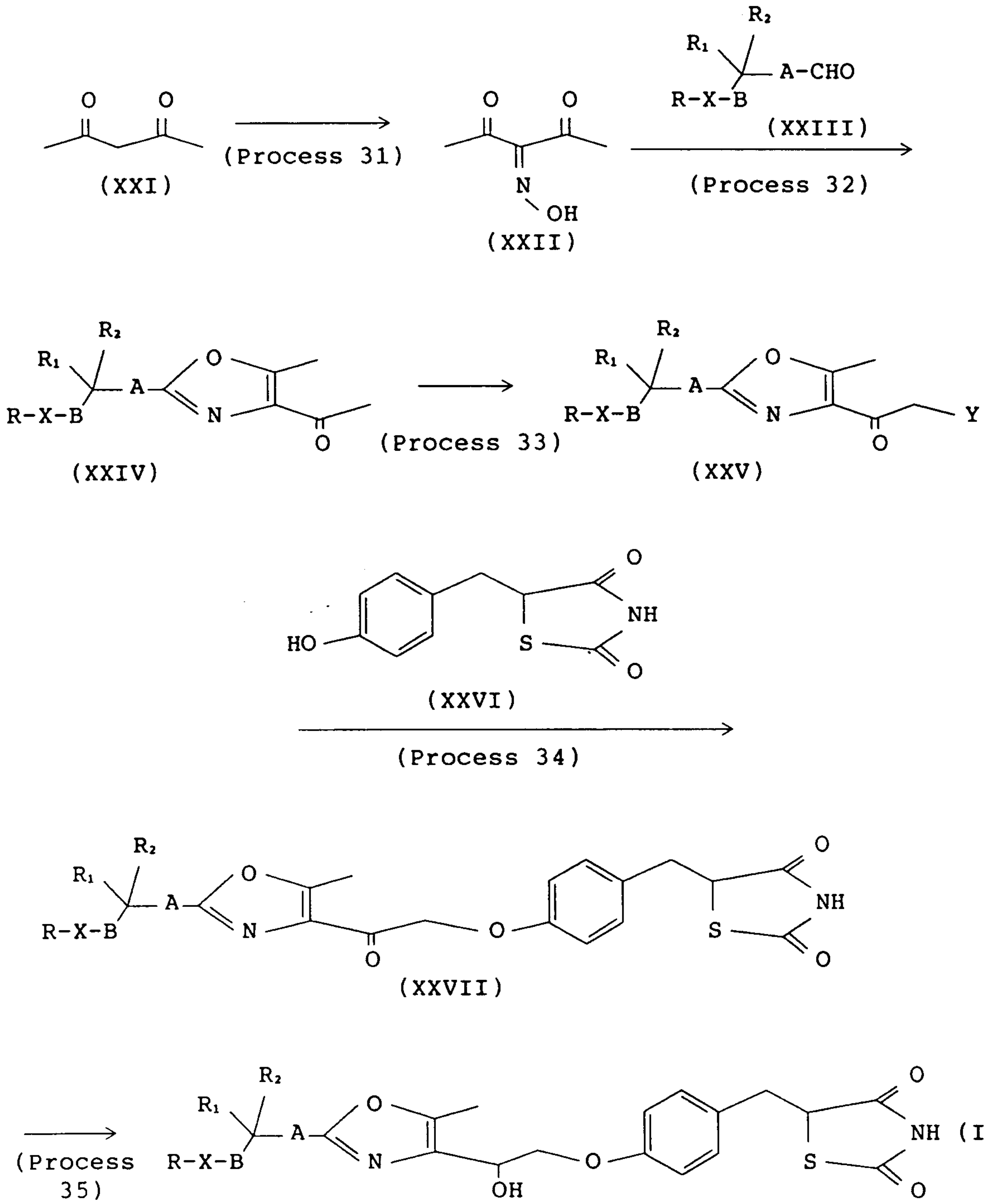


(XXI) (XXII) (XXIII) (XXIV) (XXV) (XXVI) (XXVII) (I')

(Process 31)

Compound (XXII) can be synthesized by adding an aqueous solution of sodium nitrite to acetylacetone (XXI) in an acidic solvent such as acetic acid.

(Process 32)

Compound (XXIV) (wherein R, $R_1$, $R_2$, A, B and X are as defined above) can be synthesized by reacting a compound (XXII) with a compound (XXIII) (wherein R, $R_1$, $R_2$, A, B and X are as defined above) in an organic solvent such as benzene, toluene, dichloromethane, chloroform, ether, dioxane, tetrahydrofuran, acetonitrile, dimethylformamide, dimethyl sulfoxide, sulfolane, dimethoxyethane, pyridine, acetone, ethyl acetate or the like, water or a mixed solvent thereof.

(Process 33)

Compound (XXV) (wherein R, $R_1$, $R_2$, A, B, X and Y are as defined above) can be synthesized by adding chlorine, bromine, iodide or the like to a compound (XXIV) (wherein R, $R_1$, $R_2$, A, B and X are as defined above) in an organic solvent such as chloroform, carbon tetrachloride, benzene, toluene, dichloromethane, ether, dioxane, dry tetrahydrofuran, acetonitrile, dimethylformamide, dimethyl sulfoxide, sulfolane, dimethoxyethane, pyridine, acetone, ethyl acetate or the like at 30-60 °C, or by reacting same with dibromomeldrum's acid in an organic solvent such as benzene, toluene, dichloromethane, chloroform, ether, dioxane, dry tetrahydrofuran, acetonitrile, dimethylformamide, dimethyl sulfoxide, sulfolane, dimethoxyethane, pyridine, acetone, ethyl acetate or the like.

(Process 34)

Compound (XXVII) (wherein R, $R_1$, $R_2$, A, B and X are as defined above) can be synthesized by reacting a compound (XXV) (wherein R, $R_1$, $R_2$, A, B, X and Y are as defined above) and 5-(4-hydroxy)-benzyl-2,4-thiazolidinedione (XXVI) in an organic solvent such as benzene, toluene, dichloromethane, chloroform, ether, dioxane, tetrahydrofuran, acetonitrile, dimethylformamide, dimethyl sulfoxide, sulfolane, dimethoxyethane, pyridine, acetone, ethyl acetate or the like, water or a mixed solvent thereof in the presence or absence of a base such as sodium hydride, potassium hydride, sodium amide, sodium alkoxide, potassium alkoxide, triethylamine, sodium hydroxide, sodium bicarbonate, sodium carbonate or the like under cooling to under heating.

(Process 35)

Compound (I') (wherein R, $R_1$, $R_2$, A, B and X are as defined above) can be synthesized by reducing a compound (XXVII) (wherein R, $R_1$, $R_2$, A, B and X are as defined above) in an organic solvent such as benzene, toluene, ether, dioxane, tetrahydrofuran or the like by the use of a catalyst such as sodium borohydride, lithium aluminium hydride, lithium borohydride, dibutylaluminium hydride or the like.

The present invention is explained in further detail by illustration of examples. The symbols to be used in the examples denote the following.

$^1H$ NMR proton nuclear magnetic resonance spectrometry $CDCl_3$ chloroform deuteride

Example 1

(Process 1)

Synthesis of 2-(3,5-di-tert-butyl-4-hydroxyphenylthio)-propionamide
[Formula (IV); $R_1$ is methyl, $R_2$ is hydrogen atom, m is 0, n is 0 and X is sulfur atom]

Sodium hydride (60% oily substance, 91 mg, 3.7 mmol) was washed with n-hexane and suspended in dimethylformamide (3 ml) in a stream of nitrogen at 0 °C. Thereto was gradually added 2,6-di-tert-butyl-4-mercaptophenol (751 mg, 3.15 mmol). After stirring the mixture at 0 °C for 10 minutes, 2-chloro-propionamide (339 mg, 3.15 mmol) was gradually added thereto. After stirring for 1 hour, the reaction mixture was diluted with ethyl acetate (30 ml). The diluted solution was washed with water (30 ml) and the aqueous layer was extracted with ethyl acetate (20 ml). The combined organic layer was washed with saturated brine (20 ml). After drying same over magensium sulfate, the residue was subjected to preparatory purification by silica gel column chromatography (developing solvent: chloroform:methanol = 98:2) to give 903 mg of the title compound as a white solid.

$^1H$ NMR $CDCl_3$ (300 MHz): 1.42 (s, 18H), 1.51 (d, J = 7.3, 3Hz), 3.61 (q, J = 7.3Hz, 1H), 5.29 (s, 1H), 5.40 (br, 1H), 6.42 (br, 1H), 7.25 (s, 2H)

(Process 2)

Synthesis of methyl 2-[1-(3,5-di-tert-butyl-4-hydroxyphenylthio)ethyl]-5-methyl-4-oxazolylacetate
[Formula (VI); $R_1$ is methyl, $R_2$ is hydrogen atom, $R_3$ is hydrogen atom, $R_4$ is methyl, $R_5$ is methyl, m is 0, n is 0 and X is sulfur atom]

2-(3,5-Di-tert-butyl-4-hydroxyphenylthio)propionamide (6.50 g, 21.0 mmol) obtained in Process 1 above and methyl 4-chloro-3-oxopentanoate (3.69 g, 22.4 mmol) were stirred with heating at 120 °C. The reaction mixture was dissolved in ethyl acetate (100 ml) and washed with a saturated aqueous solution of sodium hydrogencarbonate (30 ml) and saturated brine (30 ml). The organic layer was dried over magnesium sulfate, concentrated to dryness and the residue obtained was separated and purified by silica gel column chromatography (developing solvent: hexane:ethyl acetate = 8:2) to give 798 mg of the title compound as an oil (yield 9%).

$^1$H NMR $CDCl_3$ (300 MHz): 1.39 (s, 18H), 1.61 (d, J = 7.1, 3Hz), 2.24 (s, 3H), 3.43 (s, 2H), 3.68 (s, 3H), 4.19 (q, J = 7.1Hz, 1H), 5.27 (s, 1H), 7.16 (s, 2H)

(Process 3)

Synthesis of 2-[2-[1-(3,5-di-tert-butyl-4-hydroxyphenylthio)ethyl]-5-methyl-4-oxazolyl]ethanol
[Formula (VII); $R_1$ is methyl, $R_2$ is hydrogen atom, $R_3$ is hydrogen atom, $R_4$ is methyl, m is 0 and n is 0]

Lithium borohydride (44 mg, 20 mmol) was suspended in ether (3 ml) in a stream of nitrogen and the suspension was cooled to 0 °C . Then, a solution of methyl 2-[1-(3,5-di-tert-butyl-4-hydroxyphenylthio)ethyl]-5-methyl-4-oxazolylacetate (556 mg, 1.32 mmol) obtained in above Process 2 and methanol (64 mg, 2.00 mmol) in ether (6 ml) was dropwise added thereto. After stirring the mixture under reflux for 1 hour, the mixture was cooled to room temperature and water (20 ml) and ethyl acetate (50 ml) were added thereto to allow partition. The aqueous layer was extracted with ethyl acetate (20 ml). The organic layer and the extract were combined, washed with diluted hydrochloric acid (10 ml), saturated sodium hydrogencarbonate (20 ml) and saturated brine (5 ml) and dried over magnesium sulfate. After concentration, the residue was separated and purified by silica gel column chromatography (developing solvent: hexane:ethyl acetate = 6:4) to give 447 mg of the title compound as a white solid (yield 86%).

$^1$H NMR $CDCl_3$ (300 MHz): 1.39 (s, 18H), 1.61 (d, J = 7.2, 3Hz), 2.21 (s, 3H), 2.57 (t, J = 5.7Hz, 2H), 3.77 (t, J = 5.7Hz, 2H), 4.19 (q, J = 7.2Hz, 1H), 5.30 (s, 1H), 7.16 (s, 2H)

(Process 4)

Synthesis of 2-[2-[1-(3,5-di-tert-butyl-4-hydroxyphenylthio)ethyl]-5-methyl-4-oxazolyl]ethyl p-toluenesulfonate [Formula (VIII); $R_1$ is methyl, $R_2$ is hydrogen atom, $R_3$ is hydrogen atom, $R_4$ is methyl, Z is p-toluenesulfonyloxy, m is 0 and n is 0]

2-[2-[1-(3,5-Di-tert-butyl-4-hydroxyphenylthio)ethyl]-5-methyl-4-oxazolyl]ethanol (505 mg, 1.29 mmol) obtained in the above Process 3 was dissolved in dichloromethane and thereto were added triethylamine (157 mg, 1.55 mmol) and 4-dimethylaminopyridine (20 mg) in a stream of nitrogen at 0 °C and the mixture was stirred. Ten minutes later, tosyl chloride was added thereto and the temperature of the mixture was raised to room temperature, followed by stirring overnight. The reaction mixture was concentrated, dissolved in ethyl acetate (30 ml), washed with water (20 ml) and the aqueous layer was extracted with ethyl acetate (10 ml). The organic layer and the extact were combined and washed with diluted hydrochloric acid (10 ml), sodium hydrogencarbonate (10 ml) and saturated brine (10 ml). The organic layer was dried over magnesium sulfate and concentrated to dryness to give 700 mg of the title compound as a white solid (yield 100%).

$^1$H NMR $CDCl_3$ (300 MHz): 1.37 (s, 18H), 1.56 (d, J = 7.1, 3Hz), 2.19 (s, 3H), 2.43 (s, 3H), 2.74 (t, J = 6.8Hz, 2H), 4.11 (q, J = 7.1Hz, 1H), 4.17 (t, J = 6.8Hz, 2H), 5.27 (s, 1H), 7.14 (s, 2H) 7.31 (d, J = 8.2Hz, 2H), 7.72 (d, J = 8.2Hz, 2H)

(Process 5)

Synthesis of 5-[4-[2-[2-[1-(3,5-di-tert-butyl-4-hydroxyphenylthio)ethyl-5-methyl-4-oxazolyl]ethoxy]benzyl]-2,4-thiazolidinedione
[Formula (I); $R_1$ is methyl, $R_2$ is hydrogen atom, $R_3$ is hydrogen atom, $R_4$ is methyl, m is 0 and n is 0]

Sodium hydride (60% oily substance, 62 mg, 2.58 mmol) was washed with n-hexane (2 ml× 2) in a stream of nirogen and suspended in dimethylformamide (3 ml). Thereto was added 5-(p-hydroxybenzyl)-thiazolidine 2,4-dione (287 mg, 1.29 mmol) while stirring at 0 °C. After stirring the mixture for 10 minutes, a solution of 2-[2-[1-(3,5-di-tert-butyl-4-hydroxyphenylthio)ethyl]-5-methyl-4-oxazolyl]ethyl p-toluenesulfonate

(640 mg, 1.17 mmol) obtained in the above Process 4 in dimethylformamide (2 ml) was dropwise added. After stirring at room temperature for 3 hours, ethyl acetate (50 ml) was added to the reaction mixture and the mixture was washed with diluted hydrochloric acid (30 ml). The organic layer was washed with sodium hydrogencarbonate (30 ml) and saturated brine (30 ml), and dried over magensium sulfate. After concentration, the residue was separated and purified by silica gel column chromatography (developing solvent: hexane:ethyl acetate = 7:3) to give 321 mg of the title compound as a pale-white solid (yield 46%).

$^1H$ NMR $CDCl_3$ (300 MHz): 1.38 (s, 18H), 1.61 (d, J = 7.1, 3Hz), 2.25 (s, 3H), 2.84 (t, J = 6.8Hz, 2H), 3.10 (dd, J = 9.5, 11.4Hz, 1H), 3.43 (dd, J = 4.0, 11.4Hz, 1H), 4.10 (t, J = 6.8Hz, 2H), 4.49 (dd, J = 9.5, 4.0Hz, 1H), 5.27 (s, 1H), 6.81 (d, J = 8.6Hz, 2H), 7.11 (d, J = 8.6Hz, 2H), 7.13 (s, 2H), 7.82 (br, 1H) mp 72.8 - 74.0 °C

Example 2

(Process 1)

Synthesis of (3,5-di-tert-butyl-4-hydroxyphenylthio)acetamide
[Formula (IV); $R_1$ is hydrogen atom, $R_2$ is hydrogen atom, m is 0 and n is 0]

In the same manner as in Example 1, Process 1 except chloroacetamide in place of 2-chloropropionamide, the title compound was obtained.

(Process 2)

Synthesis of methyl [2-(3,5-di-tert-butyl-4-hydroxyphenylthio)methyl]-5-methyl-4-oxazolylacetate
[Formula (VI); $R_1$ is hydrogen atom, $R_2$ is hydrogen atom, $R_3$ is hydrogen atom, $R_4$ is methyl, $R_5$ is methyl, m is 0 and n is 0]

In the same manner as in Example 1, Process 2, the title compound was obtained by the use of 2-(3,5-di-tert-butyl-4-hydroxyphenylthio)acetamide obtained in the above Process 1.

(Process 3)

Synthesis of 2-[2-(3,5-di-tert-butyl-4-hydroxyphenylthio)methyl-5-methyl-4-oxazolyl]ethanol
[Formula (VII); $R_1$ is hydrogen atom, $R_2$ is hydrogen atom, $R_3$ is hydrogen atom, $R_4$ is methyl, m is 0 and n is 0]

In the same manner as in Example 1, Process 3, the title compound was obtained by the use of methyl [2-(3,5-di-tert-butyl-4-hydroxyphenylthio)methyl]-5-methyl-4-oxazolylacetate obtained in the above Process 2.

(Process 4)

Synthesis of [2-[2-(3,5-di-tert-butyl-4-hydroxyphenylthio)methyl]-5-methyl-4-oxazolyl]ethyl p-toluenesulfonate
[Formula (VIII); $R_1$ is hydrogen atom, $R_2$ is hydrogen atom, $R_3$ is hydrogen atom, $R_4$ is methyl, Z is p-toluenesulfonyloxy, m is 0 and n is 0]

In the same manner as in Example 1, Process 4, the title compound was obtained by the use of 2-[2-(3,5-di-tert-butyl-4-hydroxyphenylthio)methyl-5-methyl-4-oxazolyl]ethanol obtained in the above Process 3.

(Process 5)

Synthesis of 5-[4-[2-[2-[1-(3,5-di-tert-butyl-4-hydroxyphenylthio)methyl]-5-methyl-4-oxazolyl]ethoxy]benzyl]-2,4-thiazolidinedione
[Formula (I); $R_1$ is hydrogen atom, $R_2$ is hydrogen atom, $R_3$ is hydrogen atom, $R_4$ is methyl, m is 0 and n is 0]

In the same manner as in Example 1, Process 5, the title compound was obtained by the use of [2-[2-(3,5-di-tert-butyl-4-hydroxyphenylthio)methyl]-5-methyl-4-oxazolyl]ethyl p-toluenesulfonate obtained in the above Process 4.

$^1H$ NMR $CDCl_3$ (300MHz) : 1.39 (s, 18H), 2.26 (s, 3H), 2.85 (t, J=6.7Hz, 2H), 3.10 (dd, J=14.3,9.3Hz, 1H), 3.43 (dd, J=14.3,4.0Hz, 1H), 3.97 (s, 2H), 4.12 (t, J=6.7Hz, 2H), 4.49 (dd, J=9.3,4.0Hz, 1H), 5.25 (s, 1H), 6.81 (d, J=8.6Hz, 2H), 7.11 (d, J=8.6Hz, 2H), 7.24 (s, 2H), 7.93 (bs, 1H) mp 71.0 - 74.0°C

Example 3

(Process 1)

Synthesis of 2-(3,5-di-tert-butyl-4-hydroxyphenylthio)butyramide
[Formula (IV); $R_1$ is ethyl, $R_2$ is hydrogen atom, m is 0 and n is 0]

In the same manner as in Example 1, Process 1, the title compound was obtained by the use of 2-chlorobutyramide in place of 2-chloropropionamide.

(Process 2)

Synthesis of methyl 2-[1-(3,5-di-tert-butyl-4-hydroxyphenylthio)propyl]-5-methyl-4-oxazolylacetate
[Formula (VI); $R_1$ is ethyl, $R_2$ is hydrogen atom, $R_3$ is hydrogen atom, $R_4$ is methyl, $R_5$ is methyl, m is 0 and n is 0]

In the same manner as in Example 1, Process 2, the title compound was obtained by the use of 2-(3,5-di-tert-butyl-4-hydroxyphenylthio)butyramide.

(Process 3)

Synthesis of 2-[2-[1-(3,5-di-tert-butyl-4-hydroxyphenylthio)propyl]-5-methyl-4-oxazolyl]ethanol
[Formula (VII); $R_1$ is ethyl, $R_2$ is hydrogen atom, $R_3$ is hydrogen atom, $R_4$ is methyl, m is 0 and n is 0]

In the same manner as in Example 1, Process 3, the title compound was obtained by the use of methyl 2-[1-(3,5-di-tert-butyl-4-hydroxyphenylthio)propyl]-5-methyl-4-oxazolylacetate obtained in the above Process 2.

(Process 4)

Synthesis of 2-[2-[1-(3,5-di-tert-butyl-4-hydroxyphenylthio)propyl]-5-methyl-4-oxazolyl]ethyl p-toluenesulfonate
[Formula (VIII); $R_1$ is ethyl, $R_2$ is hydrogen atom, $R_3$ is hydrogen atom, $R_4$ is methyl, Z is p-toluenesulfonyloxy, m is 0 and n is 0]

In the same manner as in Example 1, Process 4, the title compound was obtained by the use of 2-[2-[1-(3,5-di-tert-butyl-4-hydroxyphenylthio)propyl]-5-methyl-4-oxazolyl]ethanol obtained in the above Process 3.

(Process 5)

Synthesis of 5-[4-[2-[2-[1-(3,5-di-tert-butyl-4-hydroxyphenylthio)propyl]-5-methyl-4-oxazolyl]ethoxy]benzyl]-2,4-thiazolidinedione
[Formula (I); $R_1$ is ethyl, $R_2$ is hydrogen atom, $R_3$ is hydrogen atom, $R_4$ is methyl, m is 0 and n is 0]

In the same manner as in Example 1, Process 5, the title compound was obtained by the use of 2-[2-[1-(3,5-di-tert-butyl-4-hydroxyphenylthio)propyl]-5-methyl-4-oxazolyl]ethyl p-toluenesulfonate obtained in the above Process 4.

$^1H$ NMR $CDCl_3$ (300MHz) : 1.01 (t, J=7.4Hz, 3H), 1.37 (s, 18H), 2.01 (m, 2H), 2.24 (s, 3H), 2.83 (t, J=6.9Hz, 2H), 3.08 (dd, J=9.5,14.2Hz, 1H), 3.43 (dd, J=3.9,14.2Hz, 1H), 3.98 (t, J=8.2Hz, 1H), 4.09 (t, J=6.9Hz, 2H), 4.47 (dd, J=3.9,9.5Hz, 1H), 5.25 (s, H), 6.80 (d, J=8.6Hz, 2H), 7.11 (d, J=8.6Hz, 2H), 7.14 (s, 2H), 8.27 (brs, 1H) mp 68.0 - 69.4°C

Example 4

(Process 1)

Synthesis of 2-(3,5-di-tert-butyl-4-hydroxyphenylthio)isobutyramide
[Formula (IV); $R_1$ is methyl, $R_2$ is methyl, m is 0 and n is 0]

In the same manner as in Example 1, Process 1, the title compound was obtained by the use of 2-chloroisobutyramide in place of 2-chloropropionamide.

(Process 2)

Synthesis of methyl 2-[1-(3,5-di-tert-butyl-4-hydroxyphenylthio)isopropyl]-5-methyl-4-oxazolylacetate
[Formula (VI); $R_1$ is methyl, $R_2$ is methyl, $R_3$ is hydrogen atom, $R_4$ is methyl, $R_5$ is methyl, m is 0 and n is 0]

In the same manner as in Example 1, Process 2, the title compound was obtained by the use of 2-(3,5-di-tert-butyl-4-hydroxyphenylthio)isobutyramide obtained in the above Process 1.

(Process 3)

Synthesis of 2-[2-[1-(3,5-di-tert-butyl-4-hydroxyphenylthio)isopropyl]-5-methyl-4-oxazolyl]ethanol
[Formula (VII); $R_1$ is methyl, $R_2$ is methyl, $R_3$ is hydrogen atom, $R_4$ is methyl, m is 0 and n is 0]

In the same manner as in Example 1, Process 3, the title compound was obtained by the use of methyl 2-[1-(3,5-di-tert-butyl-4-hydroxyphenylthio)isopropyl]-5-methyl-4-oxazolylacetate obtained in the above Process 2.

(Process 4)

Synthesis of 2-[2-[1-(3,5-di-tert-butyl-4-hydroxyphenylthio)isopropyl]-5-methyl-4-oxazolyl]ethyl p-toluenesulfonate
[Formula (VIII); $R_1$ is methyl, $R_2$ is methyl, $R_3$ is hydrogen atom, $R_4$ is methyl, Z is p-toluenesulfonyloxy, m is 0 and n is 0]

In the same manner as in Example 1, Process 4, the title compound was obtained by the use of 2-[2-[1-(3,5-di-tert-butyl-4-hydroxyphenylthio)isopropyl]-5-methyl-4-oxazolyl]ethanol obtained in the above Process 3.

(Process 5)

Synthesis of 5-[4-[2-[2-[1-(3,5-di-tert-butyl-4-hydroxyphenylthio)isopropyl]-5-methyl-4-oxazolyl]ethoxy]-benzyl]-2,4-thiazolidinedione
[Formula (I); $R_1$ is methyl, $R_2$ is methyl, $R_3$ is hydrogen atom, $R_4$ is methyl, m is 0 and n is 0]

In the same manner as in Example 1, Process 5, the title compound was obtained by the use of 2-[2-[1-(3,5-di-tert-butyl-4-hydroxyphenylthio)isopropyl]-5-methyl-4-oxazolyl]ethyl p-toluenesulfonate obtained in the above Process 4.

$^1$H NMR $CDCl_3$ (300MHz) : 1.85 (s, 18H), 1.67 (s, 6H), 2.26 (s, 3H), 2.83 (t, J = 6.7Hz, 2H), 3.07 (dd, J = 9.4,14.2Hz, 1H), 3.44 (dd, J = 3.9,9.4Hz, 1H), 4.07 (t, J = 6.7Hz, 2H), 4.48 (dd, J = 3.9,9.4Hz, 1H), 5.30 (s, H), 6.80 (d, J = 8.6Hz, 2H), 7.03 (s, 2H), 7.11 (d, J = 8.6Hz, 2H), 8.12 (brs, 1H) mp 71.2 - 72.8 °C

Example 5

(Process 1)

Synthesis of 2-(3,5-di-tert-butyl-4-hydroxyphenylthio)-2-phenylacetamide
[Formula (IV); $R_1$ is phenyl, $R_2$ is hydrogen atom, m is 0 and n is 0]

In the same manner as in Example 1, Process 1, the title compound was obtained by the use of 2-chloro-2-phenylacetamide in place of 2-chloropropionamide.

(Process 2)

Synthesis of methyl 2-[1-(3,5-di-tert-butyl-4-hydroxyphenylthio)-1-phenylmethyl]-5-methyl-4-oxazolylacetate
[Formula (VI); $R_1$ is phenyl, $R_2$ is hydrogen atom, $R_3$ is hydrogen atom, $R_4$ is methyl, $R_5$ is methyl, m is 0 and n is 0]

In the same manner as in Example 1, Process 2, the title compound was obtained by the use of 2-(3,5-di-tert-butyl-4-hydroxyphenylthio)-2-phenylacetamide obtained in the above Process 1.

(Process 3)

Synthesis of 2-[2-[1-(3,5-di-tert-butyl-4-hydroxyphenylthio)-1-phenylmethyl]-5-methyl-4-oxazolyl]ethanol
[Formula (VII); $R_1$ is phenyl, $R_2$ is hydrogen atom, $R_3$ is hydrogen atom, $R_4$ is methyl, m is 0 and n is 0]

In the same manner as in Example 3, the title compound was obtained by the use of methyl 2-[1-(3,5-di-tert-butyl-4-hydroxyphenylthio)-1-phenylmethyl]-5-methyl-4-oxazolylacetate obtained in the above Process 2.

(Process 4)

Synthesis of 2-[2-[1-(3,5-di-tert-butyl-4-hydroxyphenylthio)-1-phenylmethyl]-5-methyl-4-oxazolyl]ethyl p-toluenesulfonate
[Formula (VIII); $R_1$ is phenyl, $R_2$ is hydrogen atom, $R_3$ is hydrogen atom, $R_4$ is methyl, Z is p-toluenesulfonyloxy, m is 0 and n is 0]

In the same manner as in Example 1, Process 4, the title compound was obtained by the use of 2-[2-[1-(3,5-di-tert-butyl-4-hydroxyphenylthio)-1-phenylmethyl]-5-methyl-4-oxazolyl]ethanol obtained in the above Process 3.

(Process 5)

Synthesis of 5-[4-[2-[2-[1-(3,5-di-tert-butyl-4-hydroxyphenylthio)-1-phenylmethyl]-5-methyl-4-oxazolyl]-ethoxy]benzyl]-2,4-thiazolidinedione
[Formula (I); $R_1$ is phenyl, $R_2$ is hydrogen atom, $R_3$ is hydrogen atom, $R_4$ is methyl, m is 0 and n is 0]

In the same manner as in Example 1, Process 5, the title compound was obtained by the use of 2-[2-[1-(3,5-di-tert-butyl-4-hydroxyphenylthio)-1-phenyl]-5-methyl-4-oxazolyl]ethyl p-toluenesulfonate obtained in the above Process 4.

$^1$H NMR $CDCl_3$ (300MHz) : 1.32 (s, 18H), 2.27 (s, 3H), 2.86 (t, J=6.8Hz, 2H), 3.06 (dd, J=9.5,14.2Hz, 1H), 3.43 (dd, J=3.9,14.2Hz, 1H), 4.11 (t, J=6.8Hz, 2H), 4.45 (dd, J=3.9,9.5Hz, 1H), 5.26 (s, H), 5.28 (s, H), 6.79 (d, J=8.6Hz, 2H), 7.07 (s, 2H), 7.09 (d, J=8.6Hz, 2H), 7.27 (m, 3H), 7.40 (m, 2H), 8.50 (brs, 1H) mp 78.0 - 79.4 °C

Example 6

(Process 11)

Synthesis of benzyl 3-acetamido-4-oxopentanoate
[Formula (XI); $R_3$ is hydrogen atom, $R_4$ is ethyl and $R_{61}$ is benzyl]

β-Benzyl L-aspartate (400 g, 1.79 mol) was suspended in triethylamine (748 ml, 5.37 mol) and acetic anhydride (676 ml, 7.16 mol) was dropwise added thereto at 0°C with stirring. After stirring for 30 minutes, 4-dimethylaminopyridine (20.0 g, 0.16 mol) was portionwise added thereto under ice-cooling. The mixture was stirred at room temperature overnight, followed by ice-cooling by the addition of ice. After the end of exothermic reaction, water (700 ml) was added thereto. A 7.5 N aqueous solution of potassium hydroxide was portionwise added thereto to adjust the pH to 9. The mixture was extracted 3 times with ethyl acetate (1 ℓ) and the organic layer was sequentially washed with 1N hydrochloric acid (1 ℓ × 2), with a saturated aqueous solution of sodium hydrogencarbonate (500 ml × 2) and with saturated brine (500 ml). The resultant mixture was dried over magnesium sulfate and concentrated to dryness to give 390 g of the title compound.

(Process 12)

Synthesis of methyl 3-amino-4-oxopentanoate hydrochloride
[Formula (XII); $R_3$ is hydrogen atom, $R_4$ is methyl and $R_6$ is methyl]

6N Hydrochloric acid (700 ml) was added to the compound (390 g, 1.50 mol) synthesized in the above Process 11 and the mixture was stirred under reflux for 2 hours. After the reaction mixture was cooled to room temperature, it was washed twice with dichloromethane (500 ml) and the aqueous layer was concentrated to dryness. Hydrochloric acid methanol (1.5 ℓ) was added thereto and the mixture was stirred under ice-cooling and the temperature of the mixture was gradually raised. The mixture was stirred at room temperature overnight. The mixture was concentrated to dryness give 247 g of a crude product. The crude product (60 g) was recrystallized from isopropanol to give 30 g of the title compound as a white solid.

(Process 13)

Synthesis of methyl 3-chloroacetamido-4-oxopentanoate
[Formula (XIV); $R_1$ is hydrogen atom, $R_2$ is hydrogen atom, $R_3$ is hydrogen atom, $R_4$ is methyl, $R_6$ is methyl and Y is chlorine atom]

The compound synthesized in the above Process 12 (247 g, 1.36 mol) was suspended in dichloromethane (1 ℓ) under ice-cooling, chloroacetyl chloride (162 ml, 2.03 mol) was added thereto, and N-methylmorpholine (522 ml, 4.75 mol) was dropwise added thereto. After stirring the mixture for 1.5 hours, the precipitate was filtered off and the organic layer was washed with a 0.5 M aqueous solution of citric acid (500 ml × 2), a saturated aqueous solution of sodium hydrogencarbonate (500 ml × 2) and saturated brine (500 ml) in order and dried over magnesium sulfate. The resultant mixture was concentrated to dryness to give 229 g of the title compound as an oil.

(Process 14)

Synthesis of methyl (2-chloromethyl-5-methyl-4-oxazolyl)acetate
[Formula (XV); $R_1$ is hydrogen atom, $R_2$ is hydrogen atom, $R_3$ is hydrogen atom, $R_4$ is methyl, $R_6$ is methyl and Y is chlorine atom]

Acetic anhydride (46.3 ml, 0.49 mol) was added to the compound (72.5 g, 0.327 mol) synthesized in the above Process 13 and the mixture was dissolved in toluene (200 ml). Thereto was dropwise added conc. sulfuric acid (7.0 ml, 0.131 mol) with stirring. The mixture was stirred while heating at 100°C. Three hours later, the mixture was cooled to room temperature and left standing to allow partition into 2 layers. The toluene layer was separated by decantation. The acetic acid layer was washed twice with toluene (50 ml). The obtained toluene layer was adjusted to pH 7-8 with a saturated aqueous solution of sodium hydrogencarbonate and partitioned. The organic layer was washed with saturated brine (100 ml). After

drying over magnesium sulfate, the residue was concentrated to dryness to give 35.0 g of the title compound as an oil.

(Process 15)

Synthesis of 2-(2-chloromethyl-5-methyl-4-oxazolyl)ethanol
[Formula (XVI); $R_1$ is hydrogen atom, $R_2$ is hydrogen atom, $R_3$ is hydrogen atom, $R_4$ is methyl and Y is chlorine atom]

A solution of the compound (35.0 g, 0.172 mol) synthesized in the above Process 14 in toluene (200 ml) was dropwise added to a solution of diisobutyl aluminium hydride in toluene (1.02M)(506 ml, 0.516 mol) at 0 °C in a stream of nitrogen and 2 hours later, methanol (100 ml) was dropwise added thereto. Then, 2N hydrochloric acid (700 ml) was added to the obtained gel-like reaction mixture for dissolution and the mixture was extracted 4 times with ethyl acetate (500 ml). The extracted organic layers were combined and washed with saturated brine (200 ml) and dried over magnesium sulfate. By concentration to dryness, 16.7 g of the title compound was obtained as an oil.

(Process 16)

Synthesis of 2-[5-methyl-2-(4-methoxyphenylthio)methyl-4-oxazolyl]ethanol
[Formula (VII); $R_1$ is hydrogen atom, $R_2$ is hydrogen atom, $R_3$ is hydrogen atom, $R_4$ is methyl and X is sulfur atom]

Sodium hydride (60% oily substance, 388 mg, 9.70 mmol) was washed twice with n-hexane (2 ml) at 0 °C in a stream of nitrogen and thereto was added dimethylformamide (10 ml). A solution of 4-methoxythiophenol (1.36 g, 9.70 mmol) in dimethylformamide (15 ml) was dropwise added to this supension. After stirring for 10 minutes, a solution of the compound (1.70 g, 9.68 mmol) synthesized in the above Process 15 in dimethylformamide (10 ml) was dropwise added thereto. After stirring the mixture for 1 hour under cooling, ethyl acetate (100 ml) was added to the reaction mixture to dilute same and the resultant mixture was washed with water (100 ml × 2) and saturated brine (50 ml). The resultant mixture was dried over magnesium sulfate and concentrated to dryness to give 2.77 g of the title compound.

(Process 4)

Synthesis of 2-[5-methyl-2-(4-methoxyphenylthio)methyl-4-oxazolyl]-1-ethyltosylate
[Formula (VIII); $R_1$ is hydrogen atom, $R_2$ is hydrogen atom, $R_3$ is hydrogen atom, $R_4$ is methyl, X is sulfur atom and Z is p-toluenesulfonyloxy]

Triethylamine (1.39 ml, 10.0 mmol) was added to a solution (15 ml) of the compound (2.70 g, 9.68 mmol) synthesized in the above Process 16 in dichloromethane and thereto was gradually added p-toluenesulfonyl chloride (1.91 g, 10.0 mmol) at 0 °C. After stirring for 6 hours, ethyl acetate (150 ml) was added thereto to dilute same and the mixture was neutralized with diluted hydrochloric acid. The mixture was partitioned and the organic layer was washed with water (50 ml), saturated sodium hydrogencarbonate (100 ml) and saturated brine (50 ml) in order. The resultant mixture was dried over magnesium sulfate and concentrated to dryness to give 3.98 g of the title compound.

(Process 5)

Synthesis of 5-[4-[2-[5-methyl-2-(4-methoxyphenylthio)methyl-4-oxazolyl]ethoxy]benzyl]-2,4-thiazolidinedione
[Formula (I); $R_1$ is hydrogen atom, $R_2$ is hydrogen atom, $R_3$ is hydrogen atom, $R_4$ is methyl and X is sulfur atom]

Sodium hydride (60% oily substance, 800 mg, 20.0 mmol) was washed twice with n-hexane (4 ml) in a stream of nitrogen and thereto was added dimethylformamide (10 ml) and the mixture was cooled to 0 °C. A solution of 5-(4-hydroxybenzyl)-2,4-thiazolidinedione (2.23 g, 10.0 mmol) was gradually added thereto with stirring. After stirring for 10 minutes, a solution (10 ml) of the compound (3.98 g, 18.0 mmol) synthesized in the above Process 7 in dimethylformamide was dropwise added thereto. The mixture was raised to room

temperature and stirred overnight. Then, ethyl acetate (100 ml) was added thereto and neutralized with diluted hydrochloric acid. Water (100 ml) was added thereto to allow partition and the organic layer was washed with brine (100 ml) and dried over magnesium sulfate. After concentration to dryness, the residue was separated and purified by silica gel column chromatography (developing solvent: hexane:ethyl acetate = 1:1) to give 1.21 g of the title compound.

Example 7

Synthesis of 5-[4-[2-[2-(4-hydroxyphenylthio)methyl-5-methyl-4-oxazolyl]ethoxy]benzyl]-2,4-thiazolidinedione [Formula (I); $R_1$ is hydrogen atom, $R_2$ is hydrogen atom, $R_3$ is hydrogen atom, $R_4$ is methyl and X is sulfur atom]

Trifluoroacetate (25 ml), thioanisole (8.4 ml) and trifluoromethanesulfonic acid (6.8 ml) were added to the compound (360 mg, 0.74 mol) synthesized in the aforementioned Example 6, Process 5, and the mixture was stirred at room temperature overnight. After the solvent was distilled away, water was added to the mixture. The mixture was extracted with dichloromethane and dried over magnesium sulfate. After concentration to dryness, the residue was separated and purified by silica gel column chromatography (developing solvent: dichloromethane:methanol = 98:2) to give 176 mg of the title compound.

Examples 8 - 24

In the same manner as in Example 6 and Example 7, the compounds of the Tables 1 through 4 were obtained.

Table 1

| Ex. | Compound | mp | $^1H$ NMR ($CDCl_3$ δ value) |
|---|---|---|---|
| 6 | 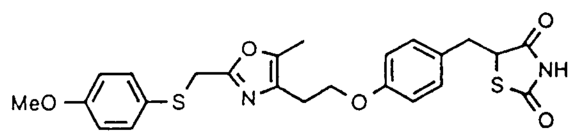 | 114.6 ~ 116.2 ℃ | 2.25(s,3H),2.83(t,J=6.6Hz,2H),3.11(dd,J=14.2,9.2Hz,1H),3.41(dd,J=14.2,3.9Hz,1H),3.76(s,3H),3.96(s,2H),4.10(t,J=6.6Hz,2H),4.48(dd,J=9.2,3.9Hz,1H),6.75(d,J=8.7Hz,2H),6.82(d,J=8.6Hz,2H),7.12(d,J=8.6Hz,2H),7.30(d,J=8.7Hz,2H),8.2(bs,1H) |
| 7 | 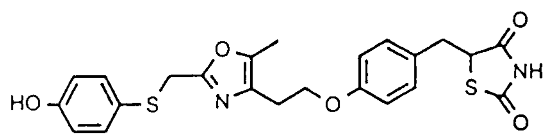 | 73.0 ~ 75.0 ℃ | 2.29(s,3H),2.81(t,J=6.3Hz,3H),3.18(dd,J=14.2,8.1Hz,1H),3.33(dd,J=14.2,4.3Hz,1H),3.95(s,2H),4.04(t,J=6.3Hz,2H),4.50(t,J=8.1,4.3Hz),6.46(d,J=8.6Hz,2H),6.78(d,J=8.6Hz,2H),7.13(m,4H),8.73(bs,1H) |
| 8 | 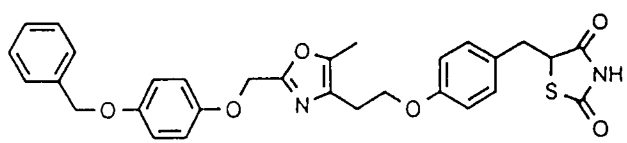 | 111.0 ~ 112.7 ℃ | 2.29(s,3H),2.90(t,J=6.5Hz,2H),3.10(dd,J=14.2,9.3Hz,1H),3.27(dd,J=9.3,4.0Hz,1H),4.18(t,J=6.5Hz,2H),4.48(dd,J=9.3,4.0Hz,1H),4.98(s,2H),5.01(s,2H),6.82(d,J=8.6Hz,2H),6.90(m,4H),7.11(dd,J=8.6Hz,2H),7.30-7.50(m,5H),7.95(bs,1H) |
| 9 | 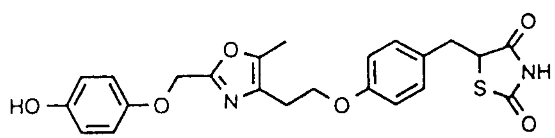 | | 2.31(s,3H),2.90(t,J=6.4Hz,2H),3.15(dd,J=14.2,8.4Hz,1H),3.33(dd,J=14.2,4.1Hz,1H),4.13(t,J=6.4Hz,2H),4.48(dd,J=8.4,4.1Hz,1H),4.99(s,2H),6.65(d,J=6.7Hz,2H),6.75(d,J=8.6Hz,2H),6.80(d,J=6.7Hz,2H),7.08(d,J=8.6Hz,2H),8.77(bs,1H) |
| 10 | 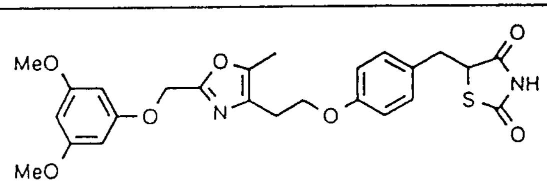 | 54.5 ~ 56.8 ℃ | 2.30(s,3H),2.91(t,J=6.5Hz,2H),3.03(dd,J=14.2,9.5Hz,1H),3.42(dd,J=14.2,3.9Hz,1H),3.75(s,6H),4.17(t,J=6.5Hz,2H),4.46(dd,J=9.5,3.9Hz,1H),5.01(s,2H),6.12(t,J=2.1Hz,1H),6.19(d,J=2.1Hz,2H),6.81(d,J=8.6Hz,2H),7.10(d,J=8.6Hz,2H),9.14(bs,1H) |

Table 2

| Ex. | Compound | mp | $^1H$ NMR ($CDCl_3$ δ value) |
|---|---|---|---|
| 11 | 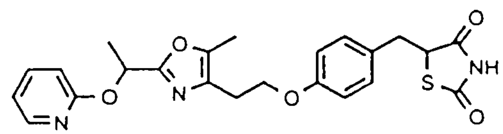 | 48.9 ~ 49.8 ℃ | 1.71,1.72(d,J=6.6Hz,3H),2.23(s,3H),2.88(t,J=7.0Hz, 3H),3.08(m,1H),3.42(m,1H),4.15(m,2H),4.46(m,2H), 6.30(q,J=6.6Hz,1H),6.81(m,4H),7.08,7.11(d,J=8.7Hz, 2H),7.56(m,1H),8.11(m,1H),9.05(brs,1H) |
| 12 | 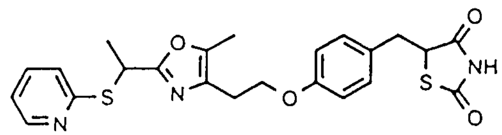 | 46.8 ~ 49.0 ℃ | 1.78,1.79(d,J=7.2,7.1Hz,3H),2.20,2.23(s,3H),2.85,2.86 (t,J=6.5,6.6Hz,3H),3.0-3.2(m,1H),3.3-3.5(m,1H), 4.1-4.2(m,3H),4.45-4.52(m,1H),5.2-5.3(m,1H), 6.75-6.85(m,2H),6.95-7.0(m,1H),7.05-7.15(m,2H), 7.15-7.22(m,1H),7.42-7.50(m,1H) |
| 13 | 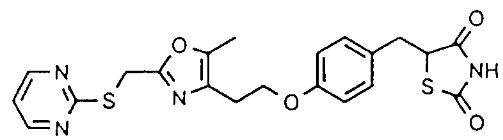 | 140.0 ~ 141.5 ℃ | 2.23(s,3H),2.86(t,J=6.6Hz,2H),3.09(dd,J=9.2,14.2Hz, 1H),3.41(dd,J=3.9,14.2Hz,1H),4.14(t,J=6.6Hz,2H),4.47 (dd,J=3.9,9.2Hz,1H),4.50(s,2H),6.79(d,J=8.7Hz,2H), 6.99(t,J=4.8Hz,1H),7.09(d,J=8.7Hz,2H),8.52(d,J=4.8Hz, 2H),9.30(brs,1H) |
| 14 | 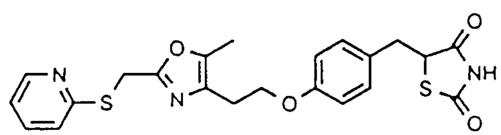 | 120.8 ~ 121.5 ℃ | 2.21(s,3H),2.85(t,J=6.6Hz,2H),3.11(dd,J=14.2,9.1Hz, 1H),3.66(dd,J=14.2,3.9Hz,1H),4.15(m,2H),4.49(dd,J= 9.2,3.9Hz,1H),4.50(s,2H),6.80(d,J=8.6Hz,2H),7.00(m, 1H),7.10(d,J=8.6Hz,2H),7.23(m,1H),7.49(m,1H),8.44 (m,1H),8.45(bs,1H) |
| 15 | 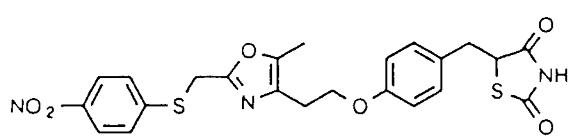 | 62.2 ~ 63.0 ℃ | 2.26(s,3H),2.86(t,J=6.2Hz,2H),3.10(dd,J=9.3,14.1Hz, 1H),3.42(dd,J=4.1,14.1Hz,1H),4.12(t,J=6.2Hz,2H),4.22 (s,2H),4.49(dd,J=4.1,9.3Hz,1H),6.78(d,J=8.4Hz,2H), 7.11(d,J=8.4Hz,2H),7.46(d,J=8.8Hz,2H),8.07(d,J=8.8 Hz,2H),8.45(brs,1H) |

Table 3

| Ex. | Compound | mp | $^1H$ NMR ($CDCl_3$ δ value) |
|---|---|---|---|
| 16 | 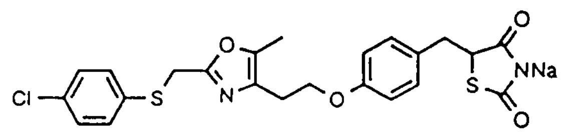 | 210 ℃ (decomp. ) | 2.25(s,3H),2.86(t,J=6.5Hz,2H),3.09(dd,J=9.2,14.2Hz,1H),3.43(dd,J=4.0,14.2Hz,1H),4.04(s,2H),4.10(t,J=6.5Hz,2H),4.48(dd,J=4.0,9.2Hz,1H),6.81(d,J=8.6Hz,2H),7.12(d,J=8.6Hz,2H),7.17(d,J=8.6Hz,2H),7.28(d,J=8.6Hz,2H),8.35(brs,1H) |
| 17 | 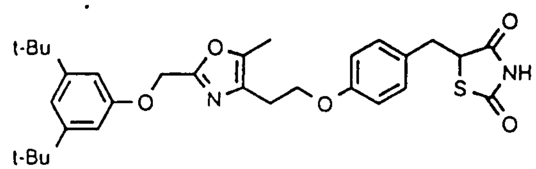 | 60.0 ~ 63.5 ℃ | 1.29(s,18H),2.30(s,3H),2.91(t,J=6.6Hz,2H),3.09(dd,J=14.1,9.5Hz,1H),3.29(dd,J=14.1,3.9Hz,1H),4.17(t,J=6.6Hz,2H),4.48(dd,J=9.5,3.9Hz,1H),5.06(s,2H),6.82(d,J=8.6Hz,2H),6.88(d,J=1.5Hz,2H),7.05(d,J=1.5Hz,1H),7.11(d,J=8.6Hz,2H),8.32(bs,1H) |
| 18 | 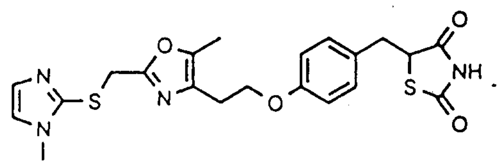 | 57.1 ~ 59.4 ℃ | 2.16(s,3H),2.81(t,J=6.2Hz,2H),3.18(dd,J=15.3,8.3Hz,1H),3.35(dd,J=15.3,4.1Hz,1H),3.44(s,3H),4.13(d,J=14.3Hz,1H),4.2(m,2H),4.22(d,J=14.3Hz,1H),4.50(dd,J=8.3,4.1Hz,1H),6.8(m,3H),7.1(m,3H) |
| 19 | 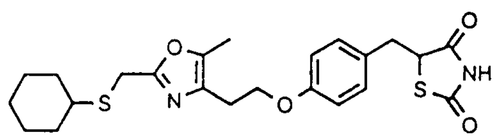 | 109.1 ~ 110.1 ℃ | 1.26(m,6H),1.71(m,2H),1.93(m,2H),2.27(s,3H),2.70(m,1H),2.87(t,J=6.6Hz,2H),3.08(dd,J=9.4,14.2Hz,1H),3.43(dd,J=4.0,14.2Hz,1H),3.74(s,2H),4.15(t,J=6.6Hz,2H),4.47(dd,J=4.0,9.4Hz,1H),6.82(d,J=8.6Hz,2H),7.11(d,J=8.6Hz,2H),8.50(brs,1H) |
| 20 | 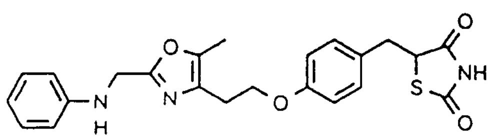 | 256.5 ~ 257.8 ℃ (decomp. ) | 2.25(s,3H),2.88(t,J=6.6Hz,2H),3.08(dd,J=9.4,14.2Hz,1H),3.41(dd,J=3.9,14.2Hz,1H),4.16(t,J=6.6Hz,2H),4.35(s,2H),4.45(dd,J=3.9,9.4Hz,1H),6.69-6.82(m,4H),7.09-7.22(m,5H),8.01(brs,1H) |

Table 4

| Ex. | Compound | mp | $^1H$ NMR ($CDCl_3$ δ value) |
|---|---|---|---|
| 21 | 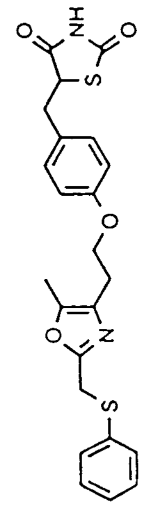 | (oily substance) | 2.24(s,3H),2.84(t,J=6.5Hz,2H),3.10(dd,J=14.2,9.2Hz,1H),3.43(dd,J=14.2,4.0Hz,1H),4.09(s,2H),4.09(t,J=6.5Hz,2H),4.46(dd,J=9.2,4.0Hz,1H),6.80(d,J=8.7Hz,2H),7.11(d,J=8.7Hz,2H),7.2(m,3H),7.4(m,2H),8.8(bs,1H) |
| 22 | 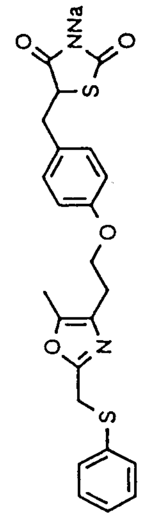 | 234.0 ~ 236.0 ℃ | 2.20(s,3H),2.60(dd,J=14.0,10.6Hz,1H),2.76(d,J=6.4Hz,2H),3.28(m,1H),4.40(m,3H),4.25(s,2H),6.76(d,J=8.6Hz,2H),7.08(d,J=8.6Hz,2H),7.1-7.4(m,5H) ($DMSO-d_6$) |
| 23 | 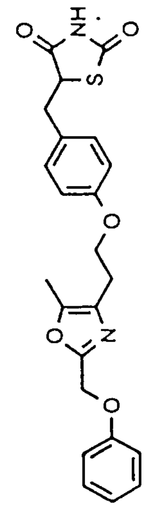 | (oily substance) | 2.30(s,3H),2.92(t,J=6.5Hz,2H),3.10(dd,J=14.2,9.3Hz,1H),3.43(dd,J=14.2,4.0Hz,1H),4.19(t,J=6.5Hz,2H),4.49(dd,J=9.3,4.0Hz,1H),5.06(s,2H),6.82(d,J=8.7Hz,2H),7.0(m,3H),7.11(d,J=8.7Hz,2H),7.3(m,2H),7.9(bs,1H) |
| 24 | 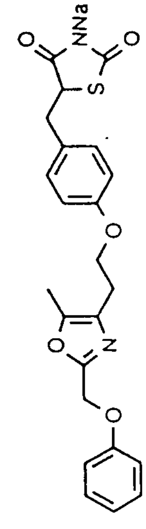 | 246.0 ~ 247.9 ℃ | 22.28(s,3H),2.61(dd,J=13.9,10.5Hz,1H),2.84(t,J=6.6Hz,2H),3.30(dd,J=13.9,3.5Hz,1H),4.06(dd,J=10.5,3.5Hz,1H),4.12(t,J=6.6Hz,2H),5.09(s,2H),6.79(d,J=8.6Hz,2H),6.9-7.1(m,3H),7.08(d,J=8.6Hz,2H),7.30(dd,J=8.6,7.4Hz,2H) ($DMSO-d_6$) |

As the intermediates (VII) in the aforementioned Example 6 through Example 24, Process 16, the compounds of Table 5 through Table 7 were obtained.

Table 5

| Ex. | Compound | $^1H$ NMR ($CDCl_3$ δvalue) |
|---|---|---|
| 1 | 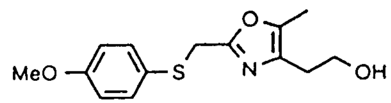 | 2.22(s,3H),2.59(t,J=5.6Hz,2H),2.7(m,1H),3.78(m,2H),3.79(s,3H),3.96(s,2H),6.8(m,2H),7.3(m,2H) |
| 2 | 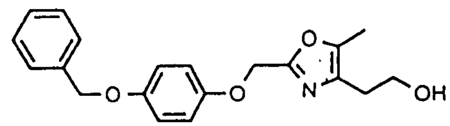 | 2.27(s,3H),2.67(t,J=5.6Hz,2H),2.7(bs,1H),3.88(t,J=5.6Hz,2H),4.98(s,2H),5.01(s,2H),6.9(m,4H),7.4(m,5H) |
| 3 | 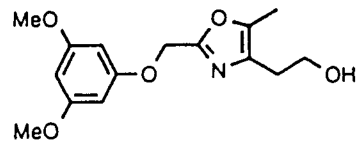 | 2.28(s,3H),2.68(t,J=5.8Hz,2H),2.7(bs,1H),3.76(s,6H),3.88(m,2H),5.00(s,2H),6.1(m,1H),6.2(m,2H) |
| 4 | 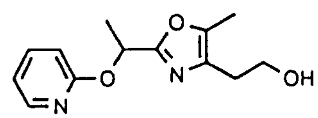 | 1.71(d,J=6.6Hz,3H),2.24(s,3H),2.65(t,J=5.7Hz,2H)3.85(t,J=5.7Hz,2H),6.29(q,J=6.6Hz,1H),6.78(d,J=8.3Hz,1H),6.87(m,1H),7.57(m,1H),8.14(dd,J=1.0,4.3Hz,1H) |
| 5 | 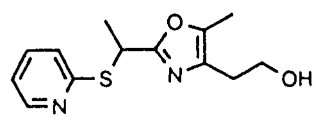 | 1.78(d,J=7.2Hz,3H),2.22(s,3H),2.62(t,J=5.6Hz,2H),3.83(m,2H),5.27(q,J=7.2Hz,1H),7.0(m,1H),7.2(m,1H),7.5(m,1H),8.5(m,1H) |

Table 6

| Ex. | Compound | $^1H$ NMR ($CDCl_3$ δ value) |
|---|---|---|
| 6 | 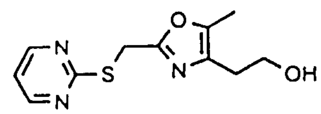 | 2.22(s,3H),2.63(t,J=5.7Hz,2H),3.84(t,J=5.7Hz,2H),4.49(s,2H),7.00(t,J=4.9Hz,1H)8.54(d,J=4.9Hz,2H) |
| 7 | 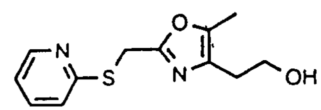 | 2.21(s,3H),2.62(t,J=5.6Hz,2H),2.8(s,1H),3.83(m,1H),4.50(s,2H),7.0(m,1H),7.2(m,1H),7.5(m,1H),8.5(m,1H) |
| 8 | 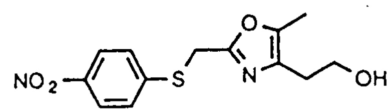 | 2.24(s,3H),2.63(t,J=5.7Hz,2H),3.84(t,J=5.7Hz,2H),4.23(s,2H),7.49(d,J=8.9Hz,2H),8.15(d,J=8.9Hz,2H) |
| 9 | 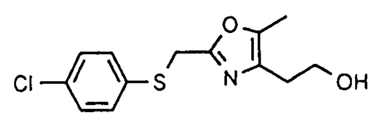 | 2.22(s,3H),2.61(t,J=5.7Hz,2H),3.81(t,J=5.7Hz,2H),4.06(s,2H),7.27(m,4H) |
| 10 | 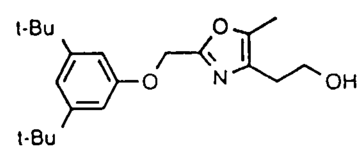 | 1.30(s,18H),2.27(s,3H),2.67(t,J=5.8Hz,2H),2.73(bs,1H),3.88(m,2H),5.06(s,2H),6.88(m,2H),7.06(m,1H) |

Table 7

| Ex. | Compound | $^1H$ NMR ($CDCl_3$ δvalue) |
|---|---|---|
| 11 | 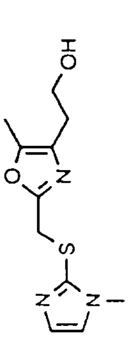 | 2.21(s,3H),2.59(t,J=5.7Hz,2H),3.58(s,3H),3.80(t,J=5.7Hz,2H),4.14(s,2H),6.96(m,1H),7.10(m,1H) |
| 12 | 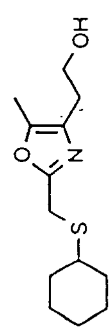 | 1.25-1.95(m,10H),2.25(s,3H),2.65(t,J=5.7Hz,2H),2.73(m,1H),3.73(s,2H),3.85(t,J=5.7Hz,2H) |
| 13 | 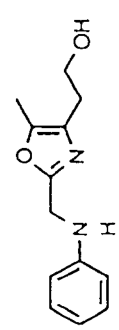 | 2.23(s,3H),2.64(t,J=5.7Hz,2H),3.86(t,J=5.7Hz,2H),4.35(s,2H),6.70(d,J=7.7Hz,2H),6.76(t,J=7.4Hz,1H),7.20(dd,J=7.4,7.7Hz,2H) |
| 14 | 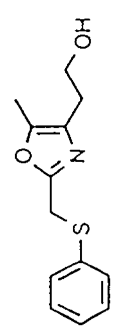 | 2.21(s,3H),2.59(t,J=5.7Hz,2H),2.7(bs,1H),3.80(m,2H),4.08(s,2H),7.3(m,3H),7.4(m,2H) |
| 15 | 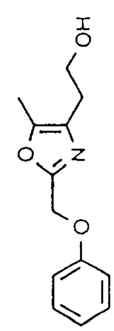 | 2.28(s,3H),2.68(t,J=5.8Hz,2H),2.7(bs,1H),3.89(m,2H),5.05(s,2H),7.0(m,3H),7.3(m,2H) |

It is needless to say that the present invention is not limited to the foregoing Examples. For example, the compounds shown in the following Tables 8 through 34 also fall within the scope of the present invention.

Table 8

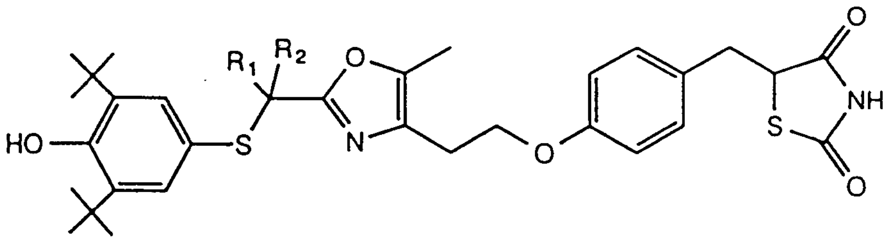

| $R_1$ | $R_2$ | $R_1$ | $R_2$ | $R_1$ | $R_2$ | $R_1$ | $R_2$ |
|---|---|---|---|---|---|---|---|
| Me | Et | Me | 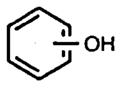 | Me | 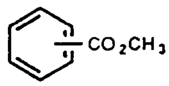 | Me | 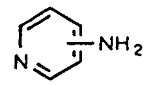 |
| 〃 | Pr | 〃 | 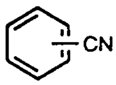 | 〃 | 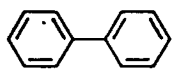 | 〃 | 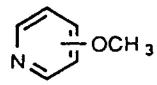 |
| 〃 | i-Pr | 〃 | 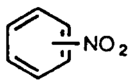 | 〃 | 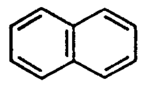 | 〃 | 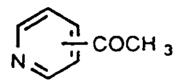 |
| 〃 | Bu | 〃 | 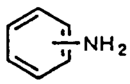 | 〃 | 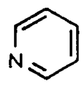 | 〃 | 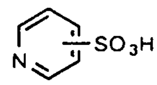 |
| 〃 | i-Bu | 〃 | 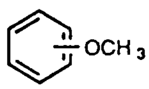 | 〃 | 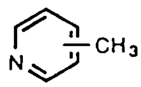 | 〃 | 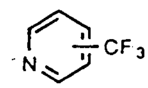 |
| 〃 | t-Bu | 〃 | 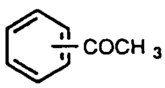 | 〃 | 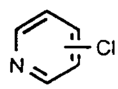 | 〃 | 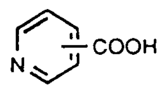 |
| 〃 | 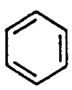 | 〃 | 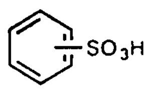 | 〃 | 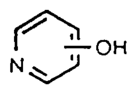 | 〃 | 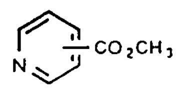 |
| 〃 | 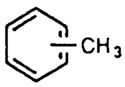 | 〃 | 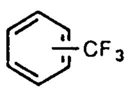 | 〃 | 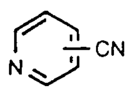 | 〃 | 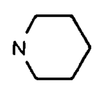 |
| 〃 | 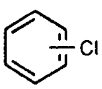 | 〃 | 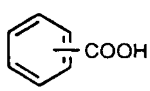 | 〃 | 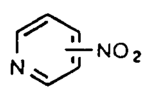 | 〃 | 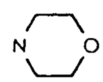 |

Table 9

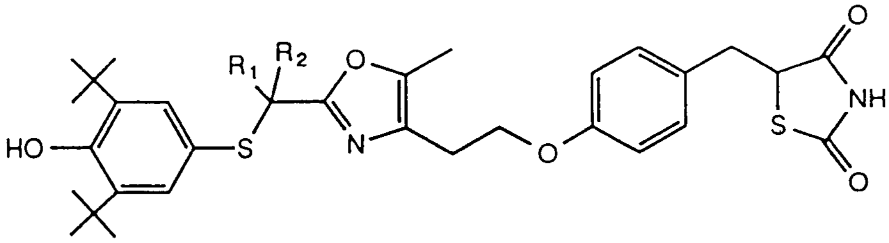

| R1 | R2 | R1 | R2 | R1 | R2 | R1 | R2 |
|---|---|---|---|---|---|---|---|
| Et | Et | Et | 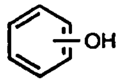 | Et | 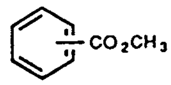 | Et | 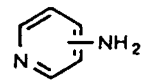 |
| 〃 | Pr | 〃 | 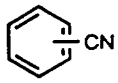 | 〃 | 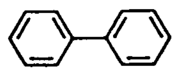 | 〃 | 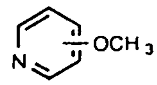 |
| 〃 | i-Pr | 〃 | 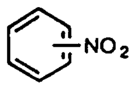 | 〃 | 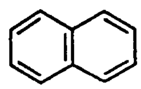 | 〃 | 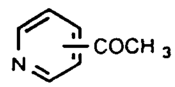 |
| 〃 | Bu | 〃 | 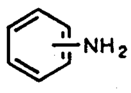 | 〃 | 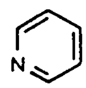 | 〃 | 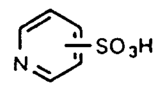 |
| 〃 | i-Bu | 〃 | 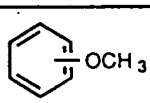 | 〃 | 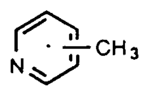 | 〃 | 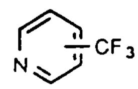 |
| 〃 | t-Bu | 〃 | 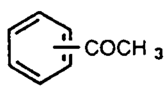 | 〃 | 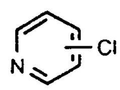 | 〃 | 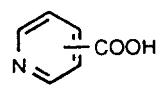 |
| 〃 | 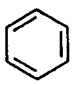 | 〃 | 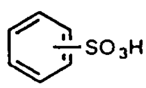 | 〃 | 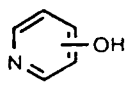 | 〃 | 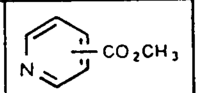 |
| 〃 | 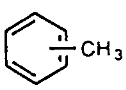 | 〃 | 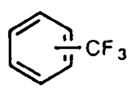 | 〃 | 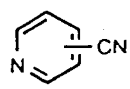 | 〃 | 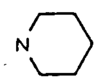 |
| 〃 | 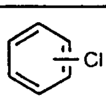 | 〃 | 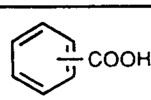 | 〃 | 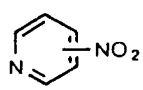 | 〃 | 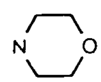 |

Table 10

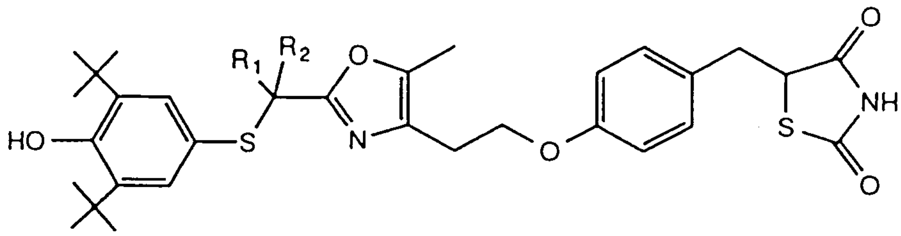

| R1 | R2 | R1 | R2 | R1 | R2 | R1 | R2 |
|---|---|---|---|---|---|---|---|
| Pr | H | Pr | 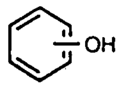 | Pr | 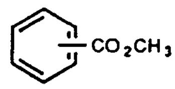 | Pr | 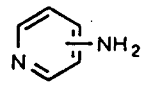 |
| 〃 | Pr | 〃 | 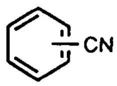 | 〃 | 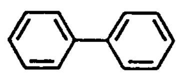 | 〃 | 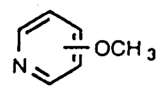 |
| 〃 | i-Pr | 〃 | 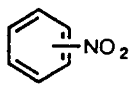 | 〃 | 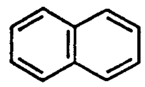 | 〃 | 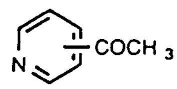 |
| 〃 | Bu | 〃 | 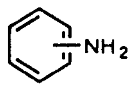 | 〃 | 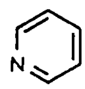 | 〃 | 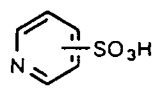 |
| 〃 | i-Bu | 〃 | 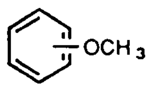 | 〃 | 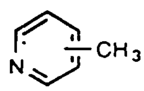 | 〃 | 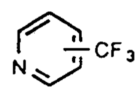 |
| 〃 | t-Bu | 〃 | 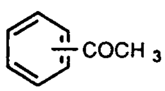 | 〃 | 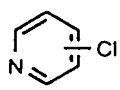 | 〃 | 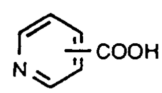 |
| 〃 | 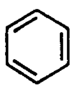 | 〃 | 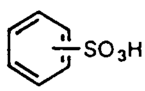 | 〃 | 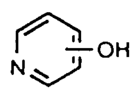 | 〃 | 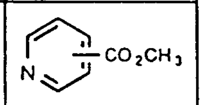 |
| 〃 | 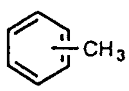 | 〃 | 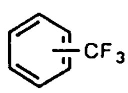 | 〃 | 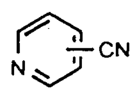 | 〃 | 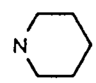 |
| 〃 | 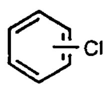 | 〃 | 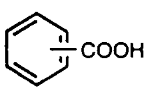 | 〃 | 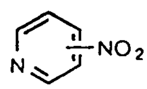 | 〃 | 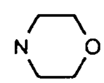 |

Table 11

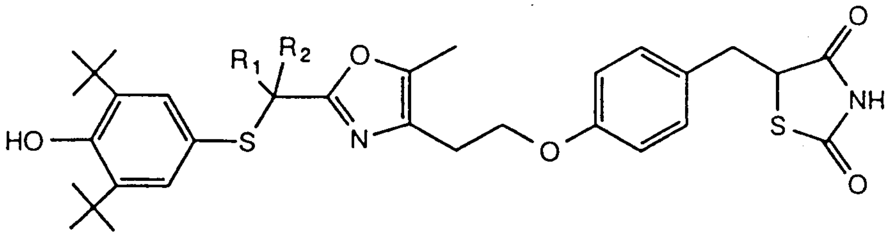

| $R_1$ | $R_2$ | $R_1$ | $R_2$ | $R_1$ | $R_2$ | $R_1$ | $R_2$ |
|---|---|---|---|---|---|---|---|
| i-Pr | H | i-Pr | 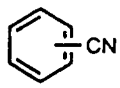 | i-Pr | 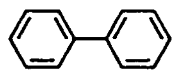 | i-Pr | 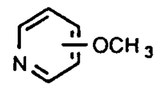 |
| 〃 | i-Pr | 〃 | 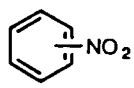 | 〃 | 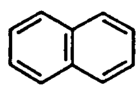 | 〃 | 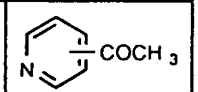 |
| 〃 | Bu | 〃 | 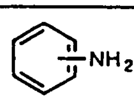 | 〃 | 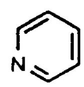 | 〃 | 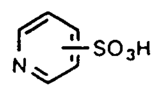 |
| 〃 | i-Bu | 〃 | 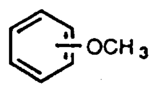 | 〃 | 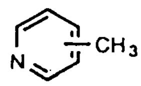 | 〃 | 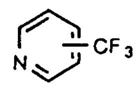 |
| 〃 | t-Bu | 〃 | 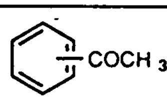 | 〃 | 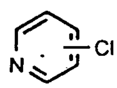 | 〃 | 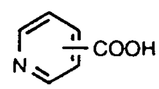 |
| 〃 | 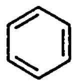 | 〃 | 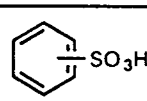 | 〃 | 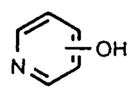 | 〃 | 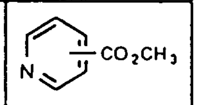 |
| 〃 | 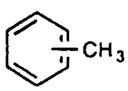 | 〃 | 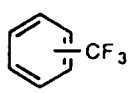 | 〃 | 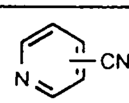 | 〃 | 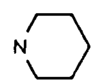 |
| 〃 | 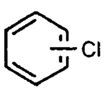 | 〃 | 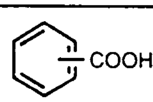 | 〃 | 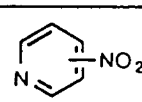 | 〃 | 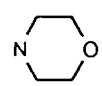 |
| 〃 | 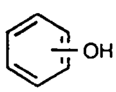 | 〃 | 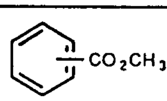 | 〃 | 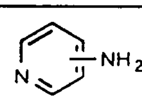 | | |

Table 12

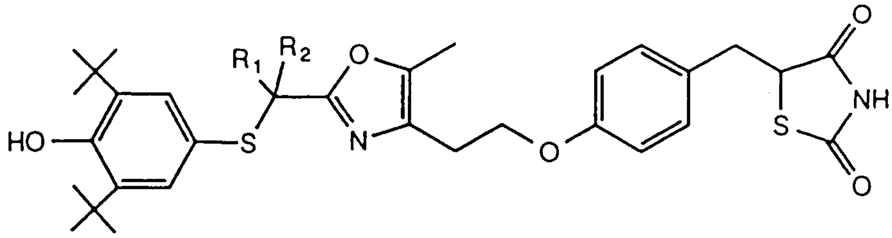

| R1 | R2 | R1 | R2 | R1 | R2 | R1 | R2 |
|---|---|---|---|---|---|---|---|
| Bu | H | Bu | 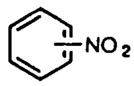 | Bu | 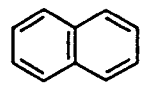 | Bu | 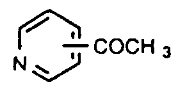 |
| 〃 | Bu | 〃 | 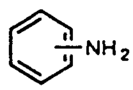 | 〃 | 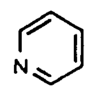 | 〃 | 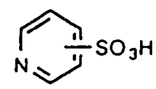 |
| 〃 | i-Bu | 〃 | 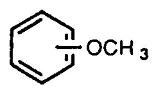 | 〃 | 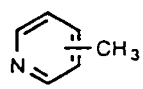 | 〃 | 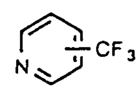 |
| 〃 | t-Bu | 〃 | 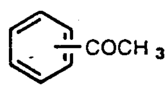 | 〃 | 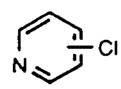 | 〃 | 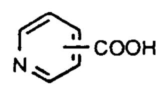 |
| 〃 | 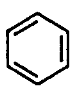 | 〃 | 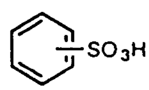 | 〃 | 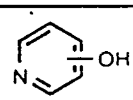 | 〃 | 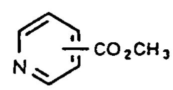 |
| 〃 | 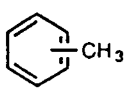 | 〃 | 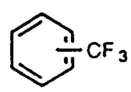 | 〃 | 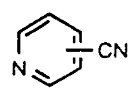 | 〃 | 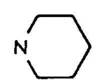 |
| 〃 | 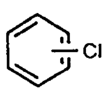 | 〃 | 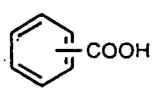 | 〃 | 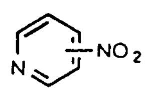 | 〃 | 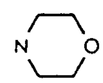 |
| 〃 | 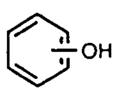 | 〃 | 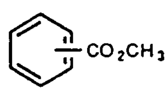 | 〃 | 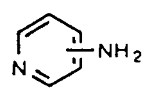 | | |
| 〃 | 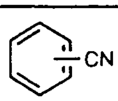 | 〃 | 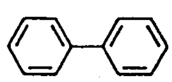 | 〃 | 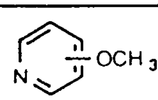 | | |

Table 13

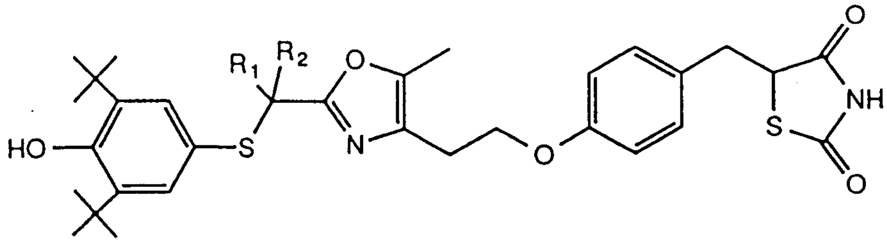

| $R_1$ | $R_2$ | $R_1$ | $R_2$ | $R_1$ | $R_2$ | $R_1$ | $R_2$ |
|---|---|---|---|---|---|---|---|
| i-Bu | H | i-Bu | 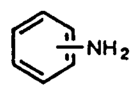 | i-Bu | 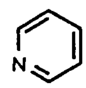 | i-Bu | 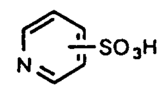 |
| 〃 | i-Bu | 〃 | 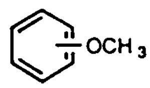 | 〃 | 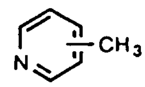 | 〃 | 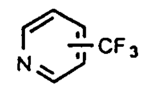 |
| 〃 | t-Bu | 〃 | 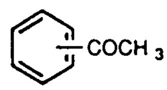 | 〃 | 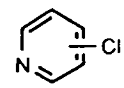 | 〃 | 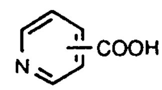 |
| 〃 | 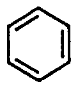 | 〃 | 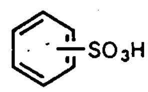 | 〃 | 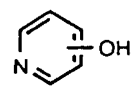 | 〃 | 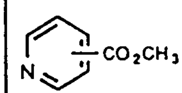 |
| 〃 | 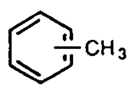 | 〃 | 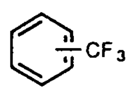 | 〃 | 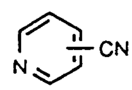 | 〃 | 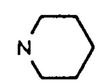 |
| 〃 | 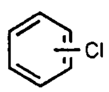 | 〃 | 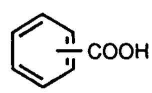 | 〃 | 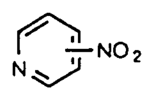 | 〃 | 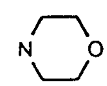 |
| 〃 | 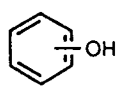 | 〃 | 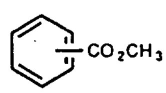 | 〃 | 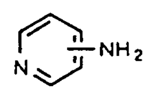 | | |
| 〃 | 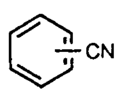 | 〃 | 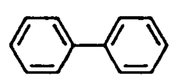 | 〃 | 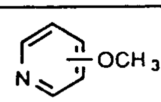 | | |
| 〃 | 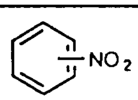 | 〃 | 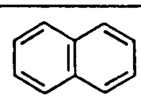 | 〃 | 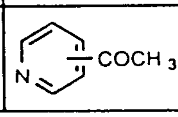 | | |

Table 14

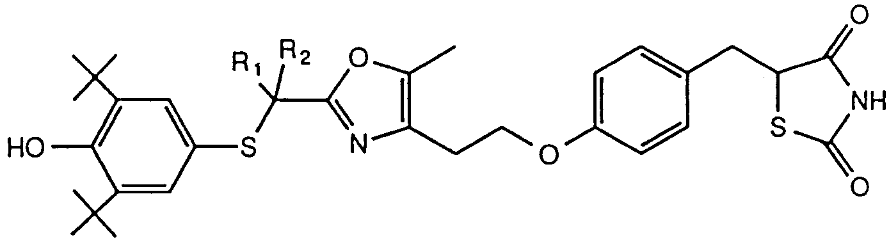

| $R_1$ | $R_2$ | $R_1$ | $R_2$ | $R_1$ | $R_2$ | $R_1$ | $R_2$ |
|---|---|---|---|---|---|---|---|
| t-Bu | H | t-Bu | 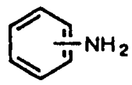 | t-Bu | 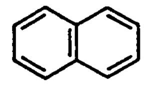 | t-Bu | 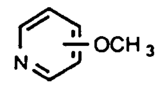 |
| 〃 | t-Bu | 〃 | 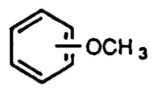 | 〃 | 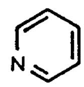 | 〃 | 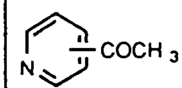 |
| 〃 | 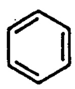 | 〃 | 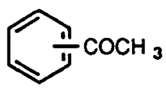 | 〃 | 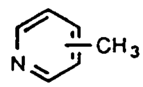 | 〃 | 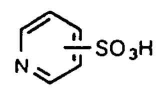 |
| 〃 | 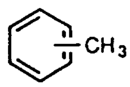 | 〃 | 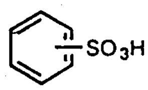 | 〃 | 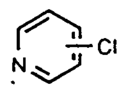 | 〃 | 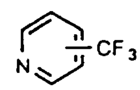 |
| 〃 | 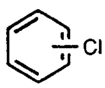 | 〃 | 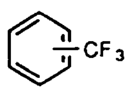 | 〃 | 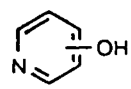 | 〃 | 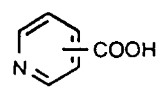 |
| 〃 | 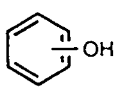 | 〃 | 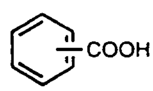 | 〃 | 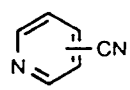 | 〃 | 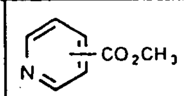 |
| 〃 | 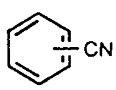 | 〃 | 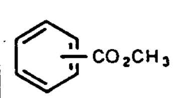 | 〃 | 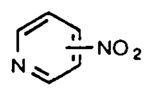 | 〃 | 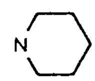 |
| 〃 | 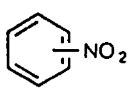 | 〃 | 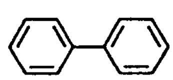 | 〃 | 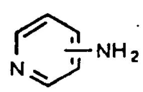 | 〃 | 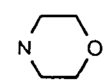 |

Table 15

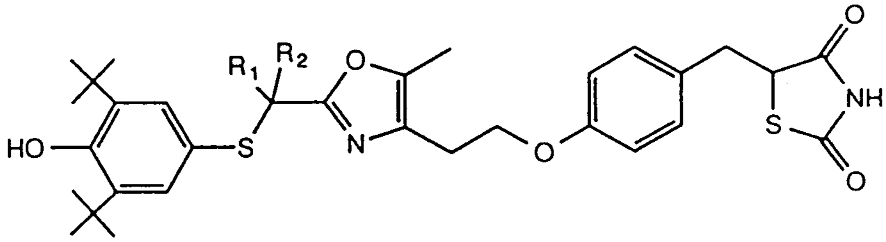

| R1 | R2 | R1 | R2 | R1 | R2 | R1 | R2 |
|---|---|---|---|---|---|---|---|
| 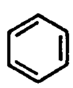 | 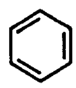 | 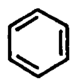 | 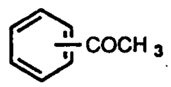 | 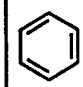 | 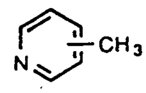 | 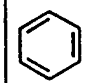 | 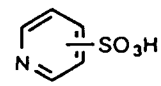 |
| 〃 | 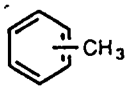 | 〃 | 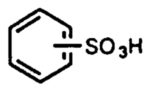 | 〃 | 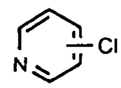 | 〃 | 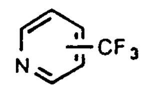 |
| 〃 | 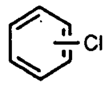 | 〃 | 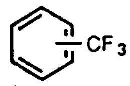 | 〃 | 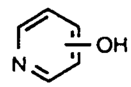 | 〃 | 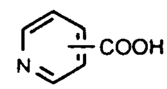 |
| 〃 | 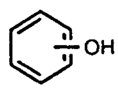 | 〃 | 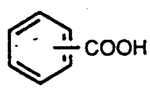 | 〃 | 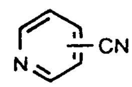 | 〃 | 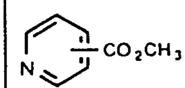 |
| 〃 | 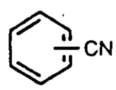 | 〃 | 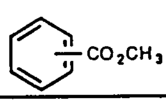 | 〃 | 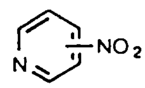 | 〃 | 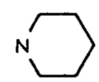 |
| 〃 | 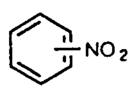 | 〃 | 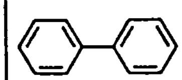 | 〃 | 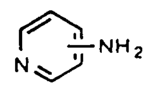 | 〃 | 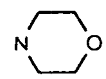 |
| 〃 | 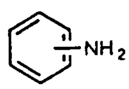 | 〃 | 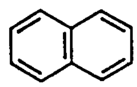 | 〃 | 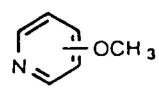 | | |
| 〃 | 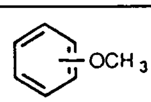 | 〃 | 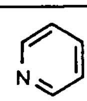 | 〃 | 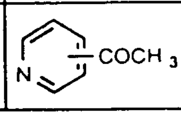 | | |

Table 16

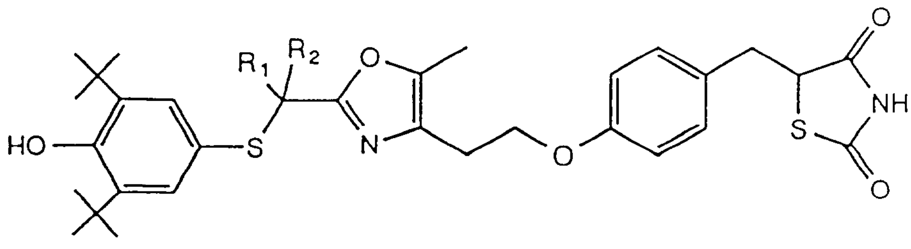

| $R_1$ | $R_2$ | $R_1$ | $R_2$ | $R_1$ | $R_2$ | $R_1$ | $R_2$ |
|---|---|---|---|---|---|---|---|
| 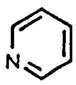 | H | 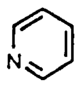 | 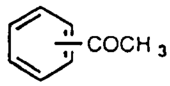 | 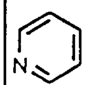 | 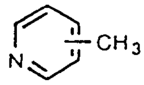 | 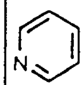 | 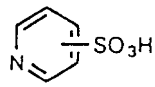 |
| 〃 | 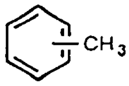 | 〃 | 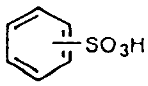 | 〃 | 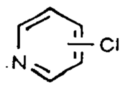 | 〃 | 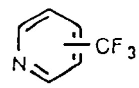 |
| 〃 | 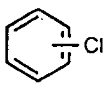 | 〃 | 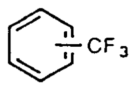 | 〃 | 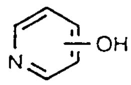 | 〃 | 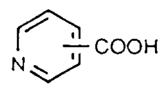 |
| 〃 | 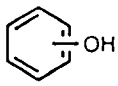 | 〃 | 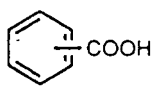 | 〃 | 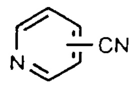 | 〃 | 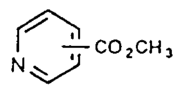 |
| 〃 | 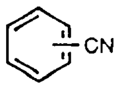 | 〃 | 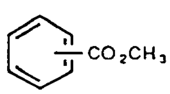 | 〃 | 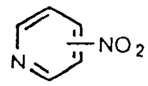 | 〃 | 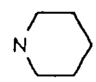 |
| 〃 | 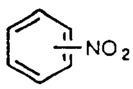 | 〃 | 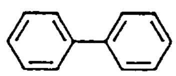 | 〃 | 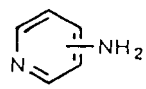 | 〃 | 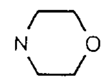 |
| 〃 | 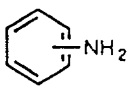 | 〃 | 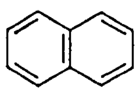 | 〃 | 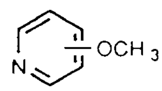 | | |
| 〃 | 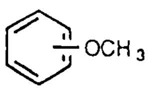 | 〃 | 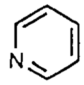 | 〃 | 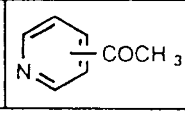 | | |

Table 17

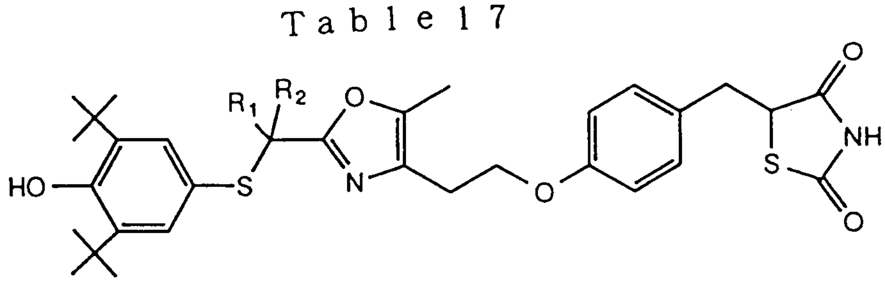

| $R_1$ | $R_2$ | $R_1$ | $R_2$ | $R_1$ | $R_2$ | $R_1$ | $R_2$ |
|---|---|---|---|---|---|---|---|
| 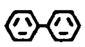 | H | 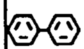 | 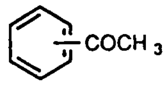 | 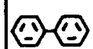 | 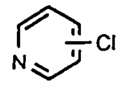 | 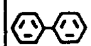 | 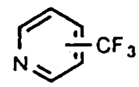 |
| 〃 | 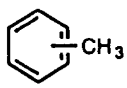 | 〃 | 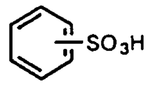 | 〃 | 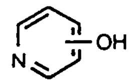 | 〃 | 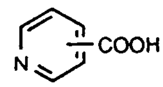 |
| 〃 | 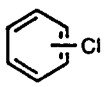 | 〃 | 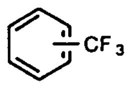 | 〃 | 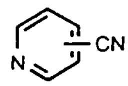 | 〃 | 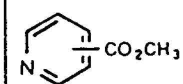 |
| 〃 | 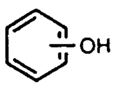 | 〃 | 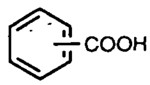 | 〃 | 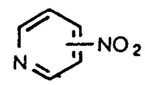 | 〃 | 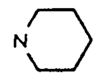 |
| 〃 | 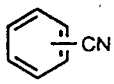 | 〃 | 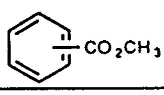 | 〃 | 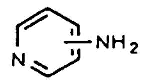 | 〃 | 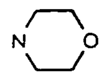 |
| 〃 | 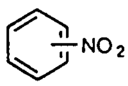 | 〃 | 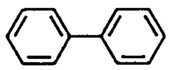 | 〃 | 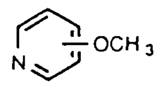 | | |
| 〃 | 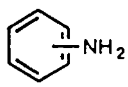 | 〃 | 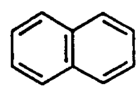 | 〃 | 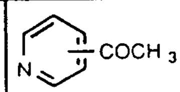 | | |
| 〃 | 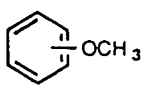 | 〃 | 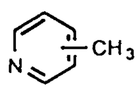 | 〃 | 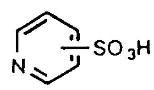 | | |

Table 18

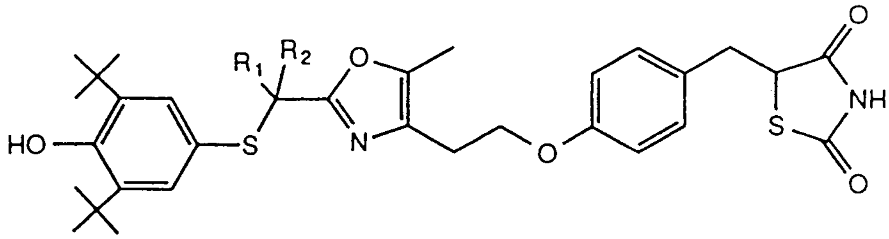

| $R_1$ | $R_2$ | $R_1$ | $R_2$ | $R_1$ | $R_2$ | $R_1$ | $R_2$ |
|---|---|---|---|---|---|---|---|
| 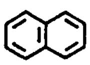 | H | 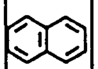 | 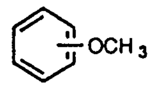 | 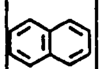 | 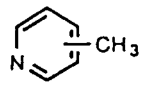 | 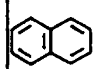 | 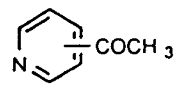 |
| 〃 | 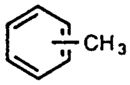 | 〃 | 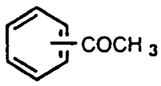 | 〃 | 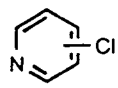 | 〃 | 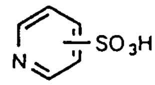 |
| 〃 | 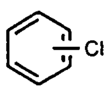 | 〃 | 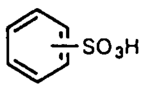 | 〃 | 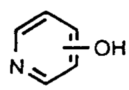 | 〃 | 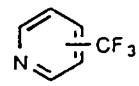 |
| 〃 | 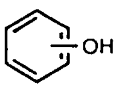 | 〃 | 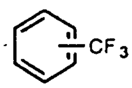 | 〃 | 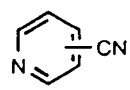 | 〃 | 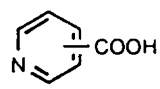 |
| 〃 | 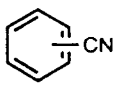 | 〃 | 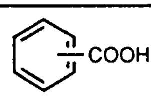 | 〃 | 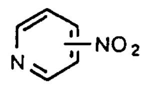 | 〃 | 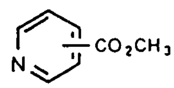 |
| 〃 | 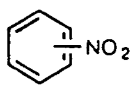 | 〃 | 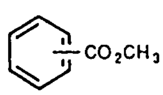 | 〃 | 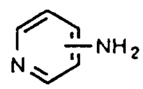 | 〃 | 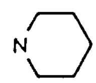 |
| 〃 | 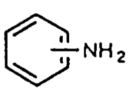 | 〃 | 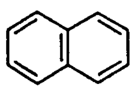 | 〃 | 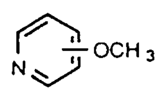 | 〃 | 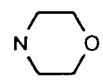 |

Table 19

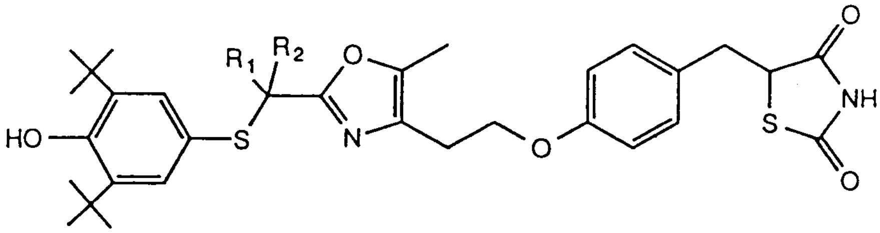

| $R_1$ | $R_2$ | $R_1$ | $R_2$ | $R_1$ | $R_2$ | $R_1$ | $R_2$ |
|---|---|---|---|---|---|---|---|
| 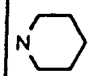 | H | 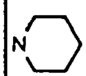 | 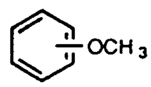 | 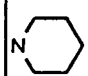 | 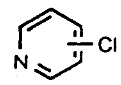 | 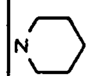 | 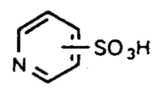 |
| 〃 | 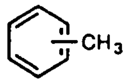 | 〃 | 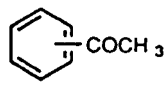 | 〃 | 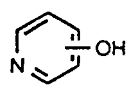 | 〃 | 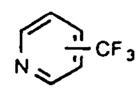 |
| 〃 | 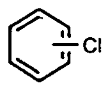 | 〃 | 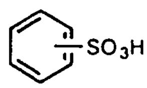 | 〃 | 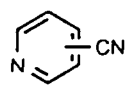 | 〃 | 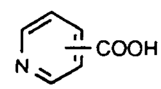 |
| 〃 | 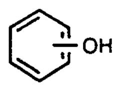 | 〃 | 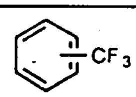 | 〃 | 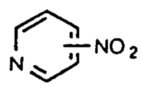 | 〃 | 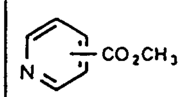 |
| 〃 | 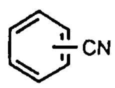 | 〃 | 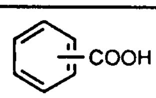 | 〃 | 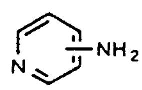 | 〃 | 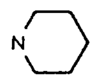 |
| 〃 | 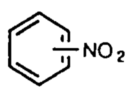 | 〃 | 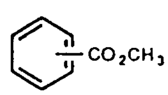 | 〃 | 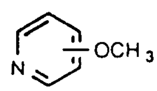 | 〃 | 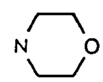 |
| 〃 | 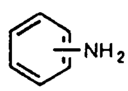 | 〃 | 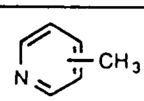 | 〃 | 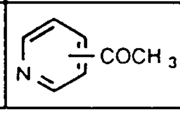 | | |

# Table 20

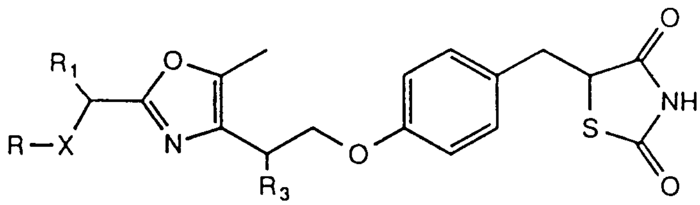

| R | X | R1 | R3 | R | X | R1 | R3 |
|---|---|---|---|---|---|---|---|
| 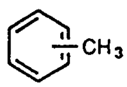 | S | Me | H | 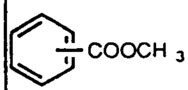 | S | Me | H |
| 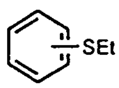 | 〃 | 〃 | 〃 | 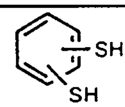 | 〃 | 〃 | 〃 |
| 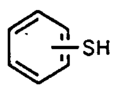 | 〃 | H | 〃 | 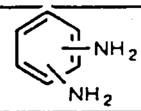 | 〃 | H | 〃 |
| 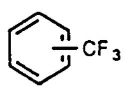 | 〃 | 〃 | 〃 | 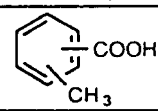 | 〃 | 〃 | 〃 |
| 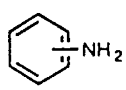 | 〃 | 〃 | 〃 | 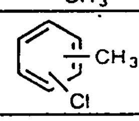 | 〃 | 〃 | OH |
| 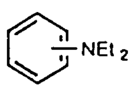 | 〃 | 〃 | 〃 | 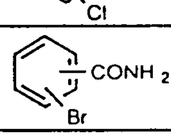 | 〃 | 〃 | H |
| 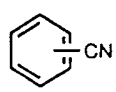 | 〃 | 〃 | 〃 | 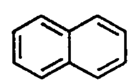 | 〃 | 〃 | 〃 |
| 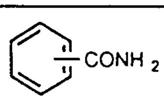 | 〃 | Et | 〃 | 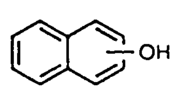 | 〃 | 〃 | 〃 |
| 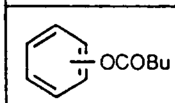 | 〃 | 〃 | OH | 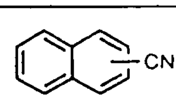 | 〃 | 〃 | OH |
| 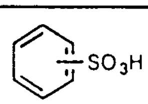 | 〃 | 〃 | 〃 | 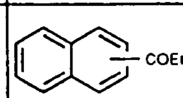 | 〃 | 〃 | H |
| 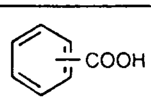 | 〃 | 〃 | OH | 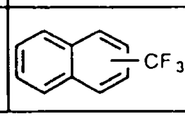 | 〃 | 〃 | 〃 |

Table 21

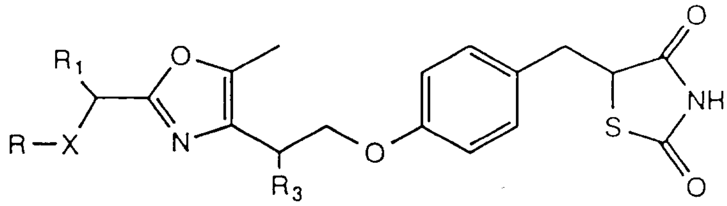

| R | X | R 1 | R 3 | R | X | R 1 | R 3 |
|---|---|---|---|---|---|---|---|
| 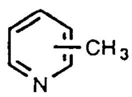 | S | H | H | 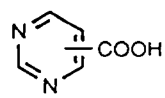 | S | H | H |
| 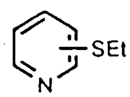 | 〃 | 〃 | 〃 | 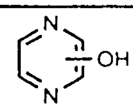 | 〃 | 〃 | 〃 |
| 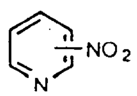 | 〃 | 〃 | 〃 | 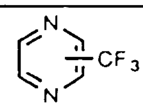 | 〃 | 〃 | 〃 |
| 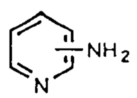 | 〃 | Me | 〃 | 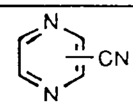 | 〃 | 〃 | 〃 |
| 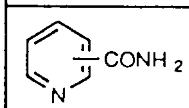 | 〃 | 〃 | 〃 | 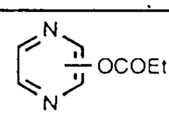 | 〃 | 〃 | 〃 |
| 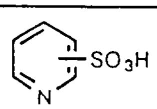 | 〃 | H | 〃 | 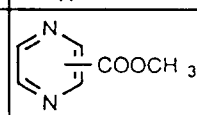 | 〃 | 〃 | 〃 |
| 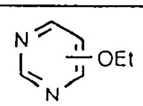 | 〃 | 〃 | OH | 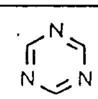 | 〃 | 〃 | 〃 |
| 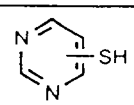 | 〃 | 〃 | 〃 | 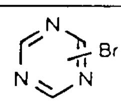 | 〃 | 〃 | 〃 |
| 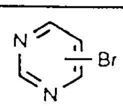 | 〃 | 〃 | H | 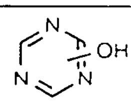 | 〃 | 〃 | 〃 |
| 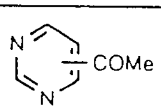 | 〃 | 〃 | 〃 | 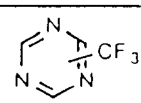 | 〃 | 〃 | 〃 |
| 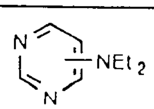 | 〃 | Et | 〃 | 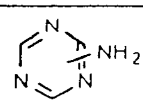 | 〃 | 〃 | 〃 |

Table 22

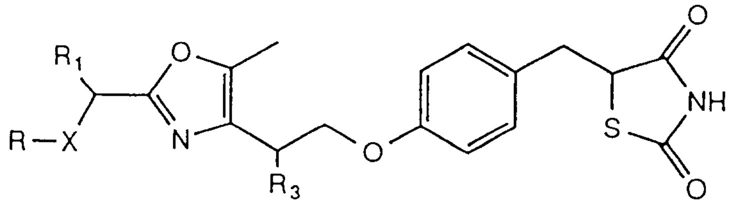

| R | X | $R_1$ | $R_3$ | R | X | $R_1$ | $R_3$ |
|---|---|---|---|---|---|---|---|
| 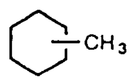 | S | H | H | 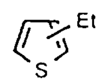 | S | H | H |
| 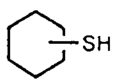 | 〃 | 〃 | 〃 | 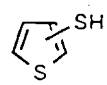 | 〃 | 〃 | 〃 |
| 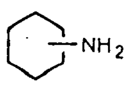 | 〃 | 〃 | 〃 | 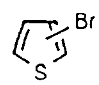 | 〃 | 〃 | 〃 |
| 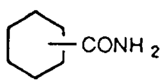 | 〃 | 〃 | 〃 | 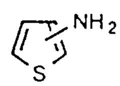 | 〃 | 〃 | 〃 |
| 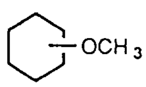 | 〃 | 〃 | 〃 | 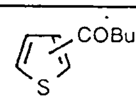 | 〃 | Et | 〃 |
| 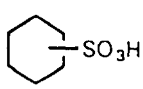 | 〃 | 〃 | 〃 | 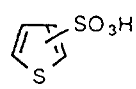 | 〃 | H | 〃 |
| 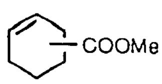 | 〃 | Bu | 〃 | 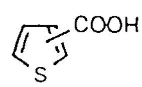 | 〃 | 〃 | 〃 |
| 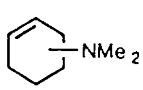 | 〃 | Me | OH | 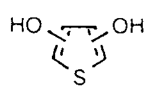 | 〃 | 〃 | 〃 |
| 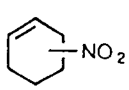 | 〃 | Et | H | 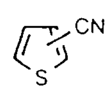 | 〃 | 〃 | 〃 |
| 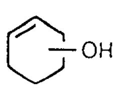 | 〃 | 〃 | 〃 | 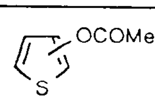 | 〃 | 〃 | 〃 |
| 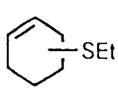 | 〃 | 〃 | 〃 | 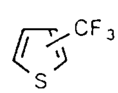 | 〃 | 〃 | 〃 |

Table 23

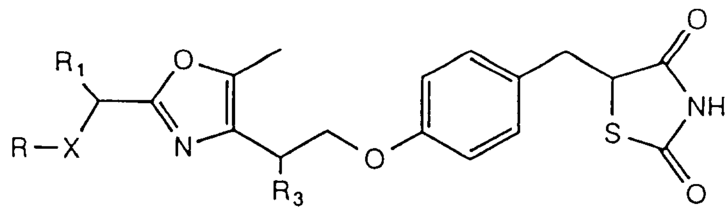

| R | X | $R_1$ | $R_3$ | R | X | $R_1$ | $R_3$ |
|---|---|---|---|---|---|---|---|
| 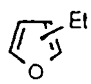 | S | H | H | 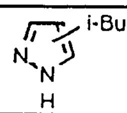 | S | H | H |
| 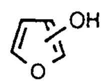 | 〃 | 〃 | 〃 | 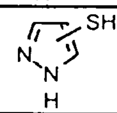 | 〃 | 〃 | 〃 |
| 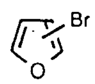 | 〃 | 〃 | 〃 | 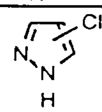 | 〃 | 〃 | 〃 |
| 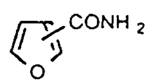 | 〃 | 〃 | 〃 | 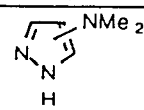 | 〃 | 〃 | 〃 |
| 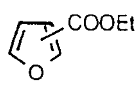 | 〃 | 〃 | 〃 | 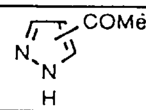 | 〃 | 〃 | 〃 |
| 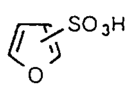 | 〃 | 〃 | 〃 | 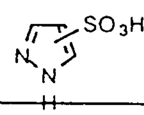 | 〃 | 〃 | 〃 |
| 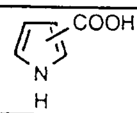 | 〃 | 〃 | 〃 | 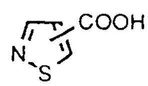 | 〃 | 〃 | 〃 |
| 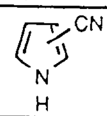 | 〃 | 〃 | 〃 | 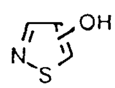 | 〃 | 〃 | 〃 |
| 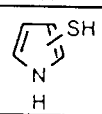 | 〃 | 〃 | 〃 | 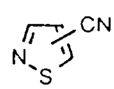 | 〃 | 〃 | 〃 |
| 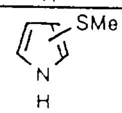 | 〃 | 〃 | 〃 | 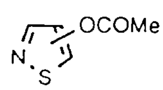 | 〃 | 〃 | 〃 |
| 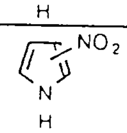 | 〃 | 〃 | 〃 | 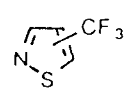 | 〃 | 〃 | 〃 |

Table 24

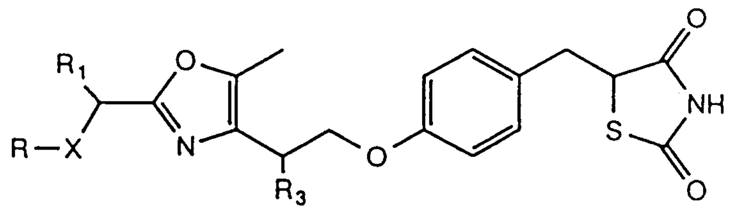

| R | X | $R_1$ | $R_3$ | R | X | $R_1$ | $R_3$ |
|---|---|---|---|---|---|---|---|
| 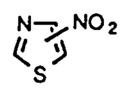 | S | H | H | 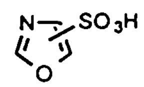 | S | H | H |
| 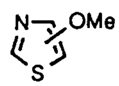 | 〃 | 〃 | 〃 | 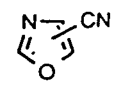 | 〃 | 〃 | 〃 |
| 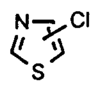 | 〃 | 〃 | 〃 | 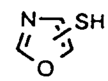 | 〃 | 〃 | 〃 |
| 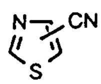 | 〃 | 〃 | 〃 | 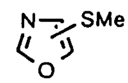 | 〃 | 〃 | 〃 |
| 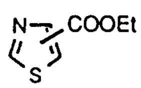 | 〃 | 〃 | 〃 | 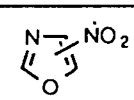 | 〃 | 〃 | 〃 |

Table 25

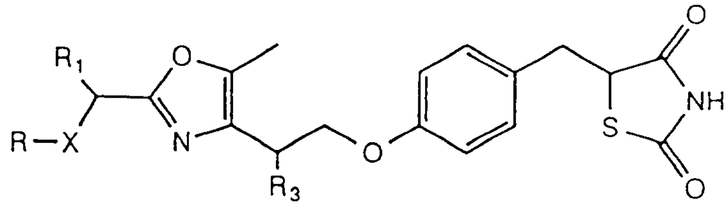

| R | X | R 1 | R 3 | R | X | R 1 | R 3 |
|---|---|---|---|---|---|---|---|
| 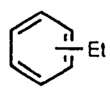 | O | Me | H | 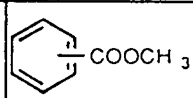 | O | Me | H |
| 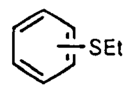 | 〃 | 〃 | 〃 | 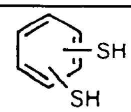 | 〃 | 〃 | 〃 |
| 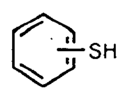 | 〃 | H | 〃 | 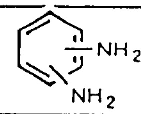 | 〃 | H | 〃 |
| 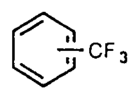 | 〃 | 〃 | 〃 | 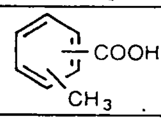 | 〃 | 〃 | 〃 |
| 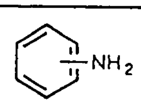 | 〃 | 〃 | 〃 | 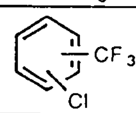 | 〃 | 〃 | OH |
| 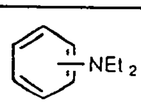 | 〃 | 〃 | 〃 | 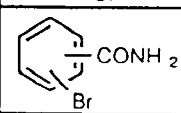 | 〃 | 〃 | H |
| 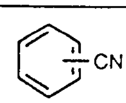 | 〃 | 〃 | 〃 | 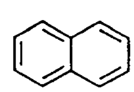 | 〃 | 〃 | 〃 |
| 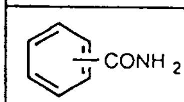 | 〃 | Et | 〃 | 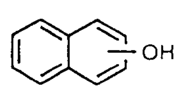 | 〃 | 〃 | 〃 |
| 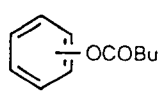 | 〃 | 〃 | OH | 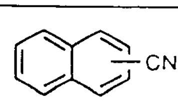 | 〃 | 〃 | OH |
| 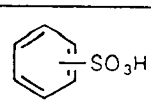 | 〃 | 〃 | 〃 | 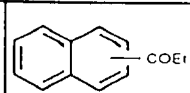 | 〃 | 〃 | H |
| 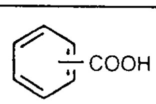 | 〃 | 〃 | OH | 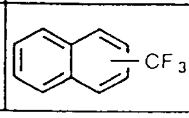 | 〃 | 〃 | 〃 |

Table 26

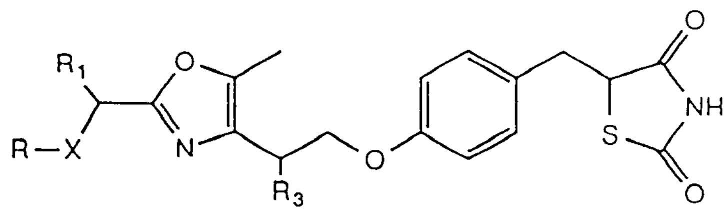

| R | X | $R_1$ | $R_3$ | R | X | $R_1$ | $R_3$ |
|---|---|---|---|---|---|---|---|
| 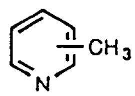 | O | H | H | 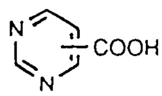 | O | H | H |
| 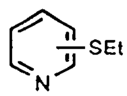 | 〃 | 〃 | 〃 | 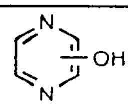 | 〃 | 〃 | 〃 |
| 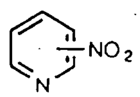 | 〃 | 〃 | 〃 | 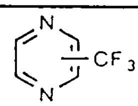 | 〃 | 〃 | 〃 |
| 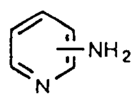 | 〃 | Me | 〃 | 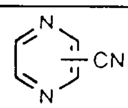 | 〃 | 〃 | 〃 |
| 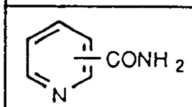 | 〃 | 〃 | 〃 | 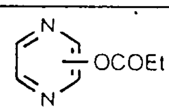 | 〃 | 〃 | 〃 |
| 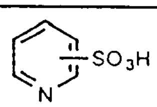 | 〃 | H | 〃 | 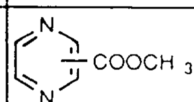 | 〃 | 〃 | 〃 |
| 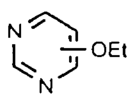 | 〃 | 〃 | OH | 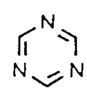 | 〃 | 〃 | 〃 |
| 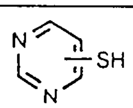 | 〃 | 〃 | 〃 | 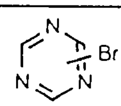 | 〃 | 〃 | 〃 |
| 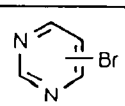 | 〃 | 〃 | H | 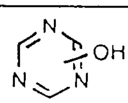 | 〃 | 〃 | 〃 |
| 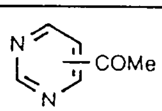 | 〃 | 〃 | 〃 | 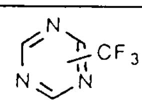 | 〃 | 〃 | 〃 |
| 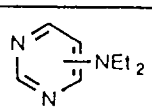 | 〃 | Et | 〃 | 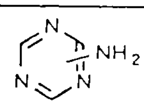 | 〃 | 〃 | 〃 |

Table 27

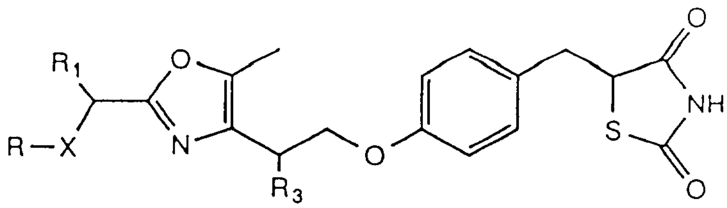

| R | X | R1 | R3 | R | X | R1 | R3 |
|---|---|---|---|---|---|---|---|
| 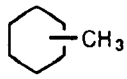 | O | H | H | 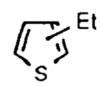 | O | H | H |
| 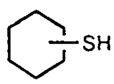 | 〃 | 〃 | 〃 | 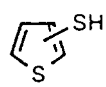 | 〃 | 〃 | 〃 |
| 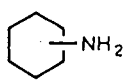 | 〃 | 〃 | 〃 | 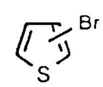 | 〃 | 〃 | 〃 |
| 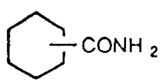 | 〃 | 〃 | 〃 | 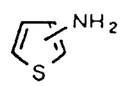 | 〃 | 〃 | 〃 |
| 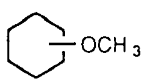 | 〃 | 〃 | 〃 | 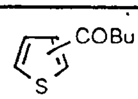 | 〃 | Et | 〃 |
| 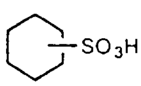 | 〃 | 〃 | 〃 | 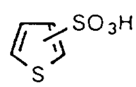 | 〃 | H | 〃 |
| 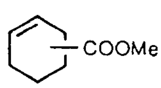 | 〃 | Bu | 〃 | 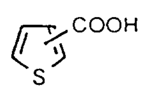 | 〃 | 〃 | 〃 |
| 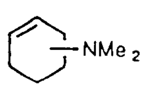 | 〃 | Me | OH | 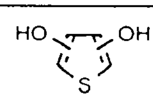 | 〃 | 〃 | 〃 |
| 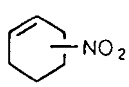 | 〃 | Et | H | 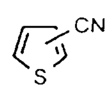 | 〃 | 〃 | 〃 |
| 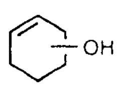 | 〃 | 〃 | 〃 | 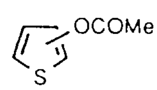 | 〃 | 〃 | 〃 |
| 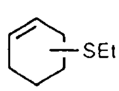 | 〃 | 〃 | 〃 | 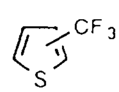 | 〃 | 〃 | 〃 |

Table 28

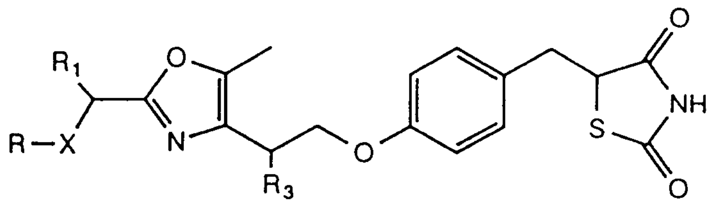

| R | X | $R_1$ | $R_3$ | R | X | $R_1$ | $R_3$ |
|---|---|---|---|---|---|---|---|
| 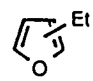 | O | H | H | 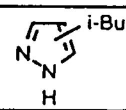 | O | H | H |
| 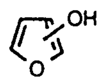 | 〃 | 〃 | 〃 | 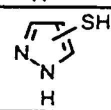 | 〃 | 〃 | 〃 |
| 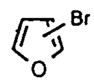 | 〃 | 〃 | 〃 | 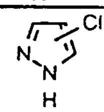 | 〃 | 〃 | 〃 |
| 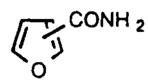 | 〃 | 〃 | 〃 | 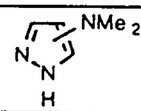 | 〃 | 〃 | 〃 |
| 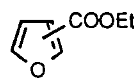 | 〃 | 〃 | 〃 | 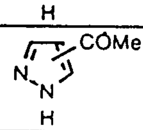 | 〃 | 〃 | 〃 |
| 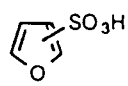 | 〃 | 〃 | 〃 | 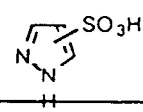 | 〃 | 〃 | 〃 |
| 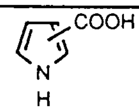 | 〃 | 〃 | 〃 | 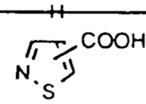 | 〃 | 〃 | 〃 |
| 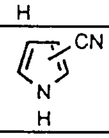 | 〃 | 〃 | 〃 | 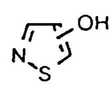 | 〃 | 〃 | 〃 |
| 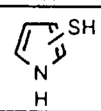 | 〃 | 〃 | 〃 | 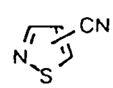 | 〃 | 〃 | 〃 |
| 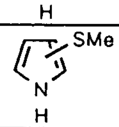 | 〃 | 〃 | 〃 | 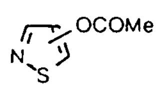 | 〃 | 〃 | 〃 |
| 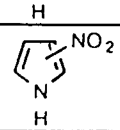 | 〃 | 〃 | 〃 | 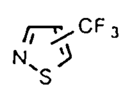 | 〃 | 〃 | 〃 |

Table 29

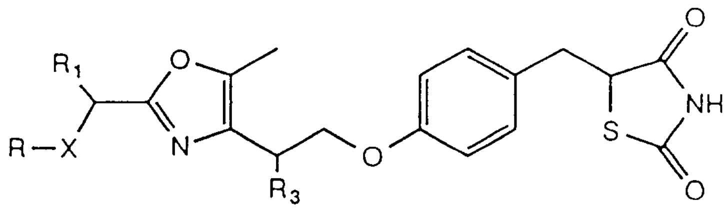

| R | X | $R_1$ | $R_3$ | R | X | $R_1$ | $R_3$ |
|---|---|---|---|---|---|---|---|
| 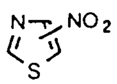 | O | H | H | 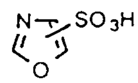 | O | H | H |
| 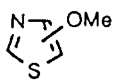 | 〃 | 〃 | 〃 | 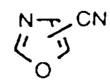 | 〃 | 〃 | 〃 |
| 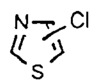 | 〃 | 〃 | 〃 | 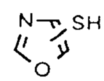 | 〃 | 〃 | 〃 |
| 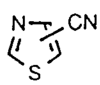 | 〃 | 〃 | 〃 | 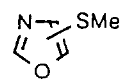 | 〃 | 〃 | 〃 |
| 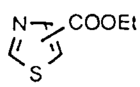 | 〃 | 〃 | 〃 | 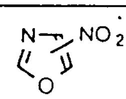 | 〃 | 〃 | 〃 |

Table 30

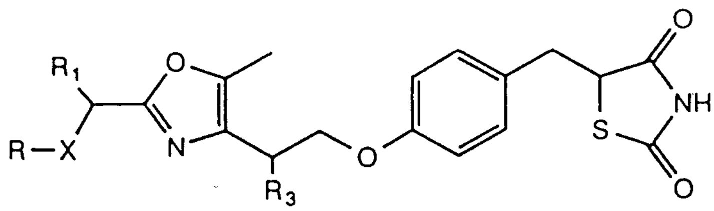

| R | X | $R_1$ | $R_3$ | R | X | $R_1$ | $R_3$ |
|---|---|---|---|---|---|---|---|
| 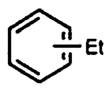 | NH | Me | H | 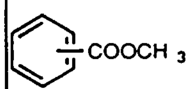 | NH | Me | H |
| 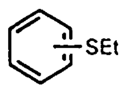 | 〃 | 〃 | 〃 | 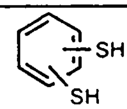 | 〃 | 〃 | 〃 |
| 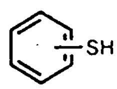 | 〃 | H | 〃 | 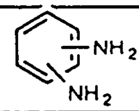 | 〃 | H | 〃 |
| 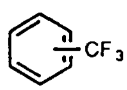 | 〃 | 〃 | 〃 | 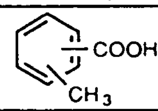 | 〃 | 〃 | 〃 |
| 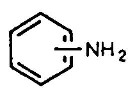 | 〃 | 〃 | 〃 | 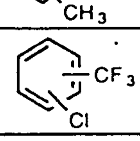 | 〃 | 〃 | OH |
| 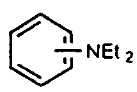 | 〃 | 〃 | 〃 | 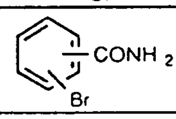 | 〃 | 〃 | H |
| 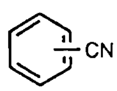 | 〃 | 〃 | 〃 | 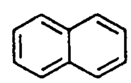 | 〃 | 〃 | 〃 |
| 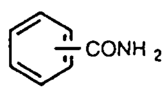 | 〃 | Et | 〃 | 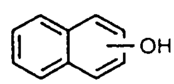 | 〃 | 〃 | 〃 |
| 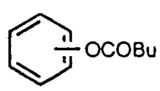 | 〃 | 〃 | OH | 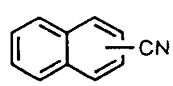 | 〃 | 〃 | OH |
| 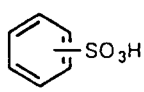 | 〃 | 〃 | 〃 | 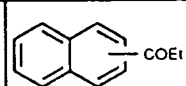 | 〃 | 〃 | H |
| 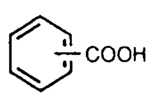 | 〃 | 〃 | OH | 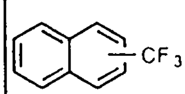 | 〃 | 〃 | 〃 |

# Table 31

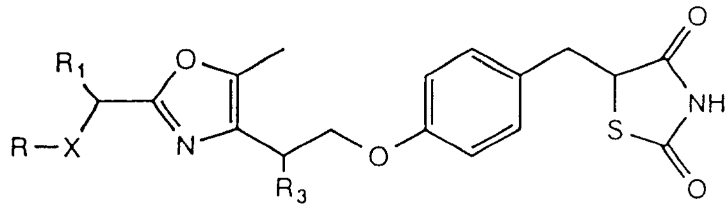

| R | X | $R_1$ | $R_3$ | R | X | $R_1$ | $R_3$ |
|---|---|---|---|---|---|---|---|
| 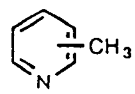 | NH | H | H | 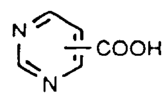 | NH | H | H |
| 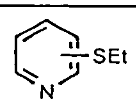 | 〃 | 〃 | 〃 | 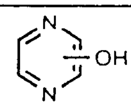 | 〃 | 〃 | 〃 |
| 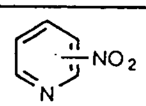 | 〃 | 〃 | 〃 | 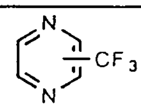 | 〃 | 〃 | 〃 |
| 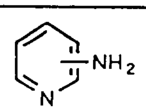 | 〃 | Me | 〃 | 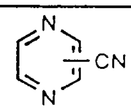 | 〃 | 〃 | 〃 |
| 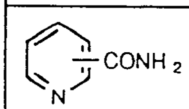 | 〃 | 〃 | 〃 | 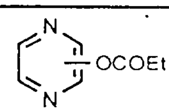 | 〃 | 〃 | 〃 |
| 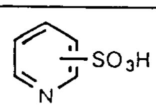 | 〃 | H | 〃 | 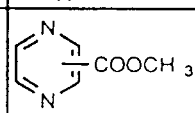 | 〃 | 〃 | 〃 |
| 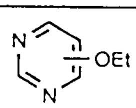 | 〃 | 〃 | OH | 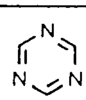 | 〃 | 〃 | 〃 |
| 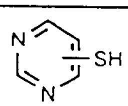 | 〃 | 〃 | 〃 | 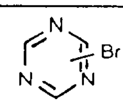 | 〃 | 〃 | 〃 |
| 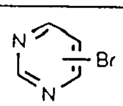 | 〃 | 〃 | H | 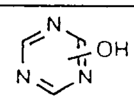 | 〃 | 〃 | 〃 |
| 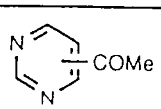 | 〃 | 〃 | 〃 | 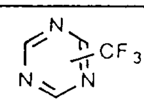 | 〃 | 〃 | 〃 |
| 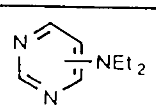 | 〃 | Et | 〃 | 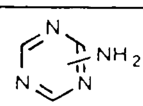 | 〃 | 〃 | 〃 |

Table 32

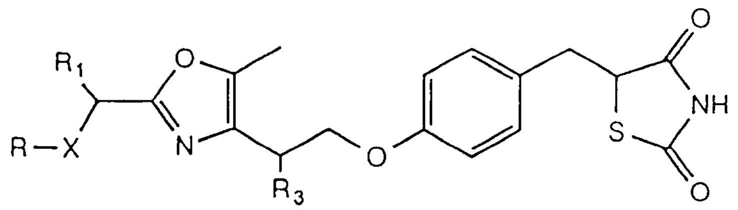

| R | X | $R_1$ | $R_3$ | R | X | $R_1$ | $R_3$ |
|---|---|---|---|---|---|---|---|
| 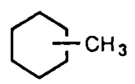 | NH | H | H | 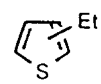 | NH | H | H |
| 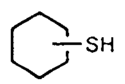 | 〃 | 〃 | 〃 | 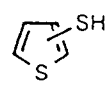 | 〃 | 〃 | 〃 |
| 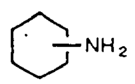 | 〃 | 〃 | 〃 | 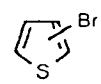 | 〃 | 〃 | 〃 |
| 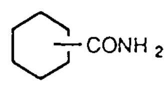 | 〃 | 〃 | 〃 | 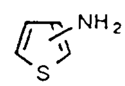 | 〃 | 〃 | 〃 |
| 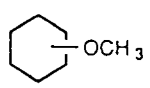 | 〃 | 〃 | 〃 | 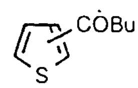 | 〃 | Et | 〃 |
| 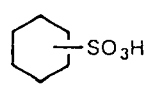 | 〃 | 〃 | 〃 | 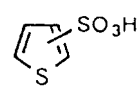 | 〃 | H | 〃 |
| 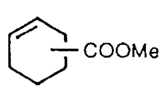 | 〃 | Bu | 〃 | 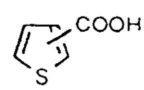 | 〃 | 〃 | 〃 |
| 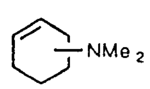 | 〃 | Me | OH | 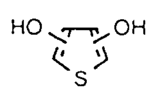 | 〃 | 〃 | 〃 |
| 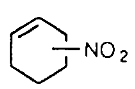 | 〃 | Et | H | 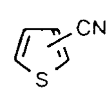 | 〃 | 〃 | 〃 |
| 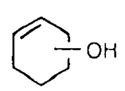 | 〃 | 〃 | 〃 | 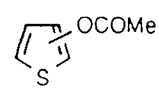 | 〃 | 〃 | 〃 |
| 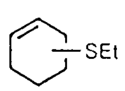 | 〃 | 〃 | 〃 | 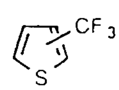 | 〃 | 〃 | 〃 |

Table 33

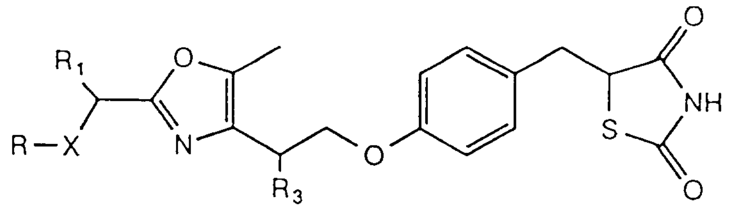

| R | X | $R_1$ | $R_3$ | R | X | $R_1$ | $R_3$ |
|---|---|---|---|---|---|---|---|
| 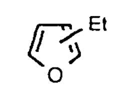 | NH | H | H | 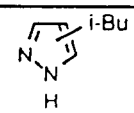 | NH | H | H |
| 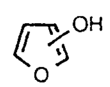 | 〃 | 〃 | 〃 | 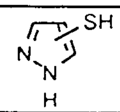 | 〃 | 〃 | 〃 |
| 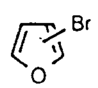 | 〃 | 〃 | 〃 | 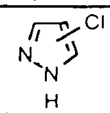 | 〃 | 〃 | 〃 |
| 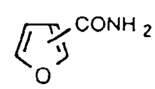 | 〃 | 〃 | 〃 | 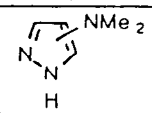 | 〃 | 〃 | 〃 |
| 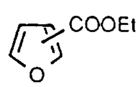 | 〃 | 〃 | 〃 | 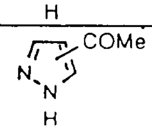 | 〃 | 〃 | 〃 |
| 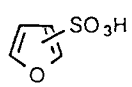 | 〃 | 〃 | 〃 | 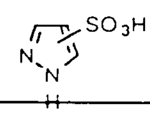 | 〃 | 〃 | 〃 |
| 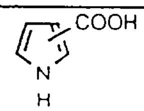 | 〃 | 〃 | 〃 | 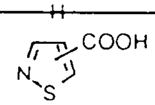 | 〃 | 〃 | 〃 |
| 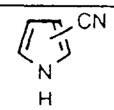 | 〃 | 〃 | 〃 | 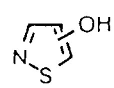 | 〃 | 〃 | 〃 |
| 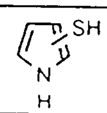 | 〃 | 〃 | 〃 | 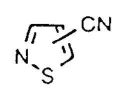 | 〃 | 〃 | 〃 |
| 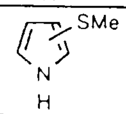 | 〃 | 〃 | 〃 | 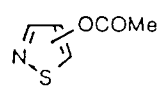 | 〃 | 〃 | 〃 |
| 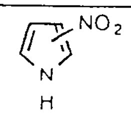 | 〃 | 〃 | 〃 | 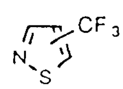 | 〃 | 〃 | 〃 |

Table 34

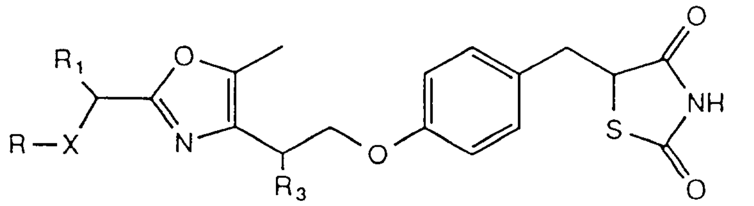

| R | X | $R_1$ | $R_3$ | R | X | $R_1$ | $R_3$ |
|---|---|---|---|---|---|---|---|
| 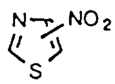 | NH | H | H | 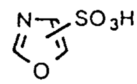 | NH | H | H |
| 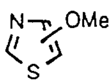 | 〃 | 〃 | 〃 | 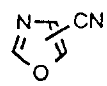 | 〃 | 〃 | 〃 |
| 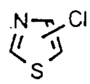 | 〃 | 〃 | 〃 | 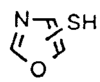 | 〃 | 〃 | 〃 |
| 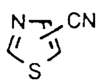 | 〃 | 〃 | 〃 | 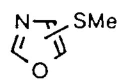 | 〃 | 〃 | 〃 |
| 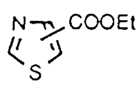 | 〃 | 〃 | 〃 | 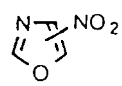 | 〃 | 〃 | 〃 |

It is needless to say that the compound (IV), which is a production intermediate in the present invention, is not limited to the foregoing Examples. For example, the compounds shown in the following Tables 35 through 46 also fall within the scope of the present invention.

Table 35

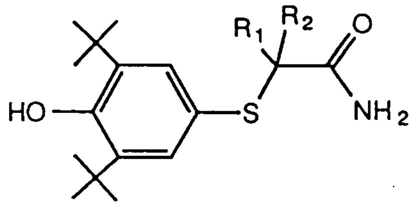

| $R_1$ | $R_2$ | $R_1$ | $R_2$ | $R_1$ | $R_2$ | $R_1$ | $R_2$ |
|---|---|---|---|---|---|---|---|
| Me | Et | Me | 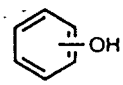 | Me | 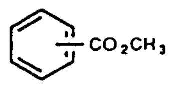 | Me | 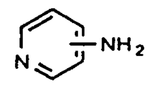 |
| 〃 | Pr | 〃 | 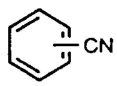 | 〃 | 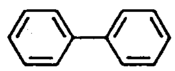 | 〃 | 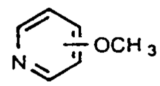 |
| 〃 | i-Pr | 〃 | 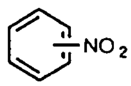 | 〃 | 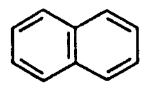 | 〃 | 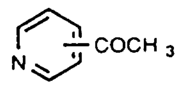 |
| 〃 | Bu | 〃 | 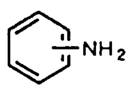 | 〃 | 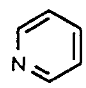 | 〃 | 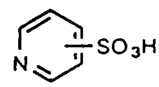 |
| 〃 | i-Bu | 〃 | 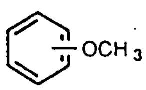 | 〃 | 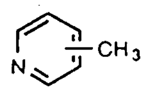 | 〃 | 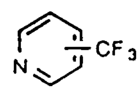 |
| 〃 | t-Bu | 〃 | 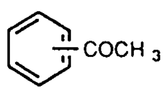 | 〃 | 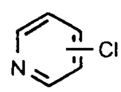 | 〃 | 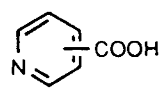 |
| 〃 | 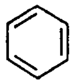 | 〃 | 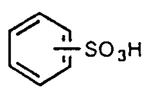 | 〃 | 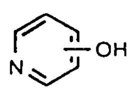 | 〃 | 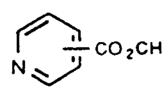 |
| 〃 | 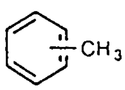 | 〃 | 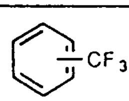 | 〃 | 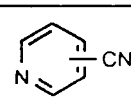 | 〃 | 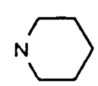 |
| 〃 | 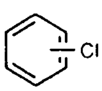 | 〃 | 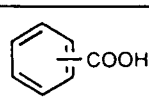 | 〃 | 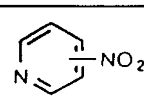 | 〃 | 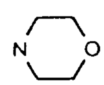 |

Table 36

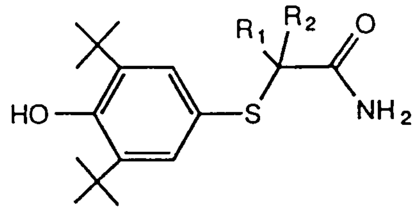

| $R_1$ | $R_2$ | $R_1$ | $R_2$ | $R_1$ | $R_2$ | $R_1$ | $R_2$ |
|---|---|---|---|---|---|---|---|
| Et | Et | Et | 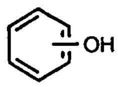 | Et | 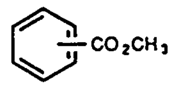 | Et | 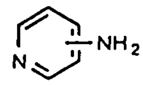 |
| 〃 | Pr | 〃 | 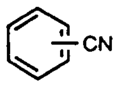 | 〃 | 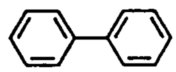 | 〃 | 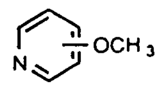 |
| 〃 | i-Pr | 〃 | 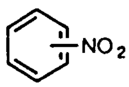 | 〃 | 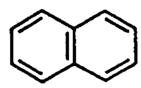 | 〃 | 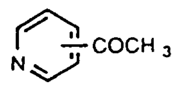 |
| 〃 | Bu | 〃 | 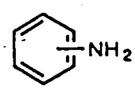 | 〃 | 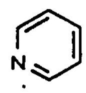 | 〃 | 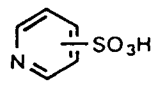 |
| 〃 | i-Bu | 〃 | 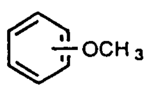 | 〃 | 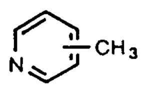 | 〃 | 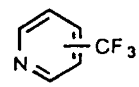 |
| 〃 | t-Bu | 〃 | 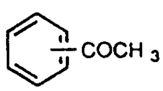 | 〃 | 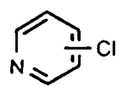 | 〃 | 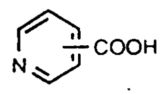 |
| 〃 | 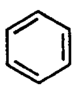 | 〃 | 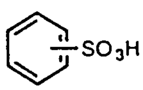 | 〃 | 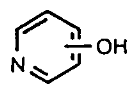 | 〃 | 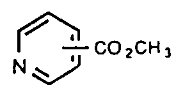 |
| 〃 | 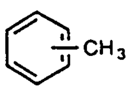 | 〃 | 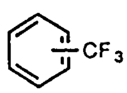 | 〃 | 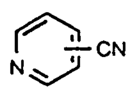 | 〃 | 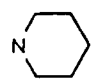 |
| 〃 | 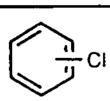 | 〃 | 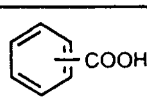 | 〃 | 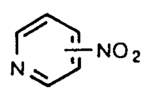 | 〃 | 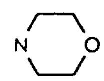 |

Table 37

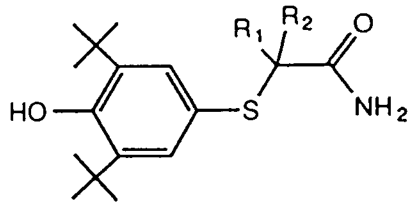

| R1 | R2 | R1 | R2 | R1 | R2 | R1 | R2 |
|---|---|---|---|---|---|---|---|
| Pr | H | Pr | 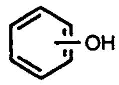 | Pr | 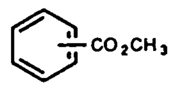 | Pr | 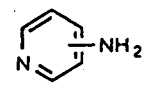 |
| 〃 | Pr | 〃 | 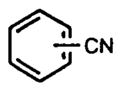 | 〃 | 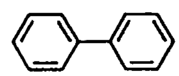 | 〃 | 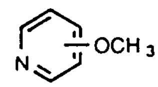 |
| 〃 | i-Pr | 〃 | 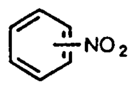 | 〃 | 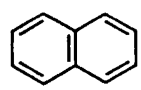 | 〃 | 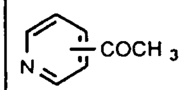 |
| 〃 | Bu | 〃 | 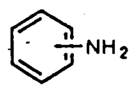 | 〃 | 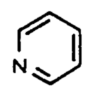 | 〃 | 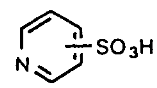 |
| 〃 | i-Bu | 〃 | 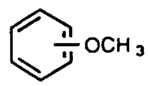 | 〃 | 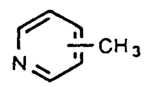 | 〃 | 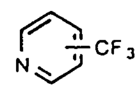 |
| 〃 | t-Bu | 〃 | 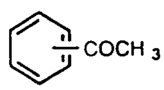 | 〃 | 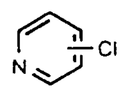 | 〃 | 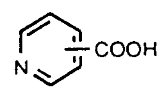 |
| 〃 | 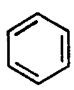 | 〃 | 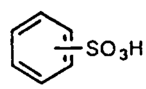 | 〃 | 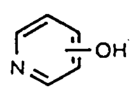 | 〃 | 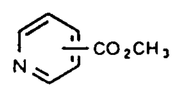 |
| 〃 | 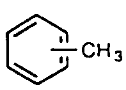 | 〃 | 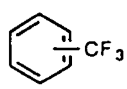 | 〃 | 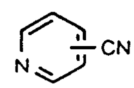 | 〃 | 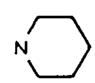 |
| 〃 | 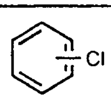 | 〃 | 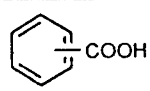 | 〃 | 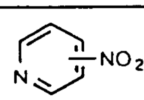 | 〃 | 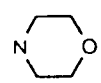 |

Table 38

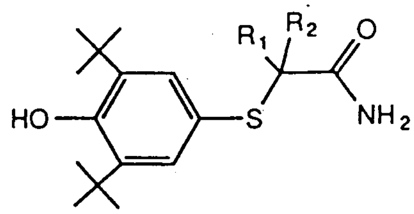

| $R_1$ | $R_2$ | $R_1$ | $R_2$ | $R_1$ | $R_2$ | $R_1$ | $R_2$ |
|---|---|---|---|---|---|---|---|
| i-Pr | H | i-Pr | 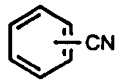 | i-Pr | 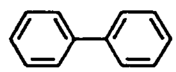 | i-Pr | 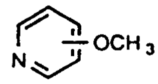 |
| 〃 | i-Pr | 〃 | 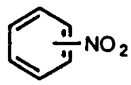 | 〃 | 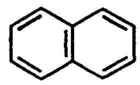 | 〃 | 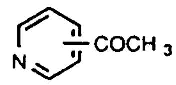 |
| 〃 | Bu | 〃 | 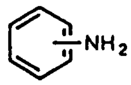 | 〃 | 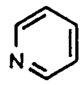 | 〃 | 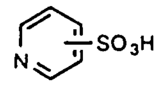 |
| 〃 | i-Bu | 〃 | 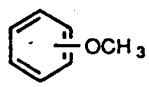 | 〃 | 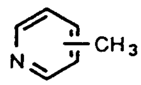 | 〃 | 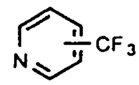 |
| 〃 | t-Bu | 〃 | 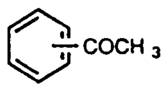 | 〃 | 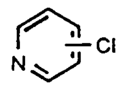 | 〃 | 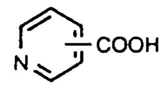 |
| 〃 | 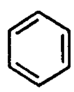 | 〃 | 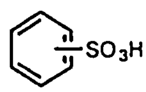 | 〃 | 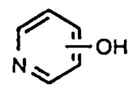 | 〃 | 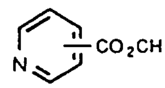 |
| 〃 | 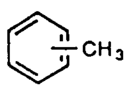 | 〃 | 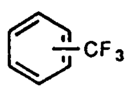 | 〃 | 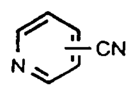 | 〃 | 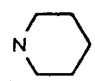 |
| 〃 | 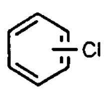 | 〃 | 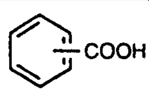 | 〃 | 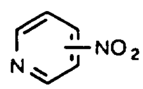 | 〃 | 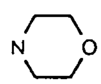 |
| 〃 | 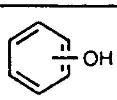 | 〃 | 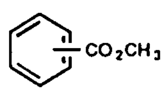 | 〃 | 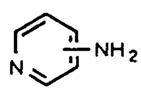 | | |

Table 39

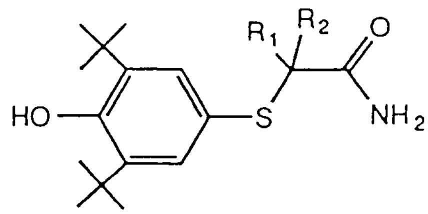

| $R_1$ | $R_2$ | $R_1$ | $R_2$ | $R_1$ | $R_2$ | $R_1$ | $R_2$ |
|---|---|---|---|---|---|---|---|
| Bu | H | Bu | 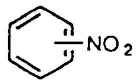 | Bu | 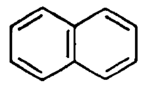 | Bu | 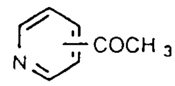 |
| 〃 | Bu | 〃 | 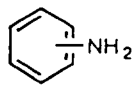 | 〃 | 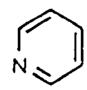 | 〃 | 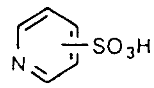 |
| 〃 | i-Bu | 〃 | 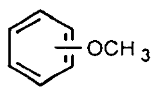 | 〃 | 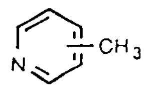 | 〃 | 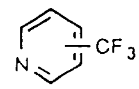 |
| 〃 | t-Bu | 〃 | 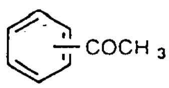 | 〃 | 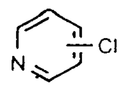 | 〃 | 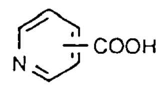 |
| 〃 | 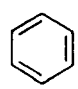 | 〃 | 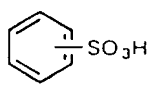 | 〃 | 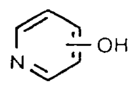 | 〃 | 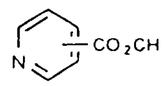 |
| 〃 | 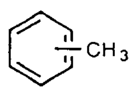 | 〃 | 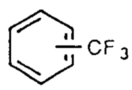 | 〃 | 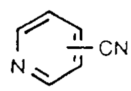 | 〃 | 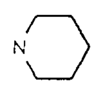 |
| 〃 | 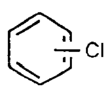 | 〃 | 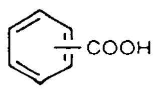 | 〃 | 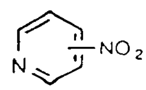 | 〃 | 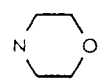 |
| 〃 | 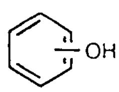 | 〃 | 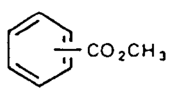 | 〃 | 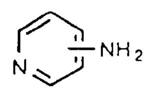 | | |
| 〃 | 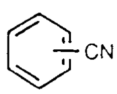 | 〃 | 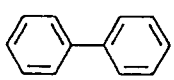 | 〃 | 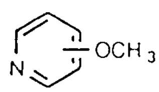 | | |

Table 40

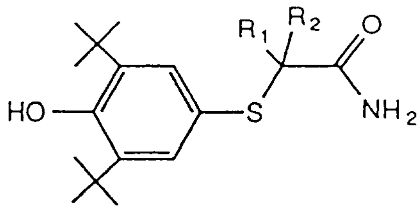

| $R_1$ | $R_2$ | $R_1$ | $R_2$ | $R_1$ | $R_2$ | $R_1$ | $R_2$ |
|---|---|---|---|---|---|---|---|
| i-Bu | H | i-Bu | 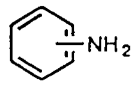 | i-Bu | 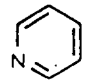 | i-Bu | 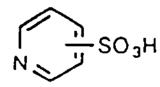 |
| 〃 | i-Bu | 〃 | 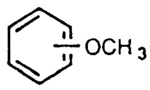 | 〃 | 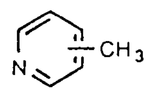 | 〃 | 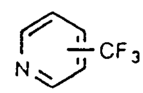 |
| 〃 | t-Bu | 〃 | 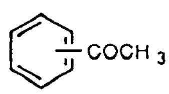 | 〃 | 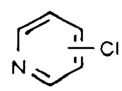 | 〃 | 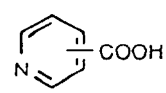 |
| 〃 | 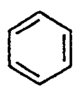 | 〃 | 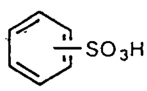 | 〃 | 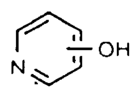 | 〃 | 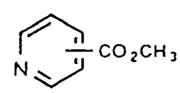 |
| 〃 | 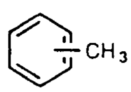 | 〃 | 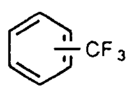 | 〃 | 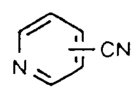 | 〃 | 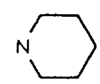 |
| 〃 | 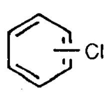 | 〃 | 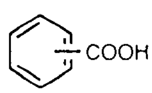 | 〃 | 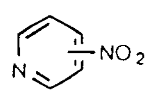 | 〃 | 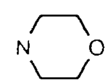 |
| 〃 | 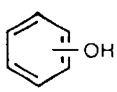 | 〃 | 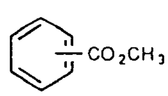 | 〃 | 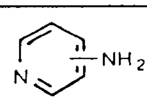 | | |
| 〃 | 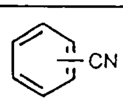 | 〃 | 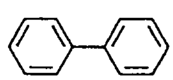 | 〃 | 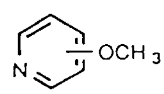 | | |
| 〃 | 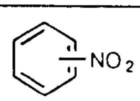 | 〃 | 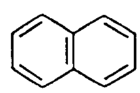 | 〃 | 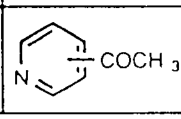 | | |

Table 41

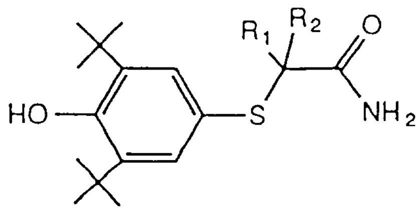

| R1 | R2 | R1 | R2 | R1 | R2 | R1 | R2 |
|---|---|---|---|---|---|---|---|
| t-Bu | H | t-Bu | 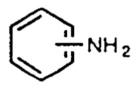 | t-Bu | 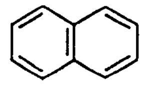 | t-Bu | 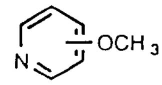 |
| 〃 | t-Bu | 〃 | 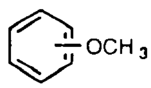 | 〃 | 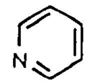 | 〃 | 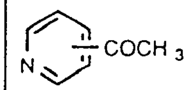 |
| 〃 | 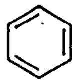 | 〃 | 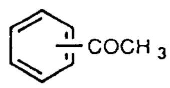 | 〃 | 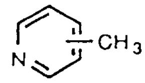 | 〃 | 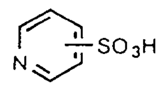 |
| 〃 | 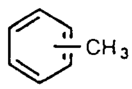 | 〃 | 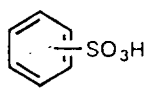 | 〃 | 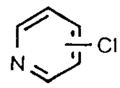 | 〃 | 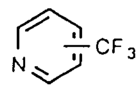 |
| 〃 | 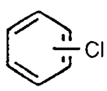 | 〃 | 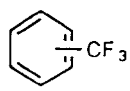 | 〃 | 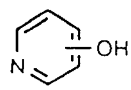 | 〃 | 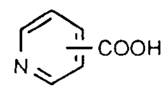 |
| 〃 | 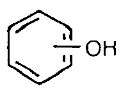 | 〃 | 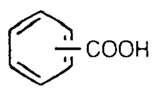 | 〃 | 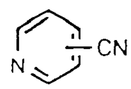 | 〃 | 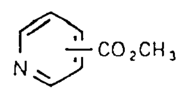 |
| 〃 | 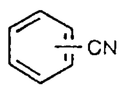 | 〃 | 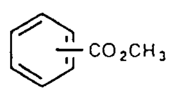 | 〃 | 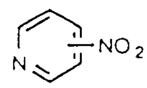 | 〃 | 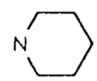 |
| 〃 | 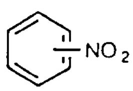 | 〃 | 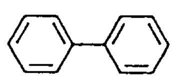 | 〃 | 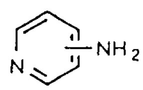 | 〃 | 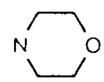 |

Table 42

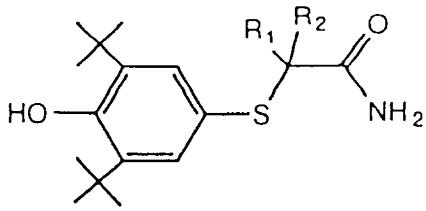

| $R_1$ | $R_2$ | $R_1$ | $R_2$ | $R_1$ | $R_2$ | $R_1$ | $R_2$ |
|---|---|---|---|---|---|---|---|
| 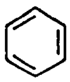 | 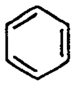 | 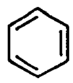 | 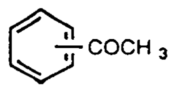 | 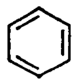 | 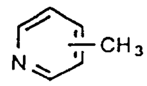 | 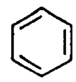 | 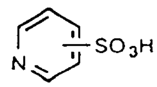 |
| 〃 | 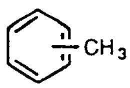 | 〃 | 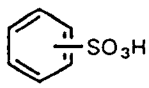 | 〃 | 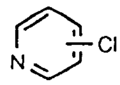 | 〃 | 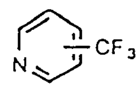 |
| 〃 | 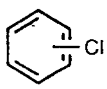 | 〃 | 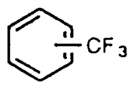 | 〃 | 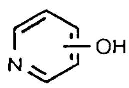 | 〃 | 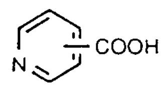 |
| 〃 | 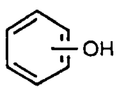 | 〃 | 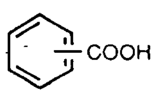 | 〃 | 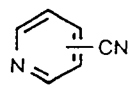 | 〃 | 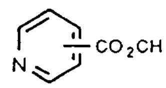 |
| 〃 | 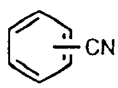 | 〃 | 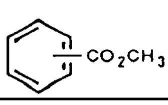 | 〃 | 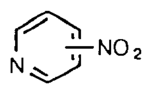 | 〃 | 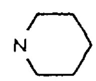 |
| 〃 | 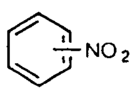 | 〃 | 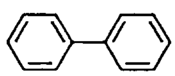 | 〃 | 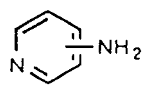 | 〃 | 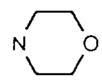 |
| 〃 | 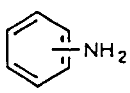 | 〃 | 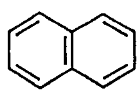 | 〃 | 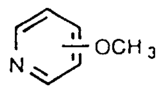 | | |
| 〃 | 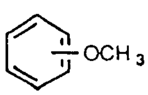 | 〃 | 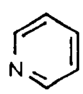 | 〃 | 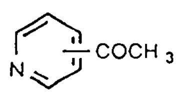 | | |

Table 43

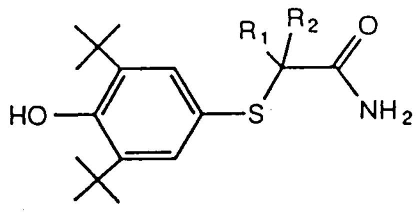

| R1 | R2 | R1 | R2 | R1 | R2 | R1 | R2 |
|---|---|---|---|---|---|---|---|
| 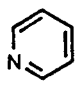 | H | 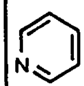 | 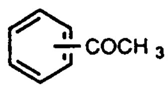 | 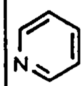 | 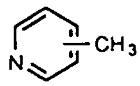 | 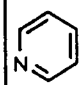 | 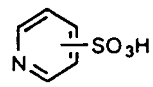 |
| 〃 | 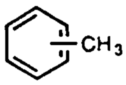 | 〃 | 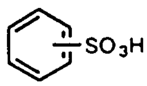 | 〃 | 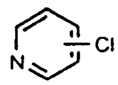 | 〃 | 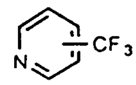 |
| 〃 | 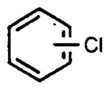 | 〃 | 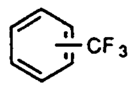 | 〃 | 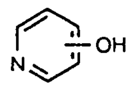 | 〃 | 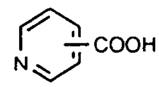 |
| 〃 | 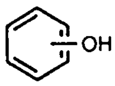 | 〃 | 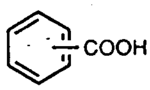 | 〃 | 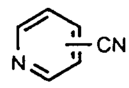 | 〃 | 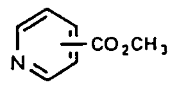 |
| 〃 | 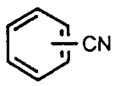 | 〃 | 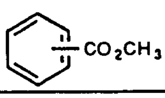 | 〃 | 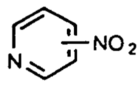 | 〃 | 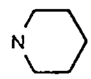 |
| 〃 | 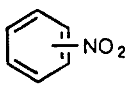 | 〃 | 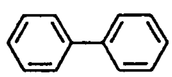 | 〃 | 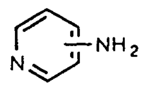 | 〃 | 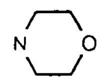 |
| 〃 | 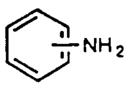 | 〃 | 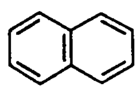 | 〃 | 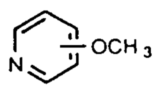 | | |
| 〃 | 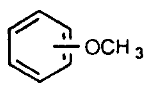 | 〃 | 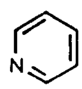 | 〃 | 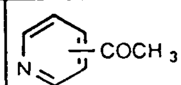 | | |

## Table 44

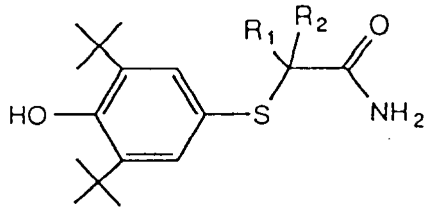

| $R_1$ | $R_2$ | $R_1$ | $R_2$ | $R_1$ | $R_2$ | $R_1$ | $R_2$ |
|---|---|---|---|---|---|---|---|
| 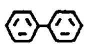 | H | 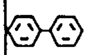 | 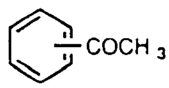 | 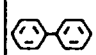 | 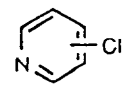 | 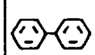 | 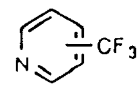 |
| 〃 | 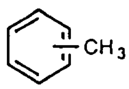 | 〃 | 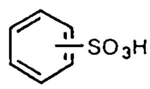 | 〃 | 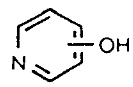 | 〃 | 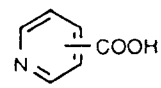 |
| 〃 | 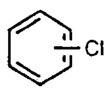 | 〃 | 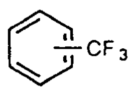 | 〃 | 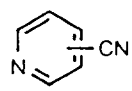 | 〃 | 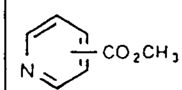 |
| 〃 | 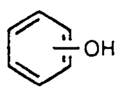 | 〃 | 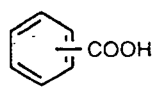 | 〃 | 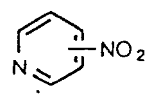 | 〃 | 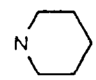 |
| 〃 | 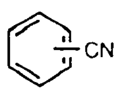 | 〃 | 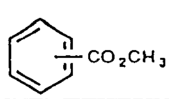 | 〃 | 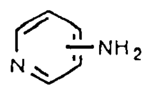 | 〃 | 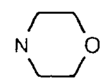 |
| 〃 | 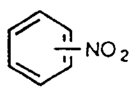 | 〃 | 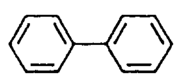 | 〃 | 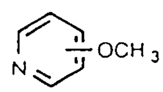 | | |
| 〃 | 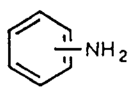 | 〃 | 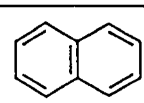 | 〃 | 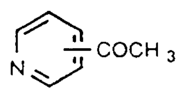 | | |
| 〃 | 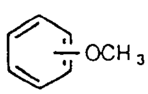 | 〃 | 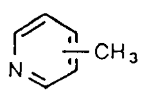 | 〃 | 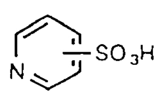 | | |

Table 45

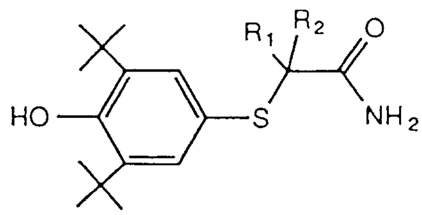

| $R_1$ | $R_2$ | $R_1$ | $R_2$ | $R_1$ | $R_2$ | $R_1$ | $R_2$ |
|---|---|---|---|---|---|---|---|
| 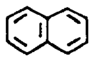 | H | 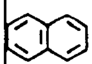 | 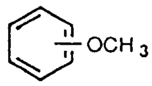 | 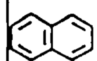 | 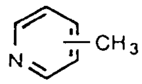 | 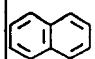 | 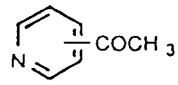 |
| 〃 | 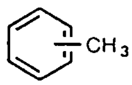 | 〃 | 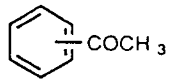 | 〃 | 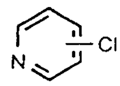 | 〃 | 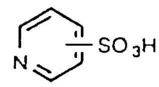 |
| 〃 | 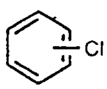 | 〃 | 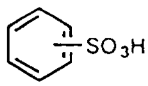 | 〃 | 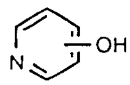 | 〃 | 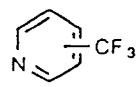 |
| 〃 | 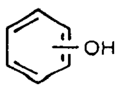 | 〃 | 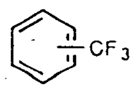 | 〃 | 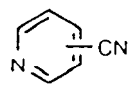 | 〃 | 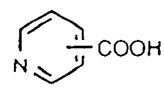 |
| 〃 | 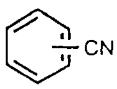 | 〃 | 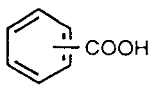 | 〃 | 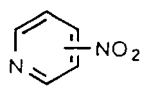 | 〃 | 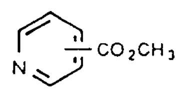 |
| 〃 | 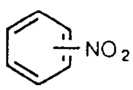 | 〃 | 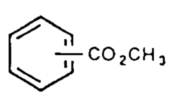 | 〃 | 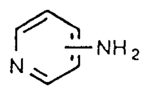 | 〃 | 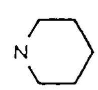 |
| 〃 | 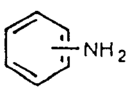 | 〃 | 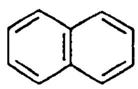 | 〃 | 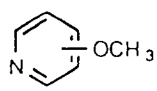 | 〃 | 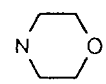 |

Table 46

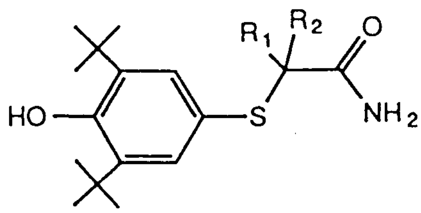

| R1 | R2 | R1 | R2 | R1 | R2 | R1 | R2 |
|---|---|---|---|---|---|---|---|
| 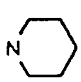 | H | 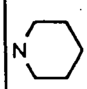 | 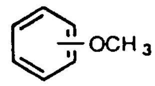 | 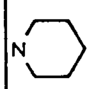 | 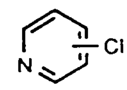 | 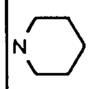 | 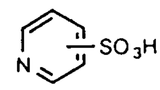 |
| 〃 | 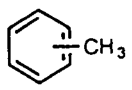 | 〃 | 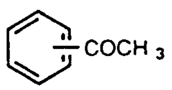 | 〃 | 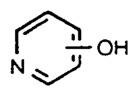 | 〃 | 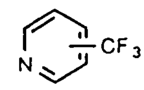 |
| 〃 | 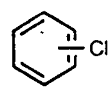 | 〃 | 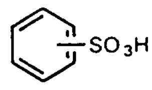 | 〃 | 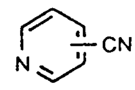 | 〃 | 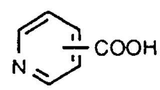 |
| 〃 | 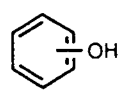 | 〃 | 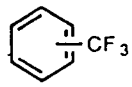 | 〃 | 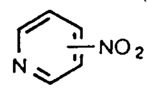 | 〃 | 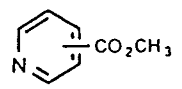 |
| 〃 | 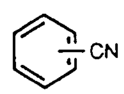 | 〃 | 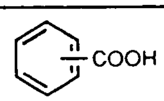 | 〃 | 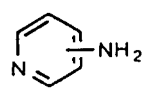 | 〃 | 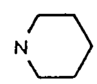 |
| 〃 | 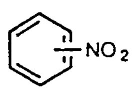 | 〃 | 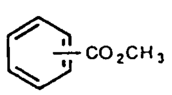 | 〃 | 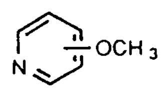 | 〃 | 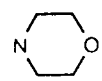 |
| 〃 | 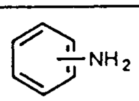 | 〃 | 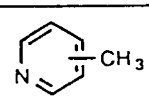 | 〃 | 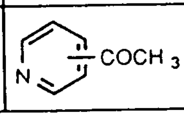 | | |

Experiment 1

Genetically fat, hyperglycemic and hyperlipemic mice with diabetes (C57BL/Ksj-db/db, male, Jackson Laboratories/Japan Kurea, 13 weeks of age and KK-$A^y$, male, Japan Kurea, 13 weeks of age) were used for the pharmacological experiments. As a reference compound, a hypoglycemic agent CS-045 [(±)-5-[4-(6-hydroxy-2,5,7,8-tetramethylchroman-2-yl-methoxy)benzyl]-2,4-thiazolidinedione] [see Diabetes, vol. 37, p. 1549 (1988)] was used.

The mice were weighed and blood samples were taken immediately before the initiation of administration on day 1. Serum glucose and serum triglyceride were measured, based on which the mice were grouped (6-8 per group) in such a manner that there existed no difference in terms of average body weight, average serum glucose value and average serum triglyceride value.

The test drugs were all suspended in a solution of 0.5% sodium carboxymethylcellulose and administered orally twice a day (the second administration was 6 hours after the first administration) on day 1, day 2, day 3 and day 4. To a vehicle control group, a solution of 0.5% sodium carboxymethylcellulose was orally administered.

At day 5, blood samples were taken again and measured for serum glucose and serum triglyceride. The blood sample was taken from fundus vein by 400 μl under anesthetization with ether and kept at ice temperature. After separation into serum (12000 rpm, 5 min.), serum glucose was measured by hexokinase

method (glucose-HK-test "BMY"; Bohelinger Mannheim Yamanouchi) and serum triglyceride was measured by enzyme method (triglycolor III "BMY"; Bohelinger Mannheim Yamanouchi). Used for the measurement was an automatic analyzer COBAS FARA (manufactured by Roche).

Change in percent of serum glucose value and serum tnglyceride value in each group was calculated using serum glucose value and serum triglyceride value, respectively, of vehicle control group at day 5 as follows:

$$\text{Change in percent of serum glucose value (\%)} = \frac{\left[\left(\begin{array}{l}\text{serum glucose value of}\\\text{each group at day 5}\end{array}\right) - \left(\begin{array}{l}\text{serum glucose value of vehi-}\\\text{cle control group at day 5}\end{array}\right)\right]}{\text{serum glucose value of vehicle control group at day 5}} \times 100$$

$$\text{Change in percent of serum triglyceride value (\%)} = \frac{\left[\left(\begin{array}{l}\text{serum triglyceride value}\\\text{of each group at day 5}\end{array}\right) - \left(\begin{array}{l}\text{serum triglyceride valus of}\\\text{vehicle control groupatday 5}\end{array}\right)\right]}{\text{serum triglyceride value of vehicle control group at day 5}} \times 100$$

The results are shown in Table 47.

Table 47

| | dose (mg/kg) | serum glucose (%) | | serum triglyceride (%) | |
|---|---|---|---|---|---|
| | | KK-A$^y$ mouse | db/db mouse | KK-A$^y$ mouse | db/db mouse |
| Ex. 1 | 10 | -50.3 | -22.4 | -46.2 | -44.7 |
| | 30 | -53.2 | -44.6 | -43.3 | -63.1 |
| Ex. 2 | 10 | -50.4 | -20.3 | -55.4 | -48.8 |
| | 30 | -40.9 | -52.2 | -62.2 | -51.0 |
| Ex. 3 | 10 | -25.7 | | -23.9 | -12.5 |
| Ex. 4 | 10 | -45.9 | | -46.6 | -27.5 |
| Ex. 5 | 10 | -23.5 | | -23.5 | -19.5 |
| Ex. 6 | 10 | -46.0 | | -28.4 | -25.9 |
| Ex. 7 | 10 | -44.9 | -21.7 | -52.8 | -32.9 |
| Ex. 8 | 30 | -50.3 | -31.8 | -62.4 | -56.1 |
| Ex. 9 | 10 | -55.6 | -44.5 | -70.7 | -63.3 |
| Ex. 10 | 10 | -57.8 | | -54.6 | -34.8 |
| Ex. 11 | 10 | -49.0 | -27.2 | -67.4 | -44.0 |
| Ex. 13 | 1 | -55.3 | | -64.9 | |
| | 3 | -48.7 | | -54.9 | |
| | 10 | -54.8 | -34.3 | -63.8 | -64.5 |
| Ex. 14 | 10 | -63.2 | -38.6 | -75.2 | -63.4 |
| Ex. 15 | 10 | -21.6 | | -32.9 | -16.9 |
| Ex. 16 | 10 | -24.2 | | | -17.1 |
| Ex. 17 | 10 | -24.7 | | | |
| | 30 | -30.0 | -28.1 | -19.2 | -41.2 |
| Ex. 18 | 10 | -54.9 | | -57.1 | -10.4 |
| Ex. 19 | 10 | -44.2 | -14.7 | -53.4 | -37.6 |
| Ex. 20 | 10 | -60.2 | -21.0 | -72.2 | -36.3 |
| Ex. 22 | 10 | -46.6 | | -32.4 | -45.3 |
| Ex. 24 | 10 | -51.2 | -51.5 | -60.8 | -57.4 |
| CS-045 | 100 | -29.4 | -21.5 | -22.9 | -55.5 |

As shown in Table 47, the compounds of the present invention lowered serum glucose value and serum triglyceride value of both kinds of mice with diabetes more singinificantly than did the control compound.

From the foregoing, it is evident that the compounds of the present invention have superior hypoglycemic and hypolipemic actions and are useful for the treatment of diabetes and hyperlipemia. In addition, the compounds of the invention are expected to be efficacious for the prevention and treatment of the complications of diabetes, particularly arteriosclerosis.

| Formulation Example (Tablets) | |
|---|---|
| (1) Compound of Example 9 | 10 g |
| (2) Lactose | 50 g |
| (3) Corn starch | 15 g |
| (4) Sodium carboxymethylcellulose | 44 g |
| (5) Magnesium stearate | 1 g |

The above-mentioned amounts of (1), (2) and (3) and 30 g of (4) were kneaded with water, dried in vacuo and granulated. The granulated powder was mixed with 14 g of (4) and 1 g of (5) and prepared into tablets by a compressor to give 1000 tablets containing 10 mg each.

The thiazolidinedione compounds and salts thereof of the present invention are novel compounds having extremely potent and low toxic hypoglycemic and hypolipemic actions as compared with known thiazolidine compounds and other therapeutic agents of diabetes, and are very useful as a therapeutic agent for diabetes, an agent for the prevention and treatment of the complications thereof and as a therapeutic agent for hyperlipemia.

**Claims**

1. A novel thiazolidinedione compound of the formula (I)

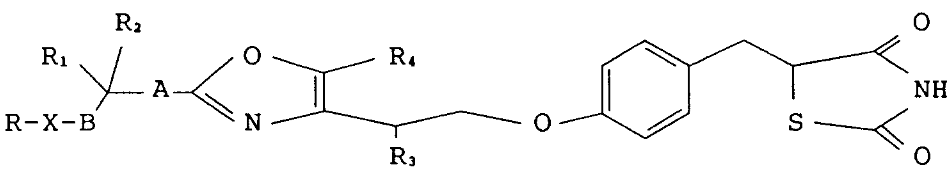

(I)

wherein;

| | |
|---|---|
| R | is optionally substituted aromatic hydrocarbon, optionally substituted alicyclic hydrocarbon or optionally substituted aromatic heterocyclic group; |
| $R_1$ and $R_2$ | are each independently hydrogen atom, lower alkyl, or aromatic hydrocarbon or aromatic heterocyclic group which may be substituted by lower alkyl, acyl, lower alkoxy, cyano, nitro, lower alkoxycarbonyl or sulfonyl; |
| $R_3$ | is hydrogen atom, hydroxy, lower alkyl, acyl, lower alkoxy, cyano, nitro, lower alkoxycarbonyl or sulfonyl; |
| $R_4$ | is lower alkyl; |
| X | is oxygen atom, sulfur atom or secondary amino; |
| A | is $-(CH_2)m-$ where m is an integer of 0, 1 or 2; and |
| B | is $-(CH_2)n-$ where n is an integer of 0, 1 or 2, |

or a pharmaceutically acceptable salt thereof.

2. The novel thiazolidinedione compound of Claim 1, wherein $R_1$ and $R_2$ are each independently hydrogen atom, lower alkyl or aromatic hydrocarbon, $R_3$ is hydrogen atom or hydroxy, $R_4$ is methyl, m is 0 and n is 0, or a pharmaceutically acceptable salt thereof.

3. The novel thiazolidinedione compound of Claim 2, wherein R is optionally substituted phenyl, optionally substituted 5- or 6-membered alicyclic hydrocarbon or optionally substituted 5- or 6-membered aromatic heterocyclic group which has a hetero atom selected from the group consisting of nitrogen atom, oxygen atom and sulfur atom, or a pharmaceutically acceptable salt thereof.

4. The novel thiazolidinedione compound of Claim 3, wherein R is optionally substituted phenyl, or a pharmaceutically acceptable salt thereof.

5. The novel thiazolidinedione compound of Claim 4, wherein the optionally substituted phenyl is 3,5-di-tert-butyl-4-hydroxyphenyl, or a pharmaceutically acceptable salt thereof.

6. The novel thiazolidinedione compound of Claim 3, wherein R is 5- or 6-membered alicyclic hydrocarbon which may have 1 or 2 double bonds, or a pharmaceutically acceptable salt thereof.

7. The novel thiazolidinedione compound of Claim 6, wherein R is cyclopentyl or cyclohexyl, or a pharmaceutically acceptable salt thereof.

8. The novel thiazolidinedione compound of Claim 3, wherein the aromatic heterocyclic group at R is a 5- or 6-membered aromatic heterocyclic group which has 1 to 3 hetero atoms selected from the group consisting of nitrogen atom, oxygen atom and sulfur atom, or a pharmaceutically acceptable salt thereof.

9. The novel thiazolidinedione compound of Claim 8, wherein the aromatic heterocyclic group at R is a 5- or 6-membered aromatic heterocyclic group which has 1 to 3 nitrogen atoms, or a pharmaceutically acceptable salt thereof.

10. The novel thiazolidinedione compound of Claim 1, wherein R is selected from the group consisting of methoxyphenyl, hydroxyphenyl, benzyloxyphenyl, pyridyl, pyrimidinyl, nitrophenyl, chlorophenyl, di-tert-butylphenyl, 1-methyl-2-imidazolyl, cyclohexyl, phenyl, dimethoxyphenyl and di-tert-butyl-hydroxyphenyl, or a pharmaceutically acceptable salt thereof.

11. A compound of the formula

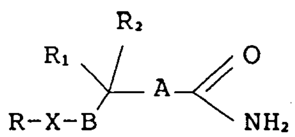

wherein;

| | |
|---|---|
| R | is optionally substituted aromatic hydrocarbon, optionally substituted alicyclic hydrocarbon or optionally substituted aromatic heterocyclic group; |
| $R_1$ and $R_2$ | are each independently hydrogen atom, lower alkyl, or aromatic hydrocarbon or aromatic heterocyclic group which may be substituted by lower alkyl, acyl, lower alkoxy, cyano, nitro, lower alkoxycarbonyl or sulfonyl; |
| X | is oxygen atom, sulfur atom or secondary amino; |
| A | is $-(CH_2)m-$ where m is an integer of 0, 1 or 2; and |
| B | is $-(CH_2)n-$ where n is an integer of 0, 1 or 2. |

12. The compound of Claim 11, wherein $R_1$ and $R_2$ are each independently hydrogen atom, lower alkyl or aromatic hydrocarbon, m is 0 and n is 0.

13. A compound of the formula

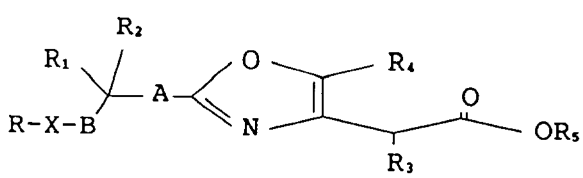

wherein;

| | |
|---|---|
| R | is optionally substituted aromatic hydrocarbon, optionally substituted alicyclic hydrocarbon or optionally substituted aromatic heterocyclic group; |
| $R_1$ and $R_2$ | are each independently hydrogen atom, lower alkyl, or aromatic hydrocarbon or aromatic heterocyclic group which may be substituted by lower alkyl, acyl, lower alkoxy, cyano, nitro, lower alkoxycarbonyl or sulfonyl; |
| $R_3$ | is hydrogen atom, hydroxy, lower alkyl, acyl, lower alkoxy, cyano, nitro, lower |

alkoxycarbonyl or sulfonyl;

$R_4$ is lower alkyl;

$R_5$ is hydrogen atom or lower alkyl;

X is oxygen atom, sulfur atom or secondary amino;

A is $-(CH_2)m-$ where m is an integer of 0, 1 or 2; and

B is $-(CH_2)n-$ where n is an integer of 0, 1 or 2.

**14.** The compound of Claim 13, wherein $R_1$ and $R_2$ are each independently hydrogen atom, lower alkyl or aromatic hydrocarbon, $R_3$ is hydrogen atom or hydroxy, $R_4$ is methyl, m is 0 and n is 0.

**15.** A compound of the formula

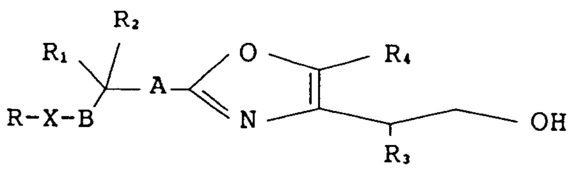

wherein;

R is optionally substituted aromatic hydrocarbon, optionally substituted alicyclic hydrocarbon or optionally substituted aromatic heterocyclic group;

$R_1$ and $R_2$ are each independently hydrogen atom, lower alkyl, or aromatic hydrocarbon or aromatic heterocyclic group which may be substituted by lower alkyl, acyl, lower alkoxy, cyano, nitro, lower alkoxycarbonyl or sulfonyl;

$R_3$ is hydrogen atom, hydroxy, lower alkyl, acyl, lower alkoxy, cyano, nitro, lower alkoxycarbonyl or sulfonyl;

$R_4$ is lower alkyl;

X is oxygen atom, sulfur atom or secondary amino;

A is $-(CH_2)m-$ where m is an integer of 0, 1 or 2; and

B is $-(CH_2)n-$ where n is an integer of 0, 1 or 2.

**16.** The compound of Claim 15, wherein $R_1$ and $R_2$ are each independently hydrogen atom, lower alkyl or aromatic hydrocarbon, $R_3$ is hydrogen atom or hydroxy, $R_4$ is methyl, m is 0 and n is 0.

**17.** A compound of the formula

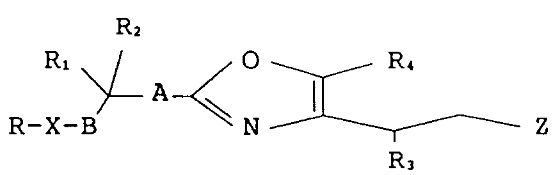

wherein;

R is optionally substituted aromatic hydrocarbon, optionally substituted alicyclic hydrocarbon or optionally substituted aromatic heterocyclic group;

$R_1$ and $R_2$ are each independently hydrogen atom, lower alkyl, or aromatic hydrocarbon or aromatic heterocyclic group which may be substituted by lower alkyl, acyl, lower alkoxy, cyano, nitro, lower alkoxycarbonyl or sulfonyl;

$R_3$ is hydrogen atom, hydroxy, lower alkyl, acyl, lower alkoxy, cyano, nitro, lower alkoxycarbonyl or sulfonyl;

$R_4$ is lower alkyl;

X is oxygen atom, sulfur atom or secondary amino;

A is $-(CH_2)m-$ where m is an integer of 0, 1 or 2;

B is $-(CH_2)n-$ where n is an integer of 0, 1 or 2; and

Z is a leaving group.

**18.** The compound of Claim 17, wherein $R_1$ and $R_2$ are each independently hydrogen atom, lower alkyl or aromatic hydrocarbon, $R_3$ is hydrogen atom or hydroxy, $R_4$ is methyl, m is 0 and n is 0.

**19.** The novel thiazolidinedione compound of Claim 1, which is selected from the group consisting of:
5-[4-[2-[2-[1-(3,5-di-tert-butyl-4-hydroxyphenylthio)ethyl]-5-methyl-4-oxazolyl]ethoxy]benzyl]-2,4-thiazolidinedione;
5-[4-[2-[2-[1-(3,5-di-tert-butyl-4-hydroxyphenylthio)methyl]-5-methyl-4-oxazolyl]ethoxy]benzyl]-2,4-thiazolidinedione;
5-[4-[2-[2-[1-(3,5-di-tert-butyl-4-hydroxyphenylthio)propyl]-5-methyl-4-oxazolyl]ethoxy]benzyl]-2,4-thiazolidinedione;
5-[4-[2-[2-[1-(3,5-di-tert-butyl-4-hydroxyphenylthio)isopropyl]-5-methyl-4-oxazolyl]ethoxy]benzyl]-2,4-thiazolidinedione;
5-[4-[2-[2-[1-(3,5-di-tert-butyl-4-hydroxyphenylthio)-1-phenylmethyl]-5-methyl-4-oxazolyl]ethoxy]benzyl]-2,4-thiazolidinedione;
5-[4-[2-[5-methyl-2-(4-methoxyphenylthio)methyl-4-oxazolyl]ethoxy]benzyl]-2,4-thiazolidinedione;
5-[4-[2-[5-methyl-2-(4-hydroxyphenylthio)methyl-4-oxazolyl]ethoxy]benzyl]-2,4-thiazolidinedione;
5-[4-[2-[5-methyl-2-(4-benzyloxyphenoxy)methyl-4-oxazolyl]ethoxy]benzyl]-2,4-thiazolidinedione;
5-[4-[2-[5-methyl-2-[1-(2-pyridyloxy)ethyl]-4-oxazolyl]ethoxy]benzyl]-2,4-thiazolidinedione;
5-[4-[2-[5-methyl-2-[1-(2-pyridylthio)ethyl]-4-oxazolyl]ethoxy]benzyl]-2,4-thiazolidinedione;
5-[4-[2-[5-methyl-2-(2-pyrimidinylthio)methyl-4-oxazolyl]ethoxy]benzyl]-2,4-thiazolidinedione;
5-[4-[2-[5-methyl-2-(2-pyridylthio)methyl-4-oxazolyl]ethoxy]benzyl]-2,4-thiazolidinedione;
5-[4-[2-[5-methyl-2-(4-nitrophenylthio)methyl-4-oxazolyl]ethoxy]benzyl]-2,4-thiazolidinedione;
5-[4-[2-[5-methyl-2-(4-chlorophenylthio)methyl-4-oxazolyl]ethoxy]benzyl]-2,4-thiazolidinedione;
5-[4-[2-[5-methyl-2-(3,5-di-tert-butylphenoxy)methyl-4-oxazolyl]ethoxy]benzyl]-2,4-thiazolidinedione;
5-[4-[2-[5-methyl-2-(1-methyl-2-imidazolylthio)methyl-4-oxazolyl]ethoxy]benzyl]-2,4-thiazolidinedione;
5-[4-[2-(5-methyl-2-cyclohexylthiomethyl-4-oxazolyl)ethoxy]benzyl]-2,4-thiazolidinedione;
5-[4-[2-(5-methyl-2-phenylaminomethyl-4-oxazolyl)ethoxy]benzyl]-2,4-thiazolidinedione;
5-[4-[2-(5-methyl-2-phenylthiomethyl-4-oxazolyl)ethoxy]benzyl]-2,4-thiazolidinedione;
5-[4[2-(5-methyl-2-phenoxymethyl-4-oxazolyl)ethoxy]benzyl]-2,4-thiazolidinedione;
5-[4-[2-[5-methyl-2-(3,5-dimethoxyphenoxy)methyl-4-oxazolyl]ethoxy]benzyl]-2,4-thiazolidinedione; and
5-[4-[2-[5-methyl-2-(4-hydroxyphenoxy)methyl-4-oxazolyl]ethoxy]benzyl]-2,4-thiazolidinedione, or a pharmaceutically acceptable salt thereof.

**20.** A compound of Claim 11, which is selected from the group consisting of:
2-(3,5-di-tert-butyl-4-hydroxyphenylthio)propionamide;
(3,5-di-tert-butyl-4-hydroxyphenylthio)acetamide;
2-(3,5-di-tert-butyl-4-hydroxyphenylthio)butyramide;
2-(3,5-di-tert-butyl-4-hydroxyphenylthio)isobutyramide; and 2-(3,5-di-tert-butyl-4-hydroxyphenylthio)-2-phenylacetamide.

**21.** A compound of Claim 13, which is selected from the group consisting of:
methyl 2-[1-(3,5-di-tert-butyl-4-hydroxyphenylthio)ethyl]-5-methyl-4-oxazolylacetate;
methyl [2-(3,5-di-tert-butyl-4-hydroxyphenylthio)methyl]-5-methyl-4-oxazolylacetate;
methyl 2-[1-(3,5-di-tert-butyl-4-hydroxyphenylthio)propyl]-5-methyl-4-oxazolylacetate;
methyl 2-[1-(3,5-di-tert-butyl-4-hydroxyphenylthio)isopropyl]-5-methyl-4-oxazolylacetate; and
methyl 2-[1-(3,5-di-tert-butyl-4-hydroxyphenylthio)-1-phenylmethyl]-5-methyl-4-oxazolylacetate.

**22.** A compound of Claim 15, which is selected from the group consisting of:
2-[2-[1-(3,5-di-tert-butyl-4-hydroxyphenylthio)ethyl]-5-methyl-4-oxazolyl]ethanol;
2-[2-(3,5-di-tert-butyl-4-hydroxyphenylthio)methyl-5-methyl-4-oxazolyl]ethanol;
2-[2-[1-(3,5-di-tert-butyl-4-hydroxyphenylthio)propyl]-5-methyl-4-oxazolyl]ethanol; 2-[2-[1-(3,5-di-tert-butyl-4-hydroxyphenylthio)isopropyl]-5-methyl-4-oxazolyl]ethanol;
2-[2-[1-(3,5-di-tert-butyl-4-hydroxyphenylthio)-1-phenylmethyl]-5-methyl-4-oxazolyl]ethanol;
2-[5-methyl-2-(4-methoxyphenylthio)methyl-4-oxazolyl]ethanol;
2-[5-methyl-2-(4-hydroxyphenylthio)methyl-4-oxazolyl]ethanol;
2-[5-methyl-2-(4-benzyloxyphenoxy)methyl-4-oxazolyl]ethanol;
2-[5-methyl-2-(4-hydroxyphenoxy)methyl-4-oxazolyl]ethanol;

2-[5-methyl-2-(3,5-dimethoxyphenoxy)methyl-4-oxazolyl]ethanol;
2-[5-methyl-2-[1-(2-pyridyloxy)ethyl]-4-oxazolyl]ethanol;
2-[5-methyl-2-[1-(2-pyridylthio)ethyl]-4-oxazolyl]ethanol;
2-[5-methyl-2-(2-pyrimidinylthio)methyl-4-oxazolyl]ethanol;
2-[5-methyl-2-(2-pyridylthio)methyl-4-oxazolyl]ethanol;
2-[5-methyl-2-(4-nitrophenylthio)methyl-4-oxazolyl]ethanol;
2-[5-methyl-2-(4-chlorophenylthio)methyl-4-oxazolyl]ethanol;
2-[5-methyl-2-(3,5-di-tert-butylphenoxy)methyl-4-oxazolyl]ethanol;
2-[5-methyl-2-(1-methyl-2-imidazolylthio)methyl-4-oxazolyl]ethanol;
2-(5-methyl-2-cyclohexylthiomethyl-4-oxazolyl)ethanol;
2-(5-methyl-2-phenylaminomethyl-4-oxazolyl)ethanol;
2-(5-methyl-2-phenylthiomethyl-4-oxazolyl)ethanol; and
2-(5-methyl-2-phenoxymethyl-4-oxazolyl)ethanol.

**23.** A compound of Claim 17, which is selected from the group consisting of:
2-[2-[1-(3,5-di-tert-butyl-4-hydroxyphenylthio)ethyl]-5-methyl-4-oxazolyl]ethyl p-toluenesulfonate;
[2-[2-(3,5-di-tert-butyl-4-hydroxyphenylthio)methyl]-5-methyl-4-oxazolyl]ethyl p-toluenesulfonate;
2-[2-[1-(3,5-di-tert-butyl-4-hydroxyphenylthio)propyl]-5-methyl-4-oxazolyl]ethyl p-toluenesulfonate;
2-[2-[1-(3,5-di-tert-butyl-4-hydroxyphenylthio)isopropyl]-5-methyl-4-oxazolyl]ethyl p-toluenesulfonate; and
2-[2-[1-(3,5-di-tert-butyl-4-hydroxyphenylthio)-1-phenylmethyl]-5-methyl-4-oxazolyl]ethyl p-toluenesulfonate.

**24.** A pharmaceutical composition comprising a novel thiazolidinedione compound of Claim 1 or a pharmaceutically acceptable salt thereof, and pharmaceutically acceptable additives.

## INTERNATIONAL SEARCH REPORT

International application No.
PCT/JP93/00967

**A. CLASSIFICATION OF SUBJECT MATTER**

Int. $Cl^5$ C07D417/12, C07D263/32, A61K31/425

According to International Patent Classification (IPC) or to both national classification and IPC

**B. FIELDS SEARCHED**

Minimum documentation searched (classification system followed by classification symbols)

Int. $Cl^5$ C07D417/12, C07D263/32, A61K31/425

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| A | EP, A, 177353 (TAKEDA CHEMICAL IND KK), April 9, 1986 (09. 04. 86), & JP, A, 61-85372 & US, A, 4725610 & WO, A, 8602073 | 1-10, 15-18, 19, 22, 23, 24 |
| A | JP, A, 61-282369 (TAKEDA CHEMICAL IND KK), December 12, 1986 (12. 12. 86), (Family: none) | 1-10, 19, 24 |
| A | EP, A, 96890 (TAKEDA CHEMICAL IND KK), December 28, 1983 (28. 12. 83), & JP, A, 58-219169 & US, A, 4602027 | 11-14, 20, 21 |

[X] Further documents are listed in the continuation of Box C. [ ] See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| October 26, 1993 (26. 10. 93) | November 16, 1993 (16. 11. 93) |
| Name and mailing address of the ISA/<br>Japanese Patent Office | Authorized officer |
| Facsimile No. | Telephone No. |